# EUROPEAN PATENT SPECIFICATION

(11) **EP 2 089 717 B1**
(45) Date of publication and mention of the grant of the patent: **10.09.2014**
(21) Application number: 07859290.4
(22) Date of filing: 23.11.2007
(51) Int. Cl.: G01N 33/574, G01N 33/53

(54) **Circulating VE-cadherin as a predictive marker of sensitivity or resistance to anti-tumoral treatment**
Zirkulierendes VE-cadherin als Marker zur Vorhersage der Empfindlichkeit oder Resistenz gegenüber einer Antitumorbehandlung
VE-cadherine circulante en tant que marqueur prédictif de la sensibilité ou de la résistance à un traitement anti-tumoral

(30) Priority: 23.11.2006 EP 06291805
(43) Date of publication of application: 19.08.2009
(73) Proprietor: INSERM (Institut National de la Santé et de la Recherche Médicale), 75654 Paris Cedex 13 (FR); Université Joseph Fourier, 38041 Grenoble Cedex 9 (FR)
(72) Inventor: VILGRAIN, Isabelle, 38360 Sassenage (FR); PELLETIER, Laurent, 38100 Grenoble (FR); CAND, Francine, 38400 Saint Martin d'Hères (FR)
(74) Representative: Holtz, Béatrice
(86) International application number: PCT/IB2007/004252
(87) International publication number: WO 2008/062314

(56) References cited:
- US-A1- 2006 199 231
- MILANO ET AL: "What clinicians need to know about antioestrogen resistance in breast cancer therapy" EUROPEAN JOURNAL OF CANCER, PERGAMON PRESS, OXFORD, GB, vol. 42, no. 16, September 2006 (2006-09), pages 2692-2705, XP005714185 ISSN: 0959-8049
- KANZAWA T ET AL: "Inhibition of telomerase activity in malignant glioma cells correlates with their sensitivity to temozolomide." BRITISH JOURNAL OF CANCER 1 SEP 2003, vol. 89, no. 5, 1 September 2003 (2003-09-01), pages 922-929, XP002481861 ISSN: 0007-0920
- HAAS-KOGAN DAPHNE A ET AL: "Epidermal growth factor receptor, protein kinase B/Akt, and glioma response to erlotinib." JOURNAL OF THE NATIONAL CANCER INSTITUTE 15 JUN 2005, vol. 97, no. 12, 15 June 2005 (2005-06-15), pages 880-887, XP002481862 ISSN: 1460-2105
- LIM MARK J ET AL: "Inflammation-induced subcellular redistribution of VE-cadherin, actin, and gamma-catenin in cultured human lung microvessel endothelial cells" MICROVASCULAR RESEARCH, vol. 62, no. 3, November 2001 (2001-11), pages 366-382, XP002441006 ISSN: 0026-2862
- GLASS R ET AL: "P2X4 and P2X6 receptors associate with VE-cadherin in human endothelial cells" CMLS CELLULAR AND MOLECULAR LIFE SCIENCES, vol. 59, no. 5, May 2002 (2002-05), pages 870-881, XP002427838 ISSN: 1420-682X
- BIANCHI CESARIO ET AL: "Biochemical and structural evidence for pig myocardium adherens junction disruption by cardiopulmonary bypass" CIRCULATION, vol. 104, no. 12 Supplement, September 2001 (2001-09), pages I-319, XP002427839 ISSN: 0009-7322
- MARTIN T A ET AL: "Matrix-bound fibroblasts regulate angiogenesis by modulation of VE-cadherin" EUROPEAN JOURNAL OF CLINICAL INVESTIGATION, vol. 31, no. 11, November 2001 (2001-11), pages 931-938, XP002427840 ISSN: 0014-2972
- LAMPUGNANI MARIA GRAZIA ET AL: "VE-cadherin regulates endothelial actin activating Rac and increasing membrane association of Tiam." MOLECULAR BIOLOGY OF THE CELL APR 2002, vol. 13, no. 4, April 2002 (2002-04), pages 1175-1189, XP002481863 ISSN: 1059-1524
- HERREN BARBARA ET AL: "Cleavage of beta-catenin and plakoglobin and shedding of VE-cadherin during endothelial apoptosis: Evidence for a role for caspases and metalloproteinases" MOLECULAR BIOLOGY OF THE CELL, vol. 9, no. 6, June 1998 (1998-06), pages 1589-1601, XP002441008 ISSN: 1059-1524
- ANONYMOUS: "Human sVE-cadherin ELISA Cat. No. KT-031" KAMIYA BIOMEDICAL COMPANY PRODUCT INFORMATION, [Online] pages 1-13, XP002441014 Retrieved from the Internet: URL:http://www.kamiyabiomedical.com/pdf//K T-031.pdf>
- WROBEL T ET AL: "sVE-cadherin and sCD146 serum levels in patients with multiple myeloma." CLINICAL AND LABORATORY HAEMATOLOGY FEB 2006, vol. 28, no. 1, February 2006 (2006-02), pages 36-39, XP002427842 ISSN: 0141-9854 cited in the application
- DOME BALAZS ET AL: "Identification and clinical significance of circulating endothelial progenitor cells in human non-small cell lung cancer." CANCER RESEARCH 15 JUL 2006, vol. 66, no. 14, 15 July 2006 (2006-07-15), pages 7341-7347, XP002481864 ISSN: 0008-5472
- LAMSZUS KATRIN ET AL: "Inhibition of glioblastoma angiogenesis and invasion by combined treatments directed against vascular endothelial growth factor receptor-2, epidermal growth factor receptor, and vascular endothelial-cadherin." CLINICAL CANCER RESEARCH : AN OFFICIAL JOURNAL OF THE AMERICAN ASSOCIATION FOR CANCER RESEARCH 1 JUL 2005, vol. 11, no. 13, 1 July 2005 (2005-07-01), pages 4934-4940, XP002481865 ISSN: 1078-0432
- LIAO FANG ET AL: "Selective targeting of angiogenic tumor vasculature by vascular endothelial-cadherin antibody inhibits tumor growth without affecting vascular permeability" CANCER RESEARCH, vol. 62, no. 9, 1 May 2002 (2002-05-01), pages 2567-2575, XP002427841 ISSN: 0008-5472
- STEPHEN TATTER, MD, PHD: 'The new WHO clasification of tumors affecting the central nervous cystem', [Online] 2006, Retrieved from the Internet: <URL:http://neurosurgery.mgh.harvard.edu/ne wwhobt.htm> [retrieved on 2010-03-24]

## Description

### FIELD OF THE INVENTION

The invention generally relates to soluble proteins, more particularly to circulating proteins. The invention notably provides improved means, including methods and kits, for the detection of such soluble or circulating proteins.

The improved methods and kits of the invention notably enabled to determine that circulating VE-cadherin can be used as a marker of sensitivity or resistance to anti-tumoral treatment, more particularly as a predictive marker of sensitivity or resistance to anti-tumoral treatment.

The invention thus also generally relates to circulating VE-cadherin, for use as a predictive marker of sensitivity or resistance to anti-tumor treatment, as well as to a method for identifying a predictive marker of sensitivity or resistance to anti-tumor treatment.

### BACKGROUND OF THE INVENTION

Biologically-related fluids, such as fluidic culture medium or the fluids of a multicellular organism, often contain soluble proteins, i.e., proteins that are not contained within a cell, nor transmembranarly attached thereto.

In many medically-relevant fields, such as the medical field, accurate detection of such soluble proteins is needed. It is notably the case in the field of the neoplasm diseases (tumors), wherein it would be desirable to identify reliable markers of susceptibility to a neoplasm disease or condition, or of the developmental stage of such a neoplasm disease or condition, or of sensitivity or resistance to anti-tumor treatment.

Such soluble proteins notably comprise those proteins, which are involved in angiogenesis more particularly in endothelial cell survival or death, and which can naturally occur in soluble form, either by secretion, excretion, cleavage or other kind of extraction or truncation from the cell, notably from the endothelial cell.

Such angiogenesis-related soluble proteins notably comprise:
- the soluble proteins, which are angiogenic activators, more particularly angiogenic growth factors, such as vascular growth factors (VGF), vascular endothelial growth factor (VEGF), basic fibroblast growth factor (bFGF), angiopoietin-1, as well as the soluble forms of the receptors of such angiogenic growth factors, such as sVEGF-R2,
- the proteins, which are involved in the adhesion of endothelial cells (ECs) to the extracellular matrix or in intercellular adhesion of ECs, such as cadherins (including N-cadherin, E-cadherin, P-cadherin, H-cadherin, VE-cadherin) and integrins, and which can occur in soluble form,
- the soluble proteins, which are angiogenic inhibitors, such as endostatin, angiostatin, thrombospondin-1.

Different methods and kits are available in the prior art for the detection of such soluble proteins (see e.g., Derycke et al. 2006 Int. J. Cancer 119:2895-2900, Billion et al. 2006 Skin Pharmacol Physiol 19:65-70).

For example, detection of circulating VE-cadherin contained in serum or plasma is usually made using the ELISA kit for detection of human soluble VE-cadherin (human sVE-cadherin), which is commercially available from Bender MedSystems GmbH (Campus Vienna Biocenter 2; A-1030 Wien; Austria) under reference BMS253.

According to the manufacturer's instructions, human serum or plasma is directly placed into the wells of the ELISA microplaque, at a ratio of 20 µL per well. Each of the sample wells also receives 80 µL of a sample diluent, which is disclosed as containing a preservative (Proclin300, i.e., 5-chloro-2-methyl-thiazol-3-one; 2-methylthiazol-3-one), but no surfactant.

Such a procedure has been followed in e.g., Soeki et al. (Soeki et al. 2004, Circ. J. 68(1):1-5).

US2006/0199231 discloses methods for assessing the likehood of response or resistance to an anti-angiogenic treatment wherein the level of a protein complex formed between a first and a second cell component is measured, one of the component being selected in a group including VE-cadherin (§ [0015]).

Lim et al., 2001 (Microvascular Res, 62 :366-382) discloses that cytoskeletal and junctional elements, such as VE-cadherin, are significantly rearranged in cultured Human Lung Microvessel Endothelial cells in response to thrombin. It also discloses anti-VE-cadherin antibodies and a buffer containing at least one surfactant.

Glass et al., 2002 (CMLS Cell Mol Life Sci, 59:870-881) discloses that VE-cadherin interacts with the P2X₄ and P2X₆ receptors in human umbilical vein endothelial cells (HUVECs) at the level of endothelial intercellular junctions. It also discloses polyclonal antibodies raised in goat for VE-cadherin and an extraction buffer containing at least one surfactant.

Bianchi et al., 2001 (Circulation, 104:319-324) investigates the steady state of adherens junctions protein cadherins, such as VE-cadherin, in a cardiopulmonary bypass (CPB) model before and after perfusion. It is disclosed that the levels of immunoreactive VE-cadherin did not significantly change before and after post-CPB perfusion. It also discloses a VE-cadherin goat polyclonal antibody and a lysis buffer containing at least one surfactant.

Martin et al., 2001 (Eur J Clin Invest, 31:931-938) discloses that VE-cadherin is crucial for the assembly of vascular tubes, implicating VE-cadherin as a mediator vital to angiogenic responses, and that matrix-bound fibroblasts modulate the expression and distribution of VE-cadherin. It also discloses a monoclonal mouse anti-VE-cadherin antibody and a buffer containing at least one surfactant.

Lampugnani et al., 2002 (Mol Biol Cell, 13:1175-1189) describes that VE-cadherin expression influences endothelial cells shape and that this effect is accompanied by reorganization of actin stress fibers and associated vinculin positive adhesion plaques. It also discloses an anti-VE-cadherin antibody and a lysis buffer containing at least one surfactant.

Herren et al., 1998 (Mol Biol Cell, 9: 1589-1601) examined modifications to adherens junctions components that occurs during growth factor deprivation-induced apoptosis of human umbilical vein endothelial cells (HUVEC). The authors demonstrated that during endothelial apoptosis there is disruption of the adherens junction complex, notably including metalloproteinase-mediated shedding of VE-cadherin. It also discloses a monoclonal anti-VE-cadherin antibody and a buffer containing at least one surfactant.

The instruction leaflet Human sVE-cadherin ELISA Kit from Kamiya Biomedical company (version 29-09-2005) discloses that specified cell adhesion molecules, such as VE-cadherin, are involved in the subsequent events of endothelial cell differentiation, apoptosis, and angiogenesis. It also discloses a Biotin-conjugate concentrate anti-VE-cadherin antibody and a wash buffer concentrate containing at least one surfactant.

The present invention provides improved means for the detection of such soluble proteins, more particularly such angiogenesis-related soluble proteins, which are much more accurate and reliable than prior art means. The means of the invention notably comprises the fact of diluting the fluid, or a sample thereof, in a surfactant-containing solution, prior to proceeding to protein detection. The means of the invention take into account the fact

that such fluids are often lipemic samples, wherein reliable detection of soluble proteins is impaired by lipidic and/or saccharidic entities being associated to the soluble proteins.
To the best of the inventor's knowledge, prior art means, at least in the filed of blood-derivable fluids, such as serum or plasma, did not take into account the problem of the presence of lipidic and/or saccharidic entities, and did not address this problem.
The means of the invention are intended to give an accurate and performing technical solution to this problem.

The improved means of the invention is notably adapted to the detection of circulating VE-cadherin.
The improved method of the invention enabled the inventors to determine that the level of circulation VE-cadherin is correlated to the patient's potential to be responsive (R) or non-responsive (NR) to anti-tumor therapy. More precisely, the invention enabled to determine that the lower the level of circulating VE-cadherin in the plasma or serum of a cancer patient, the higher the probability of said patient being resistant or not-responsive to anti-tumor treatment (e.g., chemotherapy and/or radiotherapy).

Illustrative of the performances of the invention is that such a correlation cannot be detected using the commonly-used prior art kit (human soluble VE-cadherin ELISA kit, available from Bender Medsystems under product reference BMS253); *cf.* comparative example 6 below.

To the best of the inventors' knowledge, it is the first disclosure that circulating VE-cadherin can be correlated to sensitivity or resistance to anti-tumour treatment, and it is believed that prior art technique did not enable to observe such a correlation.

For example, in the context of Multiple Myeloma (MM), which affects the plasma cells mainly in the bone marrow, Wrobel et al. 2006 (Clin. Lab. Haem. 28:36-39) report on the levels of soluble VE-cadherin that are measured with said prior art sVE-cadherin kit in the serum of MM patients at diagnosis or after chemotherapy.

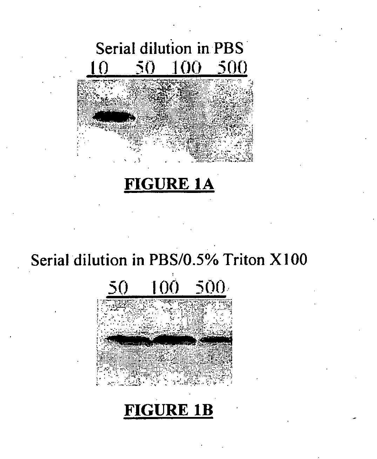

Figure 1 of said Wrobel *et al.* publication shows that sVE-cadherin level as measured by said prior art kit is lower in the healthy controls than in untreated MM patients at diagnosis, and that this sVE-cadherin level is also lower in the MM patients after chemotherapy that in untreated MM patients at diagnosis.

The authors conclude that "*s VE-cadherin (...) was increased in untreated MM patients and decreases after chemotherapy in patients in partial remission"* (see page 39, first sentence). Such a disclosure cannot be read as suggesting the correlation identified by the present inventors, i.e., that a low level of VE-cadherin in serum is a bad prognosis indicator (indicator of resistance to therapy).
This publication does not disclose, and does not contain any element, which would suggest that the fact that a MM patient has a sVE-cadherin level that is lower than the one of healthy individuals, would enable to predict that said MM patient will be resistant to chemotherapy.
The authors recognize that they *"were unable to find any significant correlations except a positive correlation between sVE-cadherin serum level and percentage of bone marrow plasma cells"* (*cf*. Wrobel *et al.,* cited supra, on page 38, right-hand column, last paragraph).
As a matter of fact, such a disclosure does not discriminate between chemo-resistant and chemo-sensitive patients, and can therefore not be interpreted as suggesting any kind of correlation between serum VE-cadherin content and chemo-resistance/chemo-sensitivity status.
It may also be noted that said Wrobel *et al.* publication does not report on the stomach content of the patients and of the healthy individuals (*cf.* table 1 in page 37), whereas, as illustrated in example 7 below, the present inventors believe that sVE-cadherin is associated with lipid entities, from which they should be diossociated to obtain a reliable measure of the sVE-cadhererin content.
Hence, said Wrobel et al. 2006 publication illustrates that the prior art sVE-cadherin kit cannot lead to the findings of the present invention, and that prior art measures of sVE-cadherin levels are not reliable measures.

Hence, to the best of the inventors' knowledge, it has never been described that circulating VE-cadherin of a tumor patient is a reliable tool for predicting whether the patient will be resistant or sensitive to anti-tumor therapy. In fact, the inventors believe that prior art VE-cadherin detection methods impaired such a finding.

The different aspects of the invention do not have the drawbacks of the prior art techniques, and further show unexpected advantages.
The improved methods of the invention are easier to achieve and/or to handle than the prior art methods. They are also much more accurate and much more reliable. As such,
the invention is directly applicable in routine to diagnosis and other medically-related determination.
The improved methods of the invention enable to identify prognosis tools that could not be detected by the prior art methods.
The invention therefore also relates to circulating VE-cadherin, as marker of sensitivity or resistance to anti-tumor treatment.

### SUMMARY OF THE INVENTION

In its broadest sense, the invention pertains to subject-matter as defined in the appended claims. Disclosed herein are improved means, which enable to accurately detect, optionally quantify, soluble proteins, more particularly soluble angiogenesis-related proteins, which can be found to be naturally-contained in a fluid, such as a patient's fluid (e.g., an extracellular fluid, such as blood), or in a fluid derivable from a patient's fluid (such as plasma, serum). Said soluble proteins preferably are soluble proteins, which are involved in angiogenesis and/or inflammation, more preferably in angiogenesis, still particularly soluble proteins, which are involved in endothelial cell survival or death. Examples of such soluble proteins notably comprise cadherins, more particularly VE-cadherin, which can occur in soluble form, e.g., in a fluid such as blood, plasma or serum.
The invention notably comprises the fact of prior diluting the fluid sample in a diluent solution that contains at least one surfactant, before implementing the protein detection/quantification procedure.
Advantageously, said dilution step comprises stirring (preferably, vortexing) the diluted fluid, and/or serially diluting the fluid sample up to the desired dilution factor, and/or diluting the fluid sample by dilution factor of at least 1/50.
Advantageously, said surfactant-containing diluent solution does not comprise Ca or Mg, and/or comprises at least one anti-protease agent, such as leupeptin.
Advantageously, said dilution is performed at a temperature of 0-10°C, preferably at a temperature of 3-5°C, more preferably at 4°C. Therefore, said surfactant advantageously is a surfactant, which does not precipitate at such a temperature.
Advantageously, said surfactant is not a denaturing surfactant (e.g., it is advantageously not SDS).

The improved means disclosed herein notably allow a far more sensitive detection of soluble proteins, more particularly of angiogenesis-related and/or inflammation-related soluble proteins, preferably angiogenesis-related soluble proteins, still more particularly of soluble proteins involved in endothelial cell survival or death, and notably of circulating cadherins, such as circulating VE-cadherin. This result is notably illustrated by Figures 1A and 1B: Figure 1B shows that circulating VE-cadherin is clearly detected in western blot, when the serum has been prior diluted at 1/500 in a buffer containing a surfactant (Triton® X-100 at 0.5% in PBS), whereas Figure 1A shows that VE-cadherin is not detected when the serum has been prior diluted at 1/500 in a buffer that does not contain any surfactant (PBS only).
The improved means further allow a far more reliable quantitative measure of soluble proteins, more particularly of soluble angiogenesis-related and/or inflammation-related proteins, preferably angiogenesis-related soluble proteins, still more particularly of soluble proteins involved in endothelial cell survival or death, such as soluble cadherins, e.g., sVE-cadherin. The present invention notably allows to perform reliable ELISA assays of quantitative performance (*cf*. e.g., example 4 below for a quantitative sVE-cadherin ELISA assay).
Accordingly, the invention notably relates to methods for the detection of soluble proteins, more particularly of soluble angiogenesis-related and/or inflammation-related proteins, preferably angiogenesis-related soluble proteins, still more particularly of soluble proteins involved in endothelial cell survival or death, i.e the soluble cadherins sVE-cadherin, as well as to a VE-cadherin fragment, for use as a standard in the detection of sVE-cadherin, and to the use of at least one surfactant for diluting a fluid sample, prior to implementing the protein detection procedure.

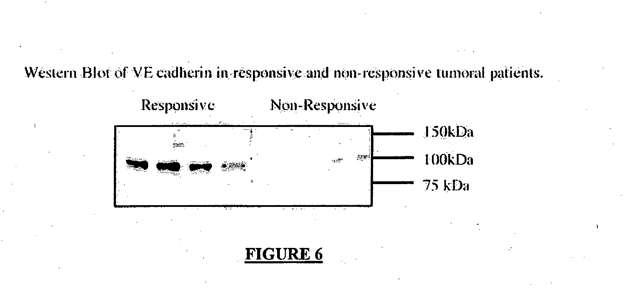

The improved detection/quantification means further enabled to determine that circulating VE-cadherin is a reliable predictive marker of sensitivity or resistance to anti-tumor treatment (*cf.* e.g., examples 2 and 6 below). More particularly, a VE-cadherin content that is significantly lower than the mean VE-cadherin content that is observed in comparable fluids of healthy volunteers, is indicative of the patient being at high risk of being resistant to anti-tumor treatment (see e.g., Figure 6).
It is demonstrated that such a correlation could not be detected using the prior art sVE-cadherin kit.
The disclosure accordingly also relates to VE-cadherin as a predictive marker of sensitivity or resistance to anti-tumor treatment, and to methods involving or comprising compounds that bind to VE-cadherin.

### BRIEF DESCRIPTION OF THE FIGURES

**Figures 1A and 1B****: dilution of serum samples in a surfactant-containing buffer improves VE-cadherin detection, compared to dilution in a surfactant-free buffer.** Figures 1A and 1B show western-blots of the sera of healthy human volunteers (provided by the *Établissement Français du Sang*) for detection of circulating VE-cadherin.
   The serum samples are diluted in a buffer, prior to preparation for SDS-PAGE under denaturing and reducing conditions:
   Figure 1A: serial dilution of serum samples in a buffer (Ca- and Mg-free PBS, containing 25 µg/mL leupeptin and 1mM EGTA), which does not contain any surfactant (first lane, dilution 1 to 10; second lane, dilution 1 to 50; third lane, dilution 1 to 100 - serial dilution 1 to 10, and then 1 to 10-; fourth lane, dilution 1 to 500 -serial dilution 1 to 10, and then 1 to 50-);
   Figure 1B: serial dilution of serum samples in a buffer (same PBS buffer as in Figure 1A), into which a surfactant has been added (0.5% (w/v) Triton® X-100): first lane, dilution 1 to 50; second lane, dilution 1 to 100 - serial dilution 1 to 10, and then 1 to 10-; third lane, dilution 1 to 500 -serial dilution 1 to 10, and then 1 to 50-.
   The circulating VE-cadherin that is contained in human serum is detected on western blot in the form of a 90 kDa form (cleaved or otherwise truncated form of human VE-cadherin).

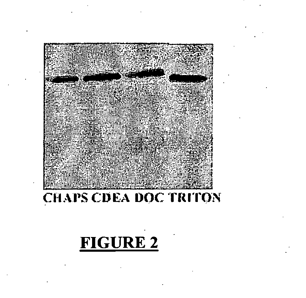

**Figure 2****: assay of different non-denaturing surfactants (Triton® X-100, CHAPS, CDEA, DOC).**
   Figure 2 shows a VE-cadherin western-blot of serum samples collected from healthy human volunteers (provided by the *Établissement Français du Sang*), and diluted (1 to 10, and then 1 to 50) in a buffer (Ca- and Mg-free PBS, containing 25 µg/mL leupeptin and 1mM EGTA), into which a surfactant has been added at 0.5% (w/v).
   First lane, detergent CHAPS (zwitterionic); second lane, detergent CDEA (cationic); third lane, detergent DOC (anionic); fourth lane, detergent Triton® X-100 (non ionic).
**Figures 3A, 3B****: assay of Triton® X-100 at different concentrations, and comparative assay with Tween® 20 or SDS as surfactant in the dilution buffer.**

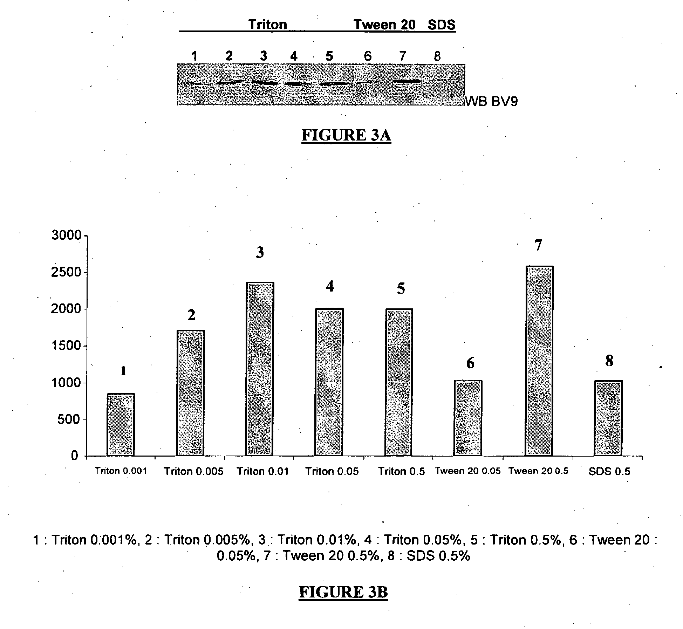

   Figure 3A shows an illustrative western blot analysis of human serum (healthy volunteers) pre-diluted at 1/500 (serial dilution -1/10 and then 1/50-) in a buffer (Ca- and Mg-free PBS, containing 25 µg/mL leupeptin and 1mM EGTA), into which a surfactant has been added as follows:
   - Triton® X-100 (lane 1: Triton® X-100 0.001%, lane 2: Triton® X-100 0.005%, lane 3: Triton® X-100 0.01%, lane 4: Triton® X-100 0.05%, lane 5: Triton® X-100 0.5%), or
   - Tween® 20 (lane 6: Tween® 20 at 0.05%; lane 7: Tween® 20 at 0.5%), or
   - SDS (lane 8: SDS 0.5%).
   Figure 3B shows the scan of the immunoreactive bands obtained in Figure 3A (ordinate axis: arbitrary unit).

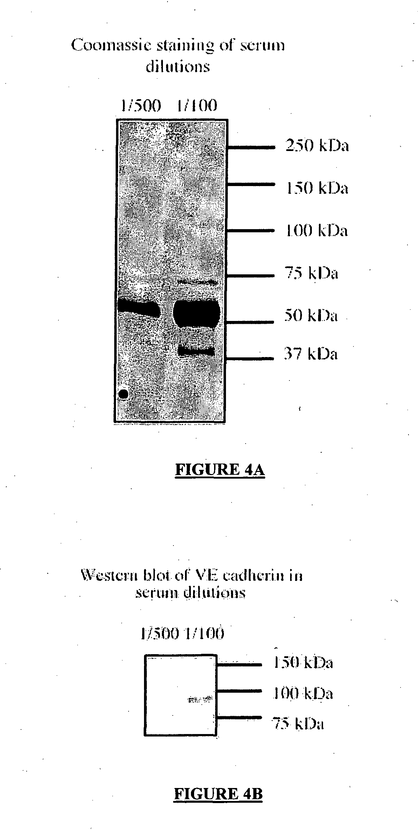

**Figures 4A and 4B****:** analysis of **healthy human serum,** showing the presence in human serum of a 90 kDa immunoreactive band corresponding to truncated VE-cadherin; Figure 4A: Coomassie staining of serum dilutions; Figure 4B: western blot of VE-cadherin in serum dilutions.
   Human serum samples were serially diluted at 1 to 500, in a surfactant-containing buffer (Triton® X-100 at 0.5% in Ca- and Mg-free PBS, containing 25 µg/mL leupeptin and 1mM EGTA), and were then mixed with the sample buffer for SDS-PAGE under denaturing and reducing conditions.

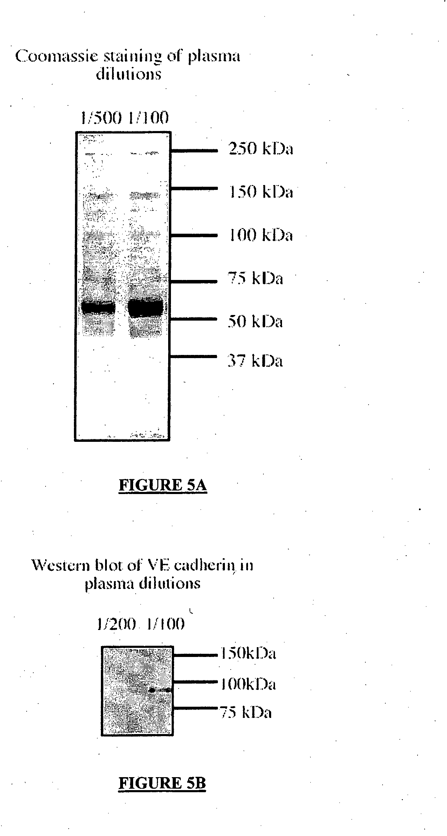

**Figures 5A and 5B****:** analysis of **healthy human plasma,** showing the same pattern of proteins as in serum; Figure 5A: Coomassie staining of plasma dilutions; Figure 5B: western blot of VE-cadherin in plasma dilutions. Human plasma samples were serially diluted at 1 to 500, in a surfactant-containing buffer (Triton® X-100 at 0.5% in Ca- and Mg-free PBS, containing 25 µg/mL leupeptin and 1mM EGTA), and were then mixed with the sample buffer for SDS-PAGE under denaturing and reducing conditions.
**Figure 6****:** western blot analysis of the **serum of glioblastoma patients (collected before any therapy),** showing that the 90 kDa form of human VE-cadherin (truncated extracellular fragment) is highly detectable in the serum of those patients, who will be responsive to chemotherapy and/or radiotherapy, whereas it is almost undetectable in the serum of those patients, who will be un-responsive to therapy. Figure 6 shows that the level of truncated extracellular fragment of VE-cadherin that is contained in the serum is a predictive marker of the sensitivity that the patient will show in response to the chemotherapy and/or radiotherapy of a tumor that is located in its brain. Serum of glioblastoma patients were serially diluted at 1/500 in a detergent-containing buffer (Triton® X-100 0.5% in Ca- and Mg-free PBS, containing 25 µg/mL leupeptin and 1mM EGTA), and were then mixed with the sample buffer for SDS-PAGE under denaturing and reducing conditions.

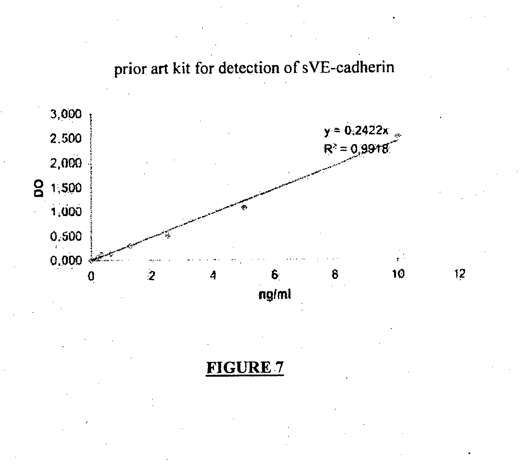

**Figure 7****:** standard VE-cadherin solutions analyzed with the **commercially-available ELISA prior art kit** (ELISA VE-cadherin from Bender MedSystems, Germany, reference BMS253): straight regression line of OD as a function of VE-cadherin content in ng/mL of serum.

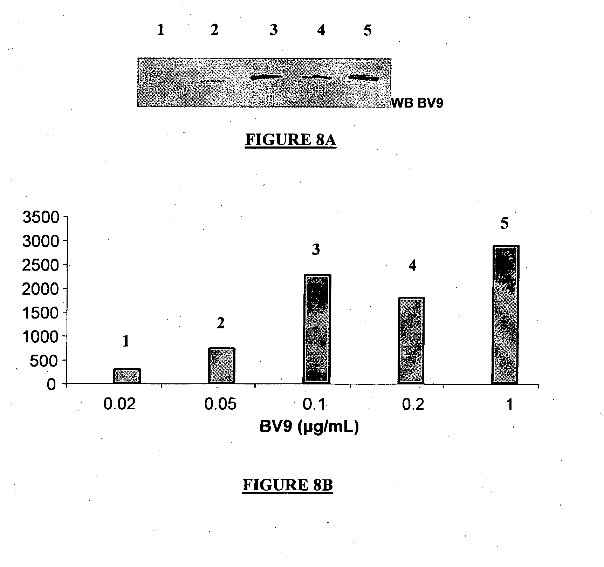

**Figures 8A, 8B****:** serum of human healthy volunteer diluted at 1/500 (serial dilution at 1/10, then 1/50) in Ca- and Mg-free PBS, containing 25 µg/mL leupeptin, 1mM EGTA and 0.5% of Triton® X-100, analyzed by western blot under reducing and denaturing conditions (Figure 8A), using an anti-VEcadherin mAb (BV9 mAb) at 0.02 µg/mL (lane 1), 0.05 µg/mL (lane 2), 0.1 µg/mL (lane 3), 0.2 µg/mL (lane 4) or 1 µg/mL (lane 5).
   Figure 8B shows the scan of the immunoreactive bands of Figure 8A (ordinate axis: arbitrary unit).

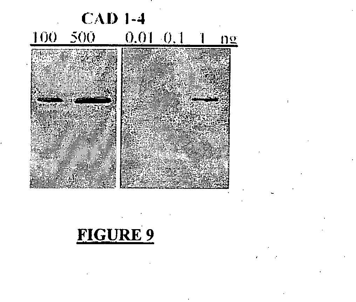

**Figure 9****: a recombinant fragment of the human VE-cadherin extracellular region,** designated VE-EC1-4 or CAD1-4 (Asp48-Glu478 fragment of human VE-cadherin) migrate with an apparent molecular weight of about 50 kDa in western blot with the BV6 mAb; the lowest detectable quantity is of 1 ng of said fragment.

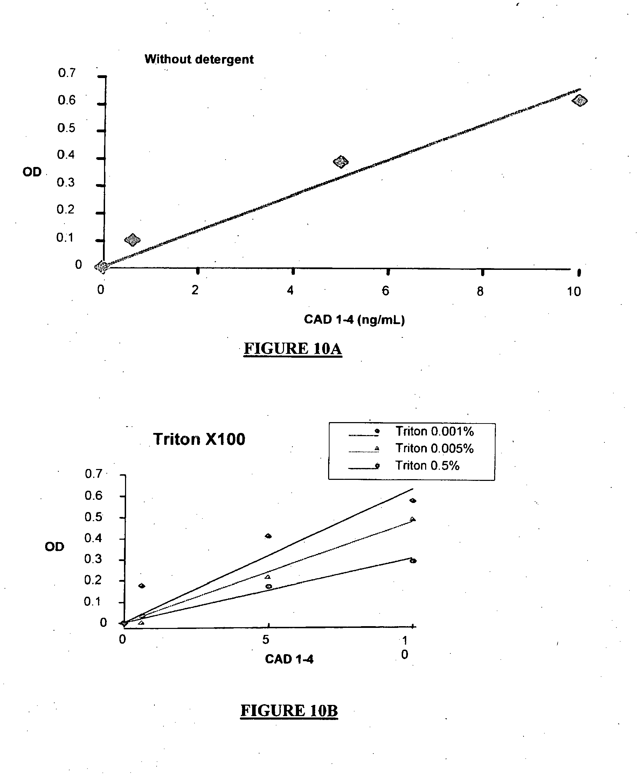

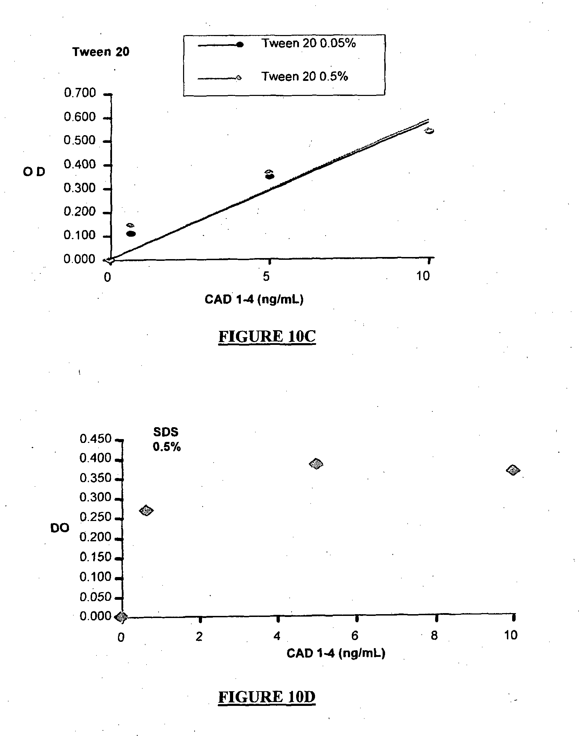

**Figures 10A, 10B****,** **10C, 10D****:** standard solutions of the VE-cadherin CAD1-4 fragment, analyzed by ELISA (capture Ab= anti-VEcadherin mAb BV9; detection with polyclonal anti-VEcadherin antibody -CAD3 epitope-): straight regression line of OD at 405 nm as a function of VE-cadherin fragment content in ng/mL.
   The standard CAD 1-4 solutions were diluted in a surfactant-free Tris buffer (Figure 10A), or in a Tris buffer, which contains Tween® 20 (0.05% or 0.5%; Figure 10B), or Triton® X-100 (at 0.5% -lower standard curve-; at 0.005% -middle standard curve-; or at 0.001% -top standard curve-; Figure 10C), or SDS (0.5%; Figure 10D). OD for each dilution of standard allows drawing a linear standard curve.

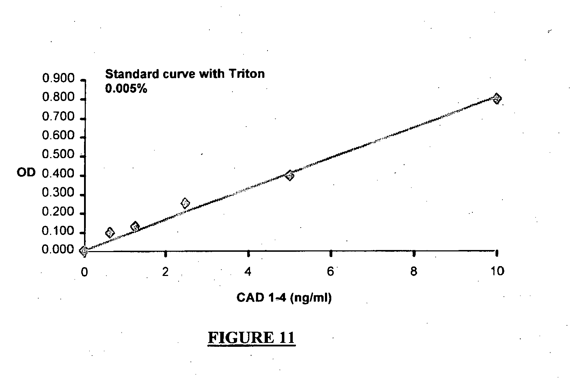

**Figure 11****:** standard solutions of the VE-cadherin CAD1-4 fragment (CAD1-4 fragment of SEQ ID NO:4 in a Tris buffer containing Triton® X-at 0.005% (w/v)), analyzed by ELISA (anti-VEcadherin mAb BV9): linear standard curve of OD at 405nm as a function of VE-cadherin fragment content in ng/mL.

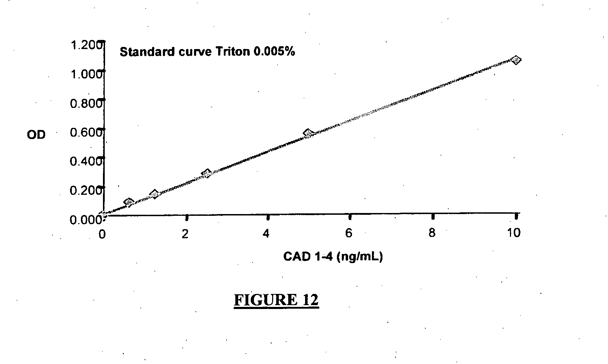

**Figure 12****:** as Figure 11.

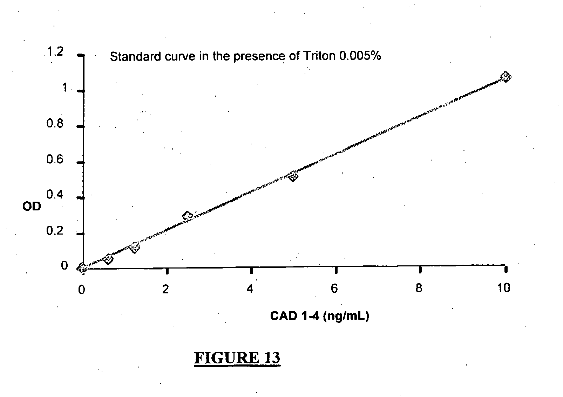

**Figure 13****:** as Figure 11.

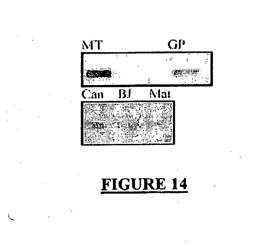

**Figure 14** shows a western blot made in accordance with the invention to analyze the VE-cadherin contained in the serum of patients "GP", "BJ", "Mat", "MT" and "Can"; patients "MT" and "Can" will be responsive to anti-tumor therapy; patients "GP", "BJ", "Mat" will be non-responsive to anti-tumor therapy. Figure 14 illustrates that the invention enables to reliably predict the response the patient will have to anti-tumor treatment.

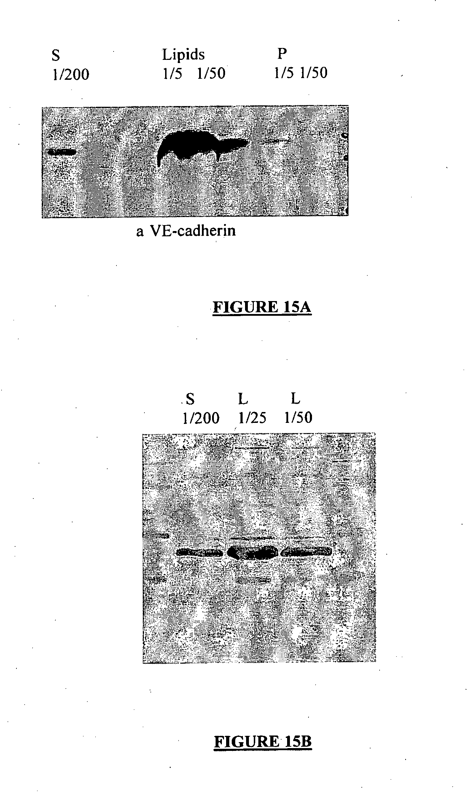

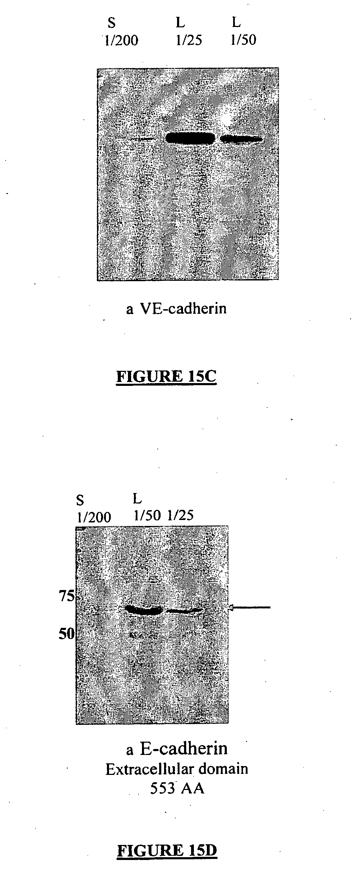

**Figures 15A, 15B****,** **15C, 15D****:** separation of lipidic fraction of serum by ultracentrifugation Figures 15A, 15B, 15C shows that the presence of sVE-cadherin is detected in the serum (S), as well as in the lipid fraction (Lipids, or L) that is obtainable by ultracentrifigation of such a serum and collection of the supernatant fraction, but that said sVE-cadherin is not detectable in the pellet (P).
   sVE-cadherin detection is performed in accordance with the invention, i.e., with dilution of the fluid sample in a (Ca- and Mg-free) PBS buffer containing Triton® X-100 at 0.5%. Dilution rates are as indicated in the figures.
   Note that a 1/25 dilution can be used for the lipidic fraction (Figure 16C).
   Figure 15D shows that the invention enables to detect other circulating proteins, e.g., E-cadherin.

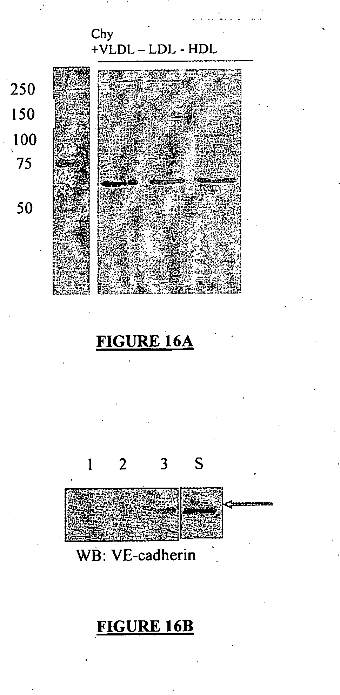

**Figures 16A and 16B****:** extraction of lipoproteins from serum by precipitation
   Figure 16A: lipoproteins were isolated according to the original method of Garcia-Parra M, Mejiade, Gardia M, and Weigandt H ("A new method for lipoprotein isolation by precipitation". In Protides of Biological Fluids., Proceedings of 25th Colloquium Brugge, Editor: H Peeters, Pergamon press, New York, 1977, p. 411-415), and each fraction (i.e. chylomicrons+VLDL, LDL, HDL) were analyzed by Coomassie staining.
   Figure 16B: immunoblot analysis of 5 µL of each fraction with anti-VEcadherin antibody. As a control, 5 µL of the serum (1/200 dilution) were analyzed on the same gel.

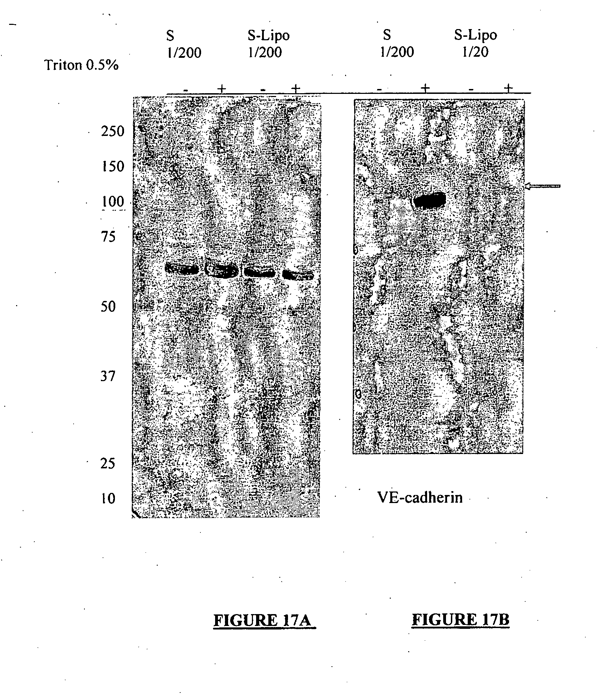

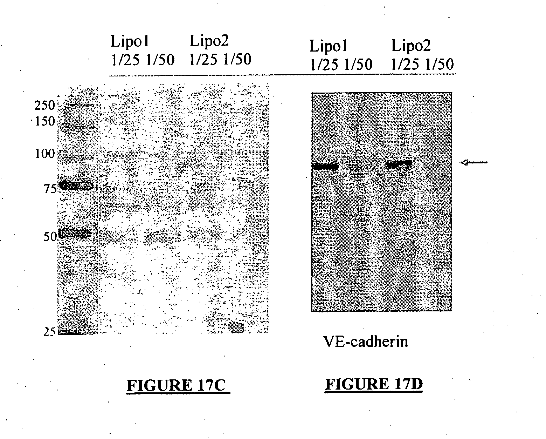

**Figures 17A, 17B****,** **17C, 17D****:** detection of soluble VE-cadherin after separation of lipoproteins
   Serum (50µL; glioma patient, who will be responsive to chemotherapy) was serially diluted 1/200 in the presence or absence of Triton® X-100 0.5% (S 1/200). An aliquot of the same serum was depleted in lipoproteins by precipitation. The supernatant (S-Lipo) was serially diluted 1/200 in the presence or absence of Triton® X-100 0.5%. Five µL of each fraction was analyzed by Coomassie staining (Figure 17A) or western blot (Figure 17B) with the BV9 mAb (0.2 µg/mL).
   After centrifugation of the serum treated with phosphotungstic acid, the pellet was solubilised in PBS/0.5% Triton® X-100, 0.5% DOC; the 1/25 and 1/50 dilutions were analyzed by Coomassie staining and western blotting with BV9. Figure 17C shows the protein patterns of 5µL of each dilution. Figure 17D shows the analysis of the same fractions by westernblotting with the BV9 mAb.

### DETAILED DESCRIPTION

The invention notably relates to means for predicting the resistance or sensitivity of a patient to anti-tumoral treatment. Also disclosed herein are
- means for detecting, optionally quantifying, soluble proteins, and means which directly derives from such detection means;
- means for dissociating soluble proteins from lipidic and/or saccharidic entities that may be associated thereto.

Throughout the application, said soluble proteins preferably are the soluble forms of proteins, which are involved in angiogenesis and/or inflammation, more preferably in angiogenesis, more particularly in endothelial cell survival or death. Examples of such proteins are given below, and notably comprise cadherins, more particularly E-cadherin or VE-cadherin, which can be found to be naturally-occurring in soluble form, e.g., in a fluid such as blood, plasma or serum.

The means for detecting, optionally quantifying, soluble proteins, more particularly soluble angiogenesis-related proteins, such as soluble VE-cadherin, notably allow a far more sensitive detection of circulating VE-cadherin.
This result is notably illustrated by Figures 1A and 1B: Figure 1B shows that circulating VE-cadherin is clearly detected when the serum has been prior diluted at 1/500 in a buffer containing a surfactant (Triton® X-100 at 0.5% in PBS), whereas Figure 1A shows that VE-cadherin is not detected when the serum has been prior diluted at 1/500 in a buffer that does not contain any surfactant (PBS only).

The means for detecting, optionally quantifying, soluble VE-cadherin enabled the inventors to determine that fluid-contained VE-cadherin is a reliable marker for predicting the resistance or sensitivity of a patient to anti-tumoral treatment.
Figure 6 is an illustration of the accuracy of the means of the invention, in predicting resistance or sensitivity to anti-tour treatment.

To the best of the inventors' knowledge, prior art kits for the detection of soluble VE-cadherin were not sufficiently accurate to enable such a finding.
Illustrative of the performances of the methods disclosed herein is that such a correlation cannot be detected using the commonly-used prior art kit (human soluble VE-cadherin ELISA kit, available from Bender Medsystems under product reference BMS253); *cf.* example 6 below.

The inventors further believe that the means for detecting, optionally quantifying, soluble proteins, more particularly soluble angiogenesis-related proteins, such as soluble VE-cadherin, allow dissociating said proteins from lipids that may be associated thereto (*cf.* example 7).

Therefore, also described herein are:
- a method for detecting and/or quantifying soluble proteins, more particularly soluble angiogenesis-related proteins, still more particularly soluble cadherins, preferably soluble E- or VE-cadherin,
- a VE-cadherin fragment of SEQ ID NO: 4, for use as a standard in a VE-cadherin quantification assay, as well as any sub-fragment thereof, which has retained the capacity of being detectable on western blot at a quantity as low as 1 nanogramme,
- a surfactant-containing solution, which can advantageously be used to dilute a fluid - that contains, or is suspected of containing soluble proteins, more particularly soluble angiogenesis-related proteins, still more particularly soluble cadherins, preferably soluble E- or VE-cadherin,
- before contacting the fluid with at least one ligand for the detection of said soluble protein,
- a method for predicting the resistance or sensitivity of an animal or a patient to anti-tumoral treatment,
- an anti-VEcadherin ligand, more particularly an anti-VEcadherin antibody or a conservative variant thereof, for use to predict sensitivity or resistance to anti-tumoral treatment,
- a VEcadherin-related element for the production of such an antibody that is for use to predict sensitivity or resistance to anti-tumoral treatment,
- an anti-VEcadherin ligand (more particularly an anti-VEcadherin antibody or a conservative variant thereof) and a surfactant-containing solution, for use to predict sensitivity or resistance to anti-tumoral treatment,
- kits (ELISA kits, as well as SDS-PAGE/western blot kits), which comprise at least one ligand that binds to VE-cadherin, and at least one solution containing at least one surfactant; as well as the uses of said kits for the prognosis of resistance or sensitivity to anti-tumor therapy, or for detecting and/or quantifying the VE-cadherin that is contained in a fluid, which is the blood or an extracellular fluid of a multicellular organism, or which is a fluid derivable from such a naturally-occurring fluid, such as plasma or serum, and which is the cell-free fluid of a cell- or tissue-culture medium,
- a method for identifying at least one prognostic marker of sensitivity or resistance to anti-tumour treatment, as well as a method for monitoring the efficiency of an anti-tumor treatment,
- a method for dissociating VE-cadherin from lipidic and/or saccharidic entities that may be associated thereto, as well as a surfactant for use to dissociate VE-cadherin from lipids that may be associated thereto.

### Soluble proteins:

As already mentioned above, the invention relates to soluble proteins.

The term "soluble" is herein intended in accordance with its usual meaning in the field of biology. Schematically, a protein is termed to be soluble, when it is contained in a fluid, and wherein, within said fluid, said protein is not contained within a cell, nor transmembranarly attached thereto. Soluble protein hence notably comprises proteins which have secreted or excreted from a cell, or cleaved from the cell membrane, or otherwise truncated or extracted from the cell.
For example, a protein can be soluble in a fluidic culture medium, or can be circulating in a biological fluid, such as blood. It can thus be soluble in the plasma or serum derivable from such a blood.
A protein may naturally occur in both cellular (e.g., transmembranar) form and soluble form. The soluble form may e.g., be an extracellular fragment of the transmembranar form. The present invention relates to the soluble form of such proteins.

In the present application, the term "protein" is used in its broadest general meaning. Accordingly, the term "protein" also encompasses polypeptides and peptides, as well as any molecular entity, which comprises at least one protein or polypeptide or peptide.

The inventors demonstrate that such proteins are likely to be somehow associated with lipids, whereby their reliable detection is altered, and observed that most prior art means for the detection of such soluble proteins, did not take into account this fact, notably in the field of the detection of soluble proteins contained in blood-derivable fluids, such as serum or plasma.

The inventors indeed observed that under prior art practice, the lipemic status of the sample collected from a patient is rarely taken into account, and that, in most circumstances, there is time available to wait for the patient having a reduced lipemic status to make the analysis. Hence, the lipemic issue is most of the time overlooked, as if it were a secondary issue, without any significant impact on the reliable detection of soluble proteins.
The inventors demonstrate that the issue of lipid entities is not a secondary issue, and that overlooking it may lead to inaccurate protein evaluation. See e.g., comparative example 6

The inventors demonstrate that this is notably the case for the detection of soluble angiogenesis-related proteins.
Such soluble proteins notably comprise those proteins, which are involved in angiogenesis, and which can naturally occur in soluble form, either by secretion, excretion, cleavage or other kind of extraction or truncation from the cell, notably from the endothelial cell.
Such angiogenesis-related soluble proteins notably comprise:
- the soluble proteins, which are angiogenic activators, such as angiogenic growth factors, such as vascular growth factors (VGF), vascular endothelial growth factor (VEGF), basic fibroblast growth factor (bFGF), angiopoietin-1, as well as the soluble forms of the receptors of such angiogenic growth factors, such as sVEGF-R2,
- the soluble forms of the proteins, which are involved in the adhesion of endothelial cells (ECs) to the extracellular matrix or in intercellular adhesion of ECs, such as cadherins (including N-cadherin, E-cadherin, P-cadherin, H-cadherin, VE-cadherin) and integrins,
- the soluble proteins, which are angiogenic inhibitors, such as endostatin, angiostatin, thrombospondin-1.
As a matter of fact, the prior art kits for the detection of such proteins do not deal with dissociating the soluble protein from possible lipid entities.

Hence, in accordance with the disclosure, and throughout the application, preferred soluble proteins are soluble angiogenesis-related proteins.

Throughout the application, preferred soluble angiogenesis-related proteins are VGF, soluble cadherins, and endostatin. More preferred soluble angiogenesis-related proteins are soluble cadherins.

Cadherins are a family of cell adhesion molecules that are involved in the formation of specific cell-cell contacts. They are single chain transmembrane glycoproteins with molecular weights of 120-140 kDa.
The members of the cadherin superfamily are characterized by domains called cadherin domains or EC domains, containing sequence motifs, which are involved in Ca2+ binding. The EC domains are tandemly repeated in the extracellular segment of the cadherin molecule. The extracellular region of classic cadherins contains five EC domains of about 110 amino acids.

The cadherin family notably comprises N-cadherin, E-cadherin, P-cadherin, H-cadherin, and VE-cadherin.

Like other classic cadherins, VE-cadherin mediates calcium-dependent, homophilic adhesion and functions as a plasma membrane attachment site for the actin cytoskeleton. However, VE-cadherin is integrated into signaling pathways and cellular systems uniquely important to the vascular endothelium, and is located specifically at the adherens junctions regions of vascular endothelial cells.
The extracellular domains of the cadherins mediate homophilic binding and adhesion between adjacent cells. Figure 5A of Corada et al. 2001 (Corada et al. 2001, Blood 97(6): 1679-1684) shows the schematic representation of VE-cadherin, illustrating the VE-cadherin extracellular domain (EC) (which consists of five cadherin-like repeats that form a rigid, rod-like structure that is stabilized by the binding of calcium ions), the transmembrane domain (TM), and the short cytoplasmic tail (Vincent et al. 2004, Am. J. Physiol. Cell Physiol. 286: C987-C997).
Human vascular endothelial cadherin (VE-cadherin; 7B4/cadherin-5) is an endothelial-specific cadherin localized at the intercellular junctions. It has been described in WO 98/25946 (IMCLONE Systems; Inventors DEJANA et al.).
The full-length cDNA has been cloned from human endothelial cells, and sequenced (Breviario et al. 1995, Arteriosclerosis, Thrombosis, and Vascular Biology 15: 1229-1239). The nucleotide sequence data are available from EMBL/Genbank (accession number X79981). The human VE-cadherin coding region has been transfected into Chinese hamster ovary cells, leading to the production of a recombinant human VE-cadherin having the same molecular weight as the naturally-occurring VE-cadherin of human endothelial cells (Breviario et al. 1995, Arteriosclerosis, Thrombosis, and Vascular Biology 15: 1229-1239). The amino acid and the nucleotide sequences of human VE-cadherin are available from EMBL/Genbank (amino acid sequence: accession number CAA56306; nucleotide sequence: X 79981).

Unlike most endothelial markers, VE-cadherins are not found in blood cells or in hematopoietic precursors. VE-cadherins are constitutively expressed by the endothelium of most organs and tissues, which suggests that their biologic properties are required for the early assembly and integrity of blood vessels.

In accordance with the invention, and throughout the application, the soluble cadherin is soluble VE-cadherin, more particularly circulating VE-cadherin. Most preferred VE-cadherin is human VE-cadherin.

### Fluid:

The soluble proteins, to which the invention relates, are contained in a fluid.
Said fluid can be essentially biological or essentially chemical.
Said fluid can be a naturally-occurring fluid, or a fluid derivable from a naturally-occurring fluid, or an artificial fluid onto which a living organism or microorganism has or not exerted a metabolic activity.
For example, said fluid can be a fluid collectable from a multicellular organism, or a fluid derivable from such a collectable fluid, or can be a fluidic cell- or tissue-culture medium.

A fluidic cell- or tissue-culture medium is a culture medium in fluid form, which has or has not been inoculated by cells or by a tissue. Such a fluidic culture medium may thus be a fluidic culture medium in its initial composition, before any cell or tissue has contacted it or has exercised any metabolic activity on it Such a fluidic culture medium may alternatively be a fluidic culture medium, the composition of which has been modified due to cells or tissues having grown on or in it, or having exerted a metabolic activity on it. Such a fluidic culture medium can be collected at any time during the cell or tissue growth, including at the end of the culture.
Preferably, such a fluidic culture medium is cell- or tissue-free. If the fluidic culture medium has been contacted by cells or tissues, and/or if cells or tissues have grown on or in it, these cells or tissues are thus preferably removed from it, whereby a cell- or tissue-free, or a substantially cell- or tissue-free culture medium is recovered.
Preferably, such a fluidic culture medium comprises at least one lipid. Said at least one lipid may be part of the initial composition of the medium, or may have been produced as a result of the metabolism of the cells or tissues that have been inoculated in it and/or that have been grown on or in it.
More preferably, such a fluidic culture medium is cell- or tissue-free, and comprises at least one lipid.

Said fluid can alternatively be the fluid of a multicellular organism.

Said multicellular organism can be an animal, preferably a mammal, most preferably a human individual. Said multicellular organism can be a male or a female. Said multicellular organism may or may not have been produced by man, or otherwise genetically engineered through the intervention of man.
A fluid of a multicellular organism can be a fluid that is leaked or excreted from said multicellular organism, or can be a fluid that is internal to said multicellular organism.

Preferably, said fluid collectable from a multicellular organism is an extracellular fluid. Extracellular fluid is any extracellular fluid that may be found in a multicellular organism. Extracellular fluids that are normally observed in a multicellular organism notably comprise blood, the naturally-occurring fluid called plasma, as well as any serous fluid that may be found in a multicellular organism, e.g., interstitial fluid, transcellular fluid, lymph, chyle.

The blood comprises blood formed elements, which are suspended in a fluid called plasma. The blood formed elements consist of blood cells and platelets. Blood cells consist of red blood cells (erythrocytes) and of white blood cells (leukocytes). Blood consists of 55 % plasma, and 45 % blood formed elements. Anatomically, blood is considered to be a connective (circulating) tissue from both its origin in the bones and its function. Blood is nevertheless also considered as a bodily fluid. In the present application, blood is considered to be a fluid.
In a living organism, naturally-occurring blood plasma is forced out of the capillaries of the circulatory system by hydrostatic pressure, whereby it leaks from said capillaries and becomes interstitial fluid, filling the space between individual cells of tissue. Additionally, passive diffusion and active cellular transport from the blood also play a role in fluid production. Interstitial fluid bathes the cells of the tissues. Interstitial fluid provides a means of delivering materials to the cells, intercellular communication, as well as removal of metabolic waste. Interstitial fluid consists of a water solvent containing amino acids, sugars, fatty acids, coenzymes, hormones, neurotransmitters, salts, as well as waste products from the cells. Most of the interstitial fluid is returned to the capillaries by osmosis. The interstitial fluid may also drain into the lymphatic sytem (lymph vessels and lymph organs, such as bone marrow, lymph nodes, spleen, thymus, peyer's patches, tonsils, vermiform appendix), e.g., by diffusion into lymph capillaries, and is processed by lymph nodes prior to being returned to the circulatory system.

Once within the lymphatic system, the interstitial fluid is called lymph, and has almost the same composition as the original interstitial fluid.
Lymph vessels, called lacteals, are present in the lining of the gastrointestinal tract. While most other nutrients absorbed by the small intestine are passed on to the portal venous system to drain, via the portal vein, into the liver for processing, fats are passed on to the lymphatic system, to be transported to the blood circulation via the thoracic duct. The enriched lymph originating in the lymphatics of the small intestine is called chyle.

Other extracellular fluids, which are naturally-occurring in a multicellular organism, and which are encompassed by the present invention, comprise cerebrospinal fluid (CSF), bone marrow, synovial fluid, aqueous humour, vitreous humour, amniotic humour, bile, chime, breast milk, urine, semen, sweat, tears, sebum (skin oil), mucus, pleural fluid, lung fluid.

Extracellular fluids may have a normal composition, i.e., a composition that is not significantly different from the average composition that would be observed in a healthy subject.
Alternatively, these fluids may have an altered composition. This is notably the case when the multicellular organism is affected by certain particular conditions, such as e.g., certain pathological diseases or conditions. Certain pathologies, notably certain neoplasm conditions, may induce the formation of fluids, which are not otherwise observed in a healthy subject. For example, certain cancers may induce the formation of an abnormal fluid, such as e.g., a fluid leaking from the abnormal cell area of a breast cancer.
Hence, said extracellular fluids can be extracellular fluids that are only present in non-healthy organisms. Such pathologically-related extracellular fluids are also encompassed by the present invention.

Fluids that are derivable from at least one extracellular fluid, more particularly from blood, are also encompassed by the present application.
Fluids that are derivable from at least one extracellular fluid notably comprise any fluid that is obtainable by collection of an extracellular fluid, and which has a composition that is slightly different from the initial composition of said extracellular fluid, due to its mode of collection, such as would be the case for an extracellular fluid that is collected by lavage. An example of such a derivable fluid is a wash fluid collected by alveolar or bronchoalveolar lavage of the lung.

Fluids that are derivable from at least one extracellular fluid notably comprise any fluid that is obtainable by collection of an extracellular fluid, and by further processing of the collected extracellular fluid, e.g., by removal of at least one fraction or component of this extracellular fluid, and/or by addition of a component, such as e.g., addition of a component that may be useful for the subsequent analysis of the sample. Any further modification that the skilled person may consider to be useful is encompassed by the present invention. Such "derivable" fluids advantageously comprise blood serum or blood plasma, which are obtainable by centrifugation of a sample of whole blood and removal of the blood cells and platelets.

Blood plasma can be obtained from whole blood by centrifugation. To prevent clotting, an anticoagulant, such as citrate or heparin, is added to the blood specimen at the time of its collection. Usually the anticoagulant is already in the evacuated blood collection tube (e.g. Vacutainer or Vacuette®), when the patient is bled. The specimen is then centrifuged to separate plasma from the blood formed elements. Plasma can be frozen below -20°C nearly indefinitely for subsequent analysis or use. This blood product derivative is known as fresh frozen plasma (FFP).
Blood plasma is a slightly alkaline fluid, with a typical yellowish color. It consists of 90 % water and 10% dry matter. Nine parts of it are made up by organic substances, whereas one part is made up by minerals. These organic substances are composed of glucides (glucose), lipids (cholesterol, triglycerides, phospholipids, lecithin, fats), proteins (globulins, albumins, fibrinogen), hormones (gonadothropins, erythropoietin, thrombopoietin), amino acids and vitamins, as well as various holoproteins, such as lipoproteins, glycoproteins, metalloproteins. The mineral substances are dissolved in ionic form that is dissociated into positive and negative ions.

Serum is the same as plasma, except that clotting factors (such as fibrin or fibronogen) have been removed.

Hence, in the present application, and throughout the application, the term fluid encompasses any of the above-described fluids, and more particularly:
- blood,
- any other extracellular fluid of a multicellular animal (e.g., interstitial fluid, transcellular fluid, lymph, chyle, cerebrospinal fluid (CSF), bone marrow, synovial fluid, aqueous humour, vitreous humour, amniotic humour, bile, chime, breast milk, urine, semen, sweat, tears, sebum (skin oil), mucus, pleural fluid, lung fluid), including any extracellular fluid induced by a pathological or age-related condition,

- a fluid derivable from at least one extracellular fluid of multicellular animal (e.g., a wash fluid collected by alveolar or bronchoalveolar lavage of the lung), more particularly a fluid derivable from blood, such as plasma, serum,
- a fluidic cell- or tissue-culture medium.

### Lipid- and/or saccharide-containing fluids:

Among the various extracellular fluids that the invention encompasses, preferred extracellular fluids for implementation of the invention are those fluids, which contain at least one lipid and/or at least one saccharose.
Said lipid or saccharose may be free lipid or (poly-)saccharose entities, or may be contained in another entity, e.g., in a protein, resulting in a holoprotein, e.g., a lipoprotein or a glycoprotein, respectively.
Hence, preferred extracellular fluids for implementation of the invention are those fluids, which contain at least one lipid (e.g., at least one lipoprotein) and/or at least one saccharose (e.g., at least one glycoprotein).

Among the various extracellular fluids that the invention encompasses, more preferred extracellular fluids for implementation of the invention are those fluids, which contain at least one lipid, e.g., those extracellular fluids, which contain at least one of the following lipids or lipid-containing entities: saturated or unsaturated fatty acids; glycerides, including triglycerides; glycerolipids; phosphoglycerides; glycerophospholipids; sphingolipids; sterol lipids, including cholesterol, LDL cholesterol and VLDL cholesterol; steroid hormones; prenol lipids; glycolipids; lipoproteins; apolipoproteins; and any derivative of at least one of such lipid entities, which differ from said lipid entity by at least one chemical group, but has retained a non-polar or hydrophobic character. Preferred lipids are those selected from the following group: sterol lipids, including cholesterol and LDL cholesterol; glycolipids; lipoproteins saturated or unsaturated fatty acids; glycerides, including triglycerides; and any derivative of at least one of such lipid entities, which differ from said lipid entity by at least one chemical group, but has retained a non-polar or hydrophobic character. In accordance with the present invention, preferred lipid-containing entities are lipoproteins.

Extracellular fluids may contain lipids in various forms. They may contain free lipids, or free lipid-containing compounds. They may contain lipids in the form of vesicles, chylomicrons or micelles.

The lipid content of such an extracellular fluid can be of any lipid composition. It may contain one type of lipid, or it may contain at least two different types of lipids. Such a lipid composition may vary depending on the physiological status of the subject, e.g., the lipid composition of a healthy subject may differ from the one of a subject, which/who is affected by a pathological disease or condition.

A lipid-containing extracellular fluid may be an extracellular fluid, which normally contains lipid, i.e., a fluid, which in a healthy subject, would contain at least one lipid.
The lipid content of such an extracellular fluid can be at any level, i.e., it can be at a sub-normal level, or at a level that is not significantly different from the normal level (i.e., the average lipid content level that would be observed in a healthy subject), or at a level that is significantly higher than the normal level. For example, the plasma of a healthy individual contains lipids; such lipids are at different levels, depending on whether the patient has an empty stomach or not when blood was collected from him/her. The plasma of a hypercholesterolemia patient also contains lipids, but at a much higher level. All these various types of fluids are encompassed by the present invention.

A lipid-containing extracellular fluid may alternatively be an extracellular fluid, which would normally not contain any detectable lipid, but which, under certain circumstances, which could be qualified as being abnormal circumstances, appears to contain at least one lipid. Such abnormal circumstances notably include certain pathological disease or condition. For example, human urine does normally not contain any detectable lipid, or only in trace amounts, but urine of lipiduria patients may contain abnormally high amounts of lipids.

Throughout the application, more preferred fluids are those fluids, which contain at least one lipid, and which are cell-free or substantially cell-free, either because they do not contain any cell *in situ* in the multicellular organism, from which they originate, or because the cells they originally contain have been substantially removed.

### Fluids, which are substantially cell free:

By "substantially" cell-free, it is herein meant that the fluid is considered to be cell-free by the physician of average skill in the art.
For example, blood of a healthy subject contains blood cells and platelets, and is therefore not a cell-free fluid. After centrifugation of whole blood, especially when centrifugation is performed at high speed (e.g., at 2,000-2,800 rpm for 5-10 min), plasma and serum do not contain any blood cell or platelet, and are considered to be cell-free by the physician of average skill in the art (platelets being herein equated to "cell" components). The expert neurologist may however consider that plasma or serum may contain some progenitor cells (e.g., endothelial progenitor cells), and that these fluids are not completely cell-free. In the present application, and in line with the views of the physician of average skill in the art, plasma and serum are nevertheless considered to be a substantially cell-free extracellular fluid, because they do not contain the cells that are constitutive of the cell-containing fluid from which they derive (i.e., they do not contain blood cells and platelets), and in spite of the fact they may contain some other cells (usually in trace amounts, compared to the amounts of blood cells and platelets contained in blood), such as e.g., progenitor cells.
Quite similarly, it may also be noted that, under certain pathological disease or condition, and/or at a certain age of the subject, certain extracellular fluid may contain cells, whereas the healthy or young subject does not have such cells in this fluid. For example, plasma and serum of a healthy subject do not contain any circulating endothelial cells, whereas plasma or serum of a cancer patient, or of a very old subject (e.g., 70 years old or higher, for a human being), may be found to contain some circulating endothelial cells, although usually only in small amounts. Under such circumstances, serum and plasma are still considered to be "substantially cell-free" within the meaning of the present invention, because they do not contain the cells that are constitutive of the cell-containing fluid from which they derive (i.e., they do not contain blood cells and platelets), and in spite of the fact that they may contain some other cells, such as e.g., circulating endothelial cells.
Hence, the term "substantially cell-free" refers to the absence in the "substantially cell-free" fluid (e.g., plasma or serum) of the main cell types that are constitutive of the cell-containing fluid (e.g., blood cells and platelets), from which the "substantially cell-free" fluid is derivable. In other words, plasma and serum are considered to be "substantially cell-free", because the plasma and serum of a healthy subject do not contain blood cells and platelets.

The term "substantially cell-free" may alternatively refer to the situation, where a given extracellular fluid do not contain any cell, when the subject is a healthy subject, but may be found to contain some cells, when the subject becomes affected by a certain pathological disease and/or condition. An example of such a situation is urine, which do not contain any detectable cell, when the urine comes from a healthy subject, but which may be found to contain blood cells in non-healthy subjects. Hence, depending of the physiological status of the subject, urine is not always cell-free. In spite of this fact, urine is herein considered to be a "substantially cell-free" extracellular fluid, even though it may sometimes contains some cells. The term "substantially cell-free" then refers to the fact that the normal status of the fluid is to be cell-free in a healthy subject.
The same understanding is to be made for extracellular fluids, which do not contain any cells, when the subject is healthy and young, but which may be found to contain some cell, when the subject is old (e.g., about 70 years old or older, for a human being).

Throughout the application, most preferred cell-free or "substantially cell-free" extracellular fluids are plasma, serum, as well as any serous fluid that may be found in a multicellular organism, more particularly in a human or animal body, preferably in a mammal, most preferably in a human individual, e.g., interstitial fluid, lymph, chyle.

A cell-containing fluid may be made cell-free, or substantially cell-free, e.g., by filtration and/or precipitation and/or clotting and/or centrifugation of the cellular elements it contains. For example, blood is a cell-containing extracellular fluid, and can be made cell-free, or substantially cell-free, by centrifugation at 2,000-2,800 rpm for 5-10 min, followed by collection of the supernatant (plasma), or by clotting and centrifugation at 2,000-2,800 rpm for 5-10 min, followed by collection of the supernatant (serum).

To implement the invention, a sample of blood or of another extracellular fluid is preferably collected from the subject (e.g., a multicellular organism, more particularly an animal, preferably a mammal, most preferably a human individual). When blood is collected, it is preferred to implement the invention with the plasma or serum sample that is derivable from this blood.

Hence, in the present application, and throughout the application, the term "fluid" encompasses any of the above-mentioned fluids. The term "fluid" more particularly relates to a fluid selected from:
∘ the blood of a multicellular organism,
∘ any other extracellular fluid of a multicellular organism, as well as
∘ the fluids derivable from said blood and extracellular fluids, preferably plasma, serum,
said multicellular organism being preferably an animal, more preferably a mammal, e.g., a non-human mammal or a human individual.
Examples of extracellular fluids other than blood notably comprise the naturally-occurring fluid that is called plasma, as well as interstitial fluid, transcellular fluid, lymph, chyle, cerebrospinal fluid (CSF), bone marrow, synovial fluid, aqueous humour, vitreous humour, amniotic humour, bile, chime, breast milk, urine, semen, sweat, tears, sebum (skin oil), mucus, pleural fluid, lung fluid.
Preferably, said fluid is a lipid- and/or saccharide-containing fluid.
Preferred fluids are those fluids, which are cell-free or substantially cell-free, either because they do not contain any cell *in situ* in the multicellular organism, from which they originate, or because the cells they originally contain have been substantially removed. Preferred fluids are those fluids, which contain lipid- and/or saccharide-entities and, which are cell-free or substantially cell-free.
More preferred fluids are blood, plasma, serum. Most preferred fluids are plasma, serum.

### Surfactant-containing solutions:

In accordance with the invention, the fluid sample is diluted in a diluent medium.
Said dilution of fluid sample can be performed in any diluent medium that the person of average skill in the art may find appropriate.
Such a diluent medium will generally be a solution, preferably an aqueous solution, preferably a solution having a pH of about 6.5-8.5, more preferably a buffer.
Said buffer preferably has a pH of about 6.5 - 8.5.
Said buffer preferably is an isotonic (100 - 150mM salt) buffer, such as a 150 mM NaCl buffer.
More preferably, said buffer preferably has a pH of about 6.5 - 8.5, and is an isotonic (100 - 150mM salt) buffer, such as a 150 mM NaCl buffer, for example Phosphate Buffer Saline (PBS) solution, or Tris Buffer Saline (e.g., 10-50 mM), Hepes Buffer Saline (e.g., 10-50 mM), Pipes Buffer Saline (e.g., 10-50 mM).

According to a preferred embodiment of the invention, said diluent medium is Ca-free and Mg-free.
According to a preferred embodiment of the invention, said diluent medium contains at least one anti-protease agent, e.g., leupeptin, for example leupeptin 25 µg/mL.
According to a preferred embodiment of the invention, said diluent medium is Ca-free and Mg-free, and contains at least one anti-protease agent, e.g., leupeptin, for example leupeptin 25 µg/mL.

According to the invention, the diluent medium further contains at least one detergent, which is
a surfactant (surface active agent).
A surfactant is briefly defined as a molecule, which can reduce the surface tension of a liquid, preferably even when used in very low concentrations. Surfactants constitute the most important group of detergent components.
Surfactant are amphipathic molecules, meaning they contain both a nonpolar (i.e., a hydrophobic) "tail", and a polar (i.e., a hydrophilic) "head". The tail usually has aliphatic or aromatic character.
Ionic character of the polar head group forms the basis for broad classification of surfactants; they may be ionic (charged, either anionic or cationic), nonionic (uncharged) or zwitterionic (having both positively and negatively charged groups but with a net charge of zero).

According to a more preferred embodiment of the invention, said diluent medium:
- is Ca-free and Mg-free, and/or contains at least one anti-protease agent, e.g., leupeptin, for example leupeptin 25 µg/mL, and
- contains at least one detergent, preferably at least one surfactant.

Said surfactant can be a non-ionic surfactant, preferably, a non-ionic surfactant selected from the group consisting of Nonidet P40 (NP-40); octyl glucoside, octylthioglucoside; n-Dodecyl α/β-D-maltoside; heptaethylene glycol monododecyl ether; hexaethylene glycol monododecyl ether; octaethylene glycol monododecyl ether; pentaethylene glycol monododecyl ether; polyethylene glycol ether; polyoxyethylene state; a non-ionic detergent of the Span® series, such as Span® 20; a non-ionic detergent of the Tergitol series, such as Tergitol Type 15-S-12; a non-ionic detergent of the Brij® series, such as Brij® 35, Brij® 56, Brij® 72, Brij® 76, Brij® 92V, Brij® 97, Brij® 58P; a non-ionic detergent of the Tween series, such as Tween® 20, Tween® 21, Tween® 40, Tween® 60, Tween® 61, Tween® 65, Tween® 80, Tween® 81, Tween® 85; a non-ionic detergent of the Triton® series, such as Triton® X-100, Triton® X-114, Triton® CF-21, Triton® CF-32, Triton® DF-12, Triton® DF-16, Triton® GR-5M, Triton® X-102, Triton® X-15, Triton® X-151, Triton® X-207, Triton® X-165, Triton® X-305, Triton® X-405, Triton® X-45, Triton® X-705-70, or a non-ionic conservative variant of at least one of said surfactants.
Preferably, said non-ionic surfactant is selected from a non-ionic detergent of the Triton® series, such as Triton® X-100, Triton® X-114, Triton® CF-21, Triton® CF-32, Triton® DF-12, Triton® DF-16, Triton® GR-5M, Triton® X-102, Triton® X-15, Triton® X-151, Triton@ X-207, Triton® X-165, Triton® X-305, Triton® X-405, Triton® X-45, Triton® X-705-70, or a non-ionic conservative variant of at least one of said surfactants.
More preferably, said non-ionic surfactant is Triton® X-100, or a non-ionic conservative variant of said surfactant.

Said surfactant can an ionic surfactant, e.g., an anionic surfactant, preferably an anionic surfactant selected from DOC (sodium deoxycholate); an anionic detergent of the Triton® series, such as Triton® QS-15, Triton® QS-44, Triton® X-200; sodium 1-butanesulfonate; sodium choleate; sodium taurocholate; glycocholic acid hydrate; SDS (sodium dodecylsulfate), or an ionic conservative variant of at least one of said surfactants.
Preferably, said anionic surfactant is DOC.

Said surfactant can an ionic surfactant, e.g., an cationic surfactant, preferably an cationic surfactant selected from CDEA (cetyldimethylethylammonium bromide), alkyltrimethylammonium bromide; benzalkonium chloride; benzyldimethylhexadecylammonium chloride; thonzonium bromide, or an ionic conservative variant of at least one of said surfactants.
Preferably, said cationic surfactant is CDEA.

Said surfactant can be a zwitterionic surfactant, preferably a zwitterionic surfactant selected from CHAPS (3-[(3-Cholamidopropyl)dimethylammonio]-1-propanesulfonate), CHAPSO (3-[(3-Cholamidopropyl)dimethylammonio]-2-hydroxy-1-propanesulfonate); 3-(decyldimethylammonio)propanesulfonate inner salt, 3-(N,N-dimethylmyristylammonio)propanesulfonate, 3-(N,N-dimethylpalmitylammonio)propanesulfonate, or a zwitterionic conservative variant of at least one of said surfactants.
Preferably, said zwitterionic surfactant is CHAPS or CHAPSO.

Such surfactants (detergents) are commercially available, e.g., from Sigma-Aldrich.

In accordance with the invention, and throughout the application, preferred surfactants are those surfactants, which do not precipitate at a temperature of 0-10°C, preferably of 3-5°C, more preferably at 4°C. Any means, which the skilled person may find appropriate, can be used to determine whether a candidate surfactant has or does not have the property of precipitating at the indicated temperature(s). For example, a solution of the candidate surfactant at 0.5% (w/v) in a Ca- and Mg-free PBS buffer (see examples below) can be placed at a temperature of 4°C for 5 min. If a precipitate appears, the candidate surfactant is not a preferred surfactant. An example of a surfactant, which precipitates at 3-5°C, is SDS. Hence, SDS is not a preferred surfactant.

In accordance with the invention, and throughout the application, preferred surfactants are those surfactants, which are not denaturing surfactants.
The term "denaturing" is herein given its ordinary meaning in the field of biology, i.e., a surfactant, which induces the alteration of the native structure of the protein, with which it is mixed, causing a loss of biological activity, without modification of the primary structure. A denaturing surfactant will cause the disruption of the hydrogen bonded structure of the protein, with which it is mixed, without beaking the covalent bonds of the its chain.
To determine whether a given surfactant is denaturing, the skilled person may proceed in accordance with any method know to him/her.
For example, an IgG can be used as a test protein, to determine whether a candidate surfactant is or is not a denaturing surfactant. Indeed, an IgG contains two Fc of about 50 kDa each, and and two Fab of about 25 kDa each. When the IgG is not denatured by the candidate surfactant, one single band of about 150 kDa will appear on the blot. It the candidate surfactant is a denaturing surfactant, the Fc and Fab chains will split apart, and two bands of about 50 and 25 kDa will appear on the blot. For example, if one volume of a solution, such as a PBS buffer (see the examples below), containing the candidate denaturing surfactant at 4% (v/v) is mixed, e.g., for 5 min at 4°C, with one volume (e.g., 5 µL) of a solution containing 1 mg/mL of IgG as test protein (for example, IgG 1 A), and if at least one of the Fc and Fab chains that initially constitute said IgG appears to have thereupon split from the Ig initial structure (for example, by detecting two bands of different molecular weights), said candidate surfactant can be considered to be a denaturing surfactant.
An example of denaturing surfactant is SDS.

More preferably, in accordance with the invention, and throughout the application, preferred surfactants are those surfactants, which do not precipitate at a temperature of 0-10°C, preferably of 3-5°C, more preferably at 4°C.
Most preferably, in accordance with the invention, and throughout the application, preferred surfactants are those surfactants, which:
- do not precipitate at a temperature of 0-10°C, preferably of 3-5°C, more preferably at 4°C, and which
- are not denaturing surfactants.

Hence, in accordance with the present invention, said surfactant more preferably is hot SDS.
More preferably, said dilution solution does not comprise any denaturing surfactant. Most preferably, said dilution solution does not comprise SDS.

In accordance with the invention, and throughout the application, preferred surfactants can alternatively or complementarily be defined as those surfactants, which enable to detect by western blot the VE-cadherin 90 kDa form that is contained in an animal serum, such as the serum of a healthy human, when said serum is diluted at 1/500 in a buffer containing said surfactant at a concentration of 0.001%-0.5% (w/v). Such preferred surfactants will be herein referred to as "qualitative surfactants".
To assess whether a candidate surfactant has the capacity of such detection, i.e., whether it is a qualitative surfactant in accordance with the invention, any means that the skilled person may find appropriate can be used.
An illustrative method comprises:
- serially diluting a sample of serum which has been previously collected from an animal, preferably a healthy human, at 1/500 (serial dilution from 1 to 10, and then from 1 to 50) in a buffer, such as PBS, which contains said candidate surfactant at a concentration of 0.001%-0.5% (w/v), prior to mixing the diluted serum with SDS-PAGE sample buffer for SDS-PAGE migration under reducing and denaturing conditions,
- running SDS-PAGE under denaturing and reducing conditions,
- blotting the gel on a nitrocellulose membrane,
- analysing the blotted membrane for presence of VE-cadherin,
whereby a positive VE-cadherin detection is indicative of the fact that said candidate surfactant is a qualitative surfactant in accordance with the invention.
Figure 1B illustrates such VE-cadherin detection -obtained using Triton® X-100 as a surfactant-. Figure 1A stands as a negative control, and illustrates that, once the serum of ahealthy human has been diluted at 1/500, the VE-cadherin that is contained in it is not detected in the absence of surfactant in the dilution buffer.
Said candidate surfactant can be contained in said buffer at a concentration ranging from 0.001% to 0.4% (w/v), preferably from 0.001% to 0.06% (w/v), more preferably from 0.001% to 0.05% (w/v), even more preferably from 0.002% to 0.05% (w/v), still more preferably from 0.004% to 0.02% (w/v), most preferably from about 0.005% to 0.01% (w/v).
Said SDS-PAGE may e.g., be a classic SDS-PAGE system, such as the one developed by Laemmli.
Said SDS-PAGE sample buffer is a buffer adapted to preparing proteins to SDS-PAGE migration under denaturing and reducing conditions, i.e., a buffer enabling to denature proteins (i.e., denaturing the secondary and non-disulfide-linked tertiary structures, and applying a negative charge to each protein in proportion to its mass), and to reduce them (i.e., to further denatures the proteins by reducing disulfide linkages, thus overcoming some forms of tertiary protein folding, and breaking up quaternary protein structure). Illustrative SDS-PAGE sample buffers comprise a Tris-HCl buffer comprising SDS and comprising beta-mercaptoethanol and/or dithiothreitol (DTT), with a pH of about 6.8, such as a Laemmli beta-mercaptoethanol buffer.
For example, one volume of serially diluted serum is mixed with one volume of SDS-PAGE sample buffer, e.g., one volume of a Laemmli beta-mercaptoethanol 2x buffer concentrate (such a Laemmli 2X concentrate contains 4% SDS, 20% glycerol, 10% 2-mercaptoethanol, 0.004% bromophenol blue and 0.125 M Tris HCl, pH of about 6.8).
The resulting solution may be boiled for 5-10 min, to further achieve denaturation and reduction of the proteins.
A volume, e.g., a 10 µL volume, of the denatured and reduced solution can then be applied to a polyacrylamide gel adapted to SDS-PAGE, e.g., a 8-15% polyacrylamide gel. Illustrative composition for a 10% polyacrylamide gel adapted to the classic Laemmli SDS-PAGE system comprises:
- running gel solution: H₂O 12.3 mL; 1.5M Tris-HCl, pH 8.8 7.5 mL; 20% (w/v) SDS 0.15 mL; acrylamide/bis-acrylamide (30% / 0.8% w/v) 9.9 mL; 10% (w/v) ammonium persulfate (APS) 0.15 mL; TEMED 0.02 mL;
- stacking gel solution: H₂O 3.075 mL; 0.5M Tris-HCl, pH 6.8 1.25 mL; 20% (w/v) SDS 0.025 mL; acrylamide/bis-acrylamide (30% / 0.8% w/v) 0.67 mL; 10% (w/v) ammonium persulfate (APS) 0.025 mL; TEMED 0.005 mL.
Electrophoresis can then be performed under standard conditions for SDS-PAGE under reducing conditions. Such standard conditions notably include running the electrophoresis in the presence of a running buffer containing SDS, e.g., a running buffer comprising 25 mM Tris-HCl, 200 mM glycine, 0.1% (w/v) SDS. The gel can for example be run at 250 V constant for about 30 min, but these voltage and duration conditions have to be adjusted to the thickness of the gel, the power supply used, and the resolution desired.
The gel containing the separated proteins can be blotted onto a nitrocellulose membrane by electro-transfer, whereby a western blot is obtained.
Analysis of the western blot can then be conducted to check whether said candidate surfactant that has been used prior to SDS-PAGE preparation enables to detect said VE-cadherin.
Said detection of VE-cadherin can be achieved with any antibody that can binds to the extracellular region of human VE-cadherin, without binding to any other human serum protein. Preferably, said extracellular region is a region of human VE-cadherin, which comprises at least one of the EC1, EC2, EC3, EC4, EC5 extracellular domains, more preferably a region of human VE-cadherin, which comprises at least one of EC3, EC4, most preferably a region of human VE-cadherin, which comprises both EC3 and EC4. Most preferably, said anti-VEcadherin antibody is a monoclonal antibody, such as e.g., the BV9 mAb, which binds to a human VE-cadherin region comprising EC3 and EC4. The BV9 mAb is commercially available, e.g., from Abcam, 24, rue Louis Blanc, 75010 Paris, France, or from Abcam plc, UK, or from Abcam Inc., USA, under product reference ab7047. The quantity of mAb to be used shall be high enough to allow detection of its binding to a potential VE-cadherin target; a positive control can be made on an adjacent lane to confirm that the quantity of mAb that has been used was sufficient. For an mAb such as the BV9 mAb (which is sold by Abcam at a concentration of 0.10 mg/mL and which Abcam recommends by to use at 10 µg/mL), it is recommended in accordance with the invention to use 0.1-1 µg/mL of said BV9 mAb, e.g., about 0.2 µg/mL. It is also recommended to let the mAb incubate with the blot for several hours, such as overnight. Prior to incubation with the anti-VEcadherin antibody, the nitrocellulose blot may be blocked in milk, such as in 5% low fat milk containing PBS-Tween® at 0.05%. Anti-VEcadherin antibodies have also been described in WO 98/25946 (IMCLONE Systems; Inventors DEJANA *et al*.). Anti-VEcadherin antibodies are also commercially available from various companies, such as Gene Tex, Novus Biologicals, Cell Science or Abcam (clone BV9), or Bender MedSystems (anti-human VE-cadherin BIOTIN, product reference BMS 158BT).
Detection of the bound mAb can be performed by any procedure that the skilled person may find appropriate, such as by incubation with a secondary antibody carrying a detectable marker, such as an enzyme (for example a horseradish peroxidase mAb that can bind to the anti-VEcadherin mAb).
Illustrative conditions for such a western blot analysis can be found in the examples below, e.g., in examples 1 and 2.

In accordance with the invention, and throughout the application, preferred surfactants can alternatively or complementarily be defined those surfactants, which enable to quantitatively detect by ELISA the VE-cadherin that is contained in an animal serum, such as the serum of a healthy human.
Such preferred surfactants will be herein referred to as "quantitative surfactants".
To assess whether a candidate surfactant has the capacity of such a quantitative detection, any means that the skilled person may find appropriate can be used.
An illustrative method comprises (see example 4):
- preparing a range of at least three standard solutions, comprising:
   ∘ at least two standard solutions, which contain the human VE-cadherin fragment CAD1-4 (SEQ ID NO: 4) at two different concentrations, respectively, in a buffer, such as the Tris buffer (see examples below), and which contains the candidate surfactant at the same concentration (e.g., a concentration of 0.001%-0.5% (w/v)), whereby at least two CAD1-4 solutions, containing the CAD1-4 polypeptides at two different concentrations respectively (e.g., at 5 and 10 ng/mL) and also containing said candidate surfactant, are obtained, and
   ∘ at least one other standard solution, which does not contain said CAD1-4 polypeptide, but contains said candidate surfactant in the same buffer as said at least two standard solutions at the same concentration as these at least two standard solutions, whereby a CAD1-4-free surfactant-containing buffer is being obtained as negative control, representative of a concentration in CAD1-4 of 0 ng/mL,
- submitting said at least three standard solutions to ELISA so as to obtain a measure of the quantity of CAD1-4 detected in each of said at least three standard solutions (e.g., by using an anti-VEcadherin antibody, such as the BV9 mAb, as capture ligand coating the wells of an ELISA microplate, by performing the detection with a rabbit polyclonal anti-VEcadherin antibody as detection ligand, anti-rabbit IgG biotin conjugate, streptavidine-alcaline phosphatase, and PNNP, and mesuring the OD for each of said at least three standard solutions),
- determining whether a reliable linear standard curve can be drawn to correlate the effective CAD1-4 concentrations of each of said at least three standard solutions to the CAD1-4 concentration measures obtained by said ELISA, whereby it is determined whether the candidate surfactant has said capacity of quantitative detection.
Reliability of the linear standard curve may e.g., be acknowledged with a correlation coefficient of at least 0.9.

Said surfactant can be contained in said buffer at a concentration ranging from 0.001% to 0.4% (w/v), preferably from 0.001% to 0.06% (w/v), more preferably from 0.001% to 0.05% (w/v), even more preferably from 0.002% to 0.05% (w/v), still more preferably from 0.004% to 0.02% (w/v), most preferably from about 0.005% to 0.01% (w/v).
Such a method is illustrated in example 4 below, as well as Figures 10B, 10C, 10D, from which it can be seen that Triton® X-100 and Tween® 20 have said capacity of quantitative detection, whereas SDS does not have.

Hence, Triton® X-100 and Tween® 20 are quantitative surfactants, whereas SDS is not a quantitative surfactant.

More preferred surfactants are those surfactants, which are qualitative, more particularly those surfactants, which are qualitative as well as non-precipitating and/or non-denaturing surfactants.

Most preferred surfactants are those surfactants, which are quantitative, more particularly those surfactants, which are quantitative as well as non-precipitating and/or non-denaturing surfactants. It is notably the case of Triton® X-100 and Tween® 20.

Said surfactant can be a commercially available surfactant, or a conservative variant thereof.
By "conservative variant of a surfactant", it is herein intended a molecule which derives by at least one chemical modification from said surfactant, but which has retained the capacity of being a qualitative and/or a quantitative surfactant as herein defined.
Said at least one chemical modification may comprise any chemical modification that the skilled person may contemplate, such as e.g., at least one modification from the following group: substitution of at least one chemical entity by another, deletion of at least one chemical entity, addition of least one chemical entity, as well as any combination of such modifications.

Once at least one surfactant has been selected, it can be mixed with a diluent solution, whereby a surfactant-containing solution intended for diluting said fluid sample is produced.
If desired, more than one surfactant can be used, e.g., at least two surfactants. However, the invention is already enabled with the use of only one surfactant.

In accordance with the invention, and throughout the application, said surfactant-containing solution comprises less than 1% (w/v) of surfactant, preferably less than 0.6% (w/v), more preferably no more than 0.5% (w/v), most preferably no more than 0.45% (w/v).
Said surfactant-containing solution preferably comprises at least 0.001% (w/v) of surfactant.
Advantageously, said surfactant-containing solution comprises said selected surfactant(s) at a concentration ranging from 0.001% to 0.5% (w/v). Preferably, said surfactant-containing solution comprises said selected surfactant(s) at a concentration ranging from 0.001% to 0.4% (w/v), still preferably from 0.001% to 0.06% (w/v), more preferably from 0.001% to 0.05% (w/v), even more preferably from 0.002% to 0.05% (w/v), still more preferably from 0.004% to 0.02% (w/v), most preferably from about 0.005% to 0.01% (w/v).
Preferably, the total concentration in surfactant(s) contained in said surfactant-containing solution is as above-mentioned.

### Dilution of fluid in a surfactant-containing solution:

In accordance with the invention, and throughout the application, said fluid or fluid sample can contain lipid and/or saccharide entities (e.g., lipid entities, such as free lipids or lipoproteins, and/or saccharide entitites, such as free polysaccharides or glycoproteins), to which the soluble protein(s) to be detected and/or quantified, can be associated. Such a situation does not impair the reliability and performance of the invention. Hence, according to an advantageous embodiment of the invention, said lipid- and/or saccharide-containing fluid or sample thereof is diluted in said surfactant-containing solution so as to dissociate the soluble protein(s) from the lipid and/or saccharide entities with which they are associated.

According to an advantageous embodiment of the invention, and throughout the application, said fluid or sample thereof is diluted in said surfactant-containing solution at a dilution of at least 1/50 (v/v), preferably of least 1/60 (v/v), more preferably of least 1/80 (v/v), most preferably of at least 1/90 (v/v), more preferably of at least 1/100 (v/v). Most preferably, said dilution is of at most 1/600 (v/v), preferably of at most 1/500 (v/v).
Hence, preferably, said dilution is within any range resulting from the combination of one of the above-mentioned lower dilution limits with one of the above-mentioned upper limits, such as e.g., a dilution of 1/50-1/500, 1/60-1/500, 1/80-1/500. Said dilution is more preferably of 1/90-1/500, most preferably of 1/100-1/500.

The expressions "dilution of X", or "dilution of a factor of X", or "dilution of 1/X" or any equivalent expression, mean that 1 volume of fluid or sample is diluted in X-1 volumes of diluent solution.

According to an advantageous embodiment of the invention, and throughout the application, the fluid sample is serially diluted. For example, the fluid sample is not directly diluted to 1/500, but is serially diluted by first diluting it at 1/10 and then at 1/50, or by first diluting it at 1/50 and then at 1/10.
A very advantageous embodiment of the invention comprises serially diluting the fluid sample by a factor of 1/500, by first diluting it at 1/10 and then at 1/50, or by first diluting it at 1/50 and then at 1/10 (a first dilution at 1/10, followed by a dilution at 1/50 being preferred to a first dilution at 1/50, followed by a dilution at 1/10).

Preferably, the diluted sample is mixed, e.g., on a vortex, preferably for at least 15 seconds, and most preferably after each dilution in case of serial dilutions.
These embodiments are highly recommended by the inventors for fluid samples, such as plasma or serum, more particularly for lipid-containing fluids, such as lipemic plasma or serum.

What has been described in the above sections of course applies to, and can be combined with the subject matter described below, and conversely.

Hence, throughout the application, the term "surfactant-containing solution" preferably relates to a surfactant-containing buffer solution (e.g., surfactant-containing PBS), more preferably to a surfactant-containing solution that is buffered at a pH of about 6.5-8.5.
Said surfactant-containing solution may contain only one surfactant; alternatively, the number of surfactants contained in said solution can be of at least two.

The inventors have found improved means for the detection of soluble proteins, more particularly soluble angiogenesis-related proteins, preferably soluble VGF, endostatin and cadherins, more preferably soluble cadherins, most preferably soluble VE-cadherin.
Such a soluble protein is contained in a fluid, and is preferably a circulating protein, i.e., a soluble protein, which can be found to be contained in a naturally-occuring biological fluid, such as an extracellular fluid, more particularly blood.
These means notably comprises the dilution of the fluid containing said soluble proteins (e.g., blood), or of a fluid derivable therefrom which has retained said soluble protein (e.g., serum, plasma), in a surfactant-containing solution, prior to conducting any step that the protein detection procedure may involve.

The examples below and the appended Figures illustrate the performance of the invention, in relation to VE-cadherin: see e.g., Figure 1B (dilution of human serum in a buffer, which comprises at least one surfactant), compared to Figure 1A (dilution in the absence of surfactant); see also examples 4-5 and comparative example 6.

The application thus relates to an improved method for the detection of such a soluble protein, and to the use of at least one surfactant for such a detection.

More particularly, the application relates to a method of qualitative and/or quantitative detection of such a soluble protein(s).
Said method comprises:
- submitting said fluid, or a sample thereof, to a contacting step, which comprises:
   contacting (preferably, in vitro contacting) said fluid or sample thereof with at least one ligand that binds to said soluble protein(s) (preferably that specifically binds thereto), and
   detecting whether said at least one ligand binds to a target in said fluid or sample thereof, whereby such a binding is indicative of the presence and/or the amount of said soluble protein(s) in said fluid or sample thereof, the non-occurrence of such a binding being indicative of the absence of said soluble protein(s) in said fluid or sample thereof,
- optionally, submitting said fluid or sample thereof to a protein separation step, prior to said contacting step, so as to separate the proteins and/or polypeptides that are contained in said fluid or sample thereof according to size and/or charge,
- optionally, submitting said fluid or sample thereof to a SDS-denaturation step, prior to said protein separation step, so as to denature the proteins and polypeptides that are contained in said fluid or sample thereof by incubating it with SDS.

Prior to said contacting step, and prior to said optional SDS-denaturation step (if any), as well as prior to said optional protein separation step (if any), said method advantageously comprises diluting said fluid or sample thereof in a solution, which comprises at least one detergent, preferably at least one surfactant.

The invention demonstrates that soluble VE-cadherin contained in human blood, serum or plasma, appears as a fragment having an apparent molecular weight of about 90 kDa on western blot in denaturing and reducing conditions. It is notably the case of the circulating VE-cadherin that is contained in the blood, serum or plasma of a patient affected by a neoplasm disease or condition.
The invention also shows that said 90 kDa VE-cadherin is a fragment of VE-cadherin corresponding to the VE-cadherin extracellular region or to a fragment thereof.

Advantageously, said fluid may contain circulating lipid entities. The method of the invention enables an accurate detection of the soluble proteins, even when said fluid is a lipemic fluid. Before implementation of the invention, said at least one soluble protein can therefore be associated to (e.g., entrapped in) at least one lipid entity, such as at least one lipid vesicle or at least one chylomicron; the invention enables an accurate detection of such soluble protein(s).

Preferably, said soluble protein occurs in said fluid in a form, wherein it is not complexed to a soluble receptor or binding protein.

As above-indicated, said at least one surfactant can be selected from non-ionic surfactants (preferably, a non-ionic surfactant of the Triton® X-100 series, such as Triton® X-100, Triton® X-114), from zwitterionic surfactants (e.g., CHAPS), from cationic surfactants (e.g., CDEA) and from anionic surfactants (e.g., DOS).
Preferably, said at least one surfactant is a non-denaturing surfactant and/or a qualitative surfactant (as above-defined), and/or a quantitative surfactant (as above-defined).
More preferably, said at least one surfactant is not SDS.
Most preferably, said at least one surfactant is not a denaturing surfactant.
Most preferably, said surfactant-containing solution does not comprise SDS, and still preferably does not comprise any denaturing surfactant.

As above-indicated, said surfactant-containing solution preferably comprises less than 1% (w/v) of surfactant, preferably less than 0.6% (w/v), more preferably no more than 0.5% (w/v), most preferably no more than 0.45% (w/v).
As above-indicated, said surfactant-containing solution preferably comprises at least 0.001% (w/v) of surfactant.
As above-indicated, said surfactant-containing solution advantageously comprises said selected surfactant(s) at a concentration ranging from 0.001% to 0.5% (w/v). Preferably, said surfactant-containing solution comprises said selected surfactant(s) at a concentration ranging from 0.001% to 0.4% (w/v), still preferably from 0.001% to 0.06% (w/v), more preferably from 0.001% to 0.05% (w/v), even more preferably from 0.002% to 0.05% (w/v), still more preferably from 0.004% to 0.02% (w/v), most preferably from about 0.005% to 0.01% (w/v).
For example, when Triton® X-100 is used as surfactant, the preferred concentration is of 0.004-0.02%, more preferably of 0.005-0.01% (w/v).
Preferably, the total concentration in surfactant(s) contained in said surfactant-containing solution is as above-mentioned.

As above-indicated, and more particularly when sVE-cadherin is to be detected with the BV9 mAb, said fluid or sample thereof is diluted in said surfactant-containing solution preferably at a dilution of at least 1/50 (v/v), preferably of least 1/60 (v/v), more preferably of least 1/80 (v/v), most preferably of at least 1/90 (v/v), more preferably of at least 1/100 (v/v). Most preferably, said dilution is of at most 1/600 (v/v), preferably of at most 1/500 (v/v).
Hence, preferably, said dilution is within any range resulting from the combination of one of the above-mentioned lower dilution limits with one of the above-mentioned upper limits, such as e.g., a dilution of 1/50-1/500, 1/60-1/500, 1/80-1/500. Said dilution is more preferably of 1/90-1/500, most preferably of 1/100-1/500.
As illustrated by Figure 1B, the invention enables dilution rates of at least 1/500, for the detection of circulating VE-cadherin in serum.
Hence, preferably, said dilution is of 1/50-1/600, 1/50-1/500, 1/60-1/600, 1/60-1/500, 1/80-1/600, 1/80-1/500, 1/90-1/600, 1/90-1/500, e.g. of 1/100, 1/200, 1/300, 1/400 or 1/500.
When the invention is implemented by ELISA, said dilution preferably is of at most 1/300 (v/v), more preferably of at most 1/250 (v/v). Hence, for an ELISA implementation, said dilution preferably is of 1/50-1/250, 1/60-1/250, 1/80-1/250, 1/90-1/250, advantageously of 1/100 or 1/200 (v/v).

The inventors observed that better results are obtained when said fluid or sample thereof is serially diluted in said surfactant-containing solution. For example, for a dilution of 1/500, better results are obtained when the fluid or sample thereof is diluted at 1/10 and then at 1/50 (or conversely), than when it is directly diluted at 1/500.
Hence, in accordance with the present invention, serial dilution is preferred to direct dilution.

Any combination of said dilution rates and of said surfactant concentration can be used. For example, said surfactant-containing solution may advantageously comprise said selected surfactant(s) at a concentration ranging from 0.001% to 0.5% (w/v), and be used to dilute the fluid or fluid sample at a dilution of 1/50-1/500. More preferably, said surfactant-containing solution may advantageously comprise said selected surfactant(s) at a concentration ranging from 0.001% to 0.5% (w/v), and be used to dilute the fluid or fluid sample at a dilution of 1/90-1/500, or 1/90-1/250 for an ELISA implementation. Most preferably, e.g., for a surfactant such as Triton® X-100, said surfactant-containing solution may advantageously comprise said selected surfactant(s) at a concentration ranging from 0.004% to 0.02% (w/v), and be used to dilute the fluid or fluid sample at a dilution of 1/90-1/500, or 1/90-1/250 for an ELISA implementation.

After having been diluted in said surfactant-containing solution, said fluid or sample thereof typically contains said surfactant(s) at less than 0.1% (w/v), preferably less than 0.6% (w/v), more preferably no more than 0.5% (w/v), most preferably no more than 0.45% (w/v).

The inventors also observed that it is highly preferable to mix, e.g., by high speed shaking, preferably by vortexing, said fluid or sample thereof for at least 15 seconds, after it has been placed in contact with said surfactant-containing solution.
If the dilution is a serial dilution, said mixing is then preferably performed after each dilution step that the dilution series comprises.
Hence, in accordance with a very advantageous embodiment of the invention, said fluid or sample thereof is diluted in said surfactant-containing solution, and is mixed with this solution (e.g., by stirring, preferably by shaking, more preferably by high speed shaking, most preferably by vortexing) for at least 5 seconds, preferably for at least 10 seconds, more preferably for at least 15 seconds (e.g., for 15-50 seconds).
Advantageous speeds for said mixing, stirring, shaking, high speed shaking or vortexing comprise a speed of at least 250 rpm, preferably of at least 500 rpm, more preferably of at least 1,000 rpm, most preferably of about 2,000-2,500 rpm.

It is highly recommended not to heat said fluid or sample thereof during said (possibly serial) dilution.
The (possibly serial) dilution is more preferably performed at a cold temperature, i.e., at a temperature of about 0-10°C, preferably of about 3-5°C, e.g., about 4°C.

Throughout the application, the term "ligand" is herein intended in a broad meaning, encompassing any entity that can bind to the concerned target. The term is not restricted to any particular kind of binding, nor by any particular entity structure. Hence, this term encompasses a binding of the antibody-antigen type, as well as any other type of binding that the skilled person may find appropriate.

In accordance with the invention, and throughout the application, any ligand that the skilled person may find appropriate to the detection of said soluble protein(s) can be used. Preferably, such a ligand is an antibody directed to the soluble protein(s) to be detected, preferably a monoclonal antibody, or a conservative variant of such antibodies. Preferred conservative variants notably comprise those variants, which are a fragment of antibody or which comprise at least one of such fragments, and which have retained the capacity of binding to said soluble protein(s) to be detected.
Preferred ligands are those, which are specific of the soluble protein(s) to be detected, more particularly those ligands, which do not bind to any plasma-naturally occurring molecule other than said protein(s) to be detected.
Such ligands, antibodies, monoclonal antoibodies and conservative variants thereof, are described in greater details below, in the context of the prediction of the sensitivity or resistance to anti-tumoral treatment.

Said method may comprise a protein separation step, e.g., by electrophoresis.

Prior to said separation step, said method may comprise a SDS-denaturation step. Said SDS-denaturation step is a standard SDS-denaturation step, which comprises the denaturation, or the denaturation and the reduction, of the proteins and polypeptides contained in said fluid or sample thereof, by incubating it with SDS, or with SDS and a reducing agent (such as beta-mercaptoethanol). Said separation may thus be a protein gel electrophoresis, such as a SDS-PAGE under denaturing conditions, or under denaturing and reducing conditions. Said separation can be followed by a western blot to transfer the separated proteins and/or polypeptides on a membrane, such as a nitrocellulose membrane.

In the prior art, when western blot has been used to detect circulating proteins that may be contained in serum or plasma, the protein electrophoresis has usually been performed under denaturing conditions to allow separation on a true charge/mass ratio basis.
Hence, prior to running such an electrophoresis, a sample preparation step takes place, whereby the protein sample is denatured.
The goal of the sample preparation step is to denature the proteins fully, to disrupt any disulfide bonds through reduction, and to dissolve any particles, which would interfere with electrophoresis.
In this sample preparation step, the sample is mixed with a buffer containing a denaturing agent, and possibly also a reducing agent (such as beta-mercaptoethanol) and/or a hydrogen bonding agent (such as urea).
The denaturing agent that is the most popular is SDS, which is an anionic detergent.
Hence, standard protein electrophoresis comprises:
a step of sample preparation, which comprises mixing the sample with a buffer containing SDS as a denaturing agent (such as the Tris-HCl sample buffer of the Laemmli system), so as to denature the proteins, and
loading the denatured solution on SDS-PAGE gel, and running SDS-PAGE under denaturing conditions.
The prior art western blot detection procedure thus comprises a step, wherein the circulating proteins are contacted with the anionic detergent SDS, with the goal of denaturing the proteins, but not with the goal of increasing the sensitivity of protein detection.
In the prior art, a serum or plasma sample that would have been submitted to western blot analysis would have been directly submitted to said SDS-PAGE sample preparation step, to run a SDS-PAGE under denaturing conditions.
The invention teaches that the serum or plasma sample should be prior-diluted, preferably in a surfactant-containing diluent, before being subjected to said SDS-PAGE sample preparation step.
The SDS-PAGE sample preparation step does not lead in and of its own to the increased sensitivity in protein detection that is observed when the sample is prior-diluted in a surfactant-containing diluent in accordance with the invention (Figure 1B).

Alternatively, said method may comprise a protein separation step, but no any SDS-denaturation step. The method may indeed comprise the separation of the proteins and/or polypeptides that are contained in said fluid or sample thereof under non-denaturing conditions, e.g., by native PAGE.

Said method may alternatively not comprise any protein separation step, whereby said contacting with at least one anti-VEcadherin ligand is performed without any prior protein separation. For example, said contacting step can be performed by ELISA or cytometry.

The method of the invention may comprise the quantification of the amount of soluble protein(s) contained in the fluid or fluid sample.
Such quantification can be made by reference to at least one standard solution.
For the quantification of soluble VE-cadherin, such a standard solution advantageously comprises a (possibly recombinant) VE-cadherin fragment, corresponding to a VE-cadherin extracellular region or to a fragment thereof.
Preferably, a range of such standard solutions is made by serial dilution
Said quantification may be performed by reference to a standard curve, which has been established by submitting a dilution range of standard solutions to the detection method of the invention.

In accordance with the invention, for the quantification of soluble VE-cadherin, such a standard solution preferably comprises the human VE-cadherin fragment of SEQ ID NO: 4 (Asp48-Glu478 fragment of human VE-cadherin), or a sub-fragment thereof, which has retained the capacity of being detectable on western blot at a quantity as low as 1 nanogramme.

The inventors have indeed found that such fragments are detectable at very low concentrations (as low as 1 ng/mL), and that they are therefore useful as standards for quantification.

Disclosed herein is the VE-cadherin fragment of SEQ ID NO: 4, or a sub-fragment thereof, which has retained the capacity of being detectable on western blot (preferably on the western blot of a SDS-PAGE that has been performed in denaturing and reducing conditions), at a quantity as low as 1 nanogramme, for use as a standard in a VE-cadherin quantification assay.

Also disclosed is the use of at least one surfactant in the manufacture of a solution, wherein said solution is intended for diluting a fluid, which contains, or is suspected of containing soluble protein(s) to be detected, before contacting the diluted fluid with at least one ligand directed to said soluble protein(s), as well as prior to any SDS-denaturation step which may precede said contacting step. As above-described, said at least one surfactant preferably is not SDS, more preferably not a denaturing surfactant. More preferably, said surfactant-containing solution does not comprise SDS, and most preferably no denaturing surfactant.

Also disclosed are kits, which are intended for detecting and/or quantifying said soluble protein(s).

The application thus relates to kits, which comprise:
at least one ligand that binds to at least one of said soluble proteins, and
at least one solution, which contains at least one surfactant, preferably at least one ligand other than SDS.
As above-indicated, as described in more detailed for the detection of soluble VE-cadherin, any ligand that the skilled person may find appropriate may be used.
Said surfactant-solution is as above-described.

The application more particularly relates to kits, which are intended for detecting and/or quantifying soluble protein(s) by ELISA, and to kits, which are intended for detecting, optionally quantifying, soluble protein(s) by SDS-PAGE and/or western blot.
The elements that are contained in a kit preferably are preferably contained as distinct and separate elements.

The application thus relates to a kit, which is intended for detecting and/or quantifying soluble protein(s) by ELISA, wherein said kit comprises:
- at least two ligands, wherein each of said at least two ligands binds to said solubel protein(s) to be detected and/or quantified, and wherein:
   one of said at least two ligands is intended as a capture ligand to capture said soluble protein(s), said capture ligand being optionally immobilized in the wells of a multiwell plate, and
   the other of said at least two ligands is intended as a detection ligand that is intended to detect and/or quantify the soluble protein(s) that is bound to said capture ligand, said detection ligand being either directly coupled to an enzyme, or coupled to a molecule (e.g., biotin) enabling an indirect coupling of said second ligand to an enzyme, said enzyme (e.g., Horse Radish Peroxidase) being capable of eliciting a detectable signal (preferably, a chromogenic or fluorescent signal), e.g., upon incubation with an appropriate substrate; and
- at least one surfactant-containing solution, intended for diluting said fluid or at least one sample thereof, before contacting said fluid or sample thereof with said capture ligand, wherein said at least one surfactant-containing solution comprises less than 1% of surfactant (preferably no SDS), said at least one surfactant-containing solution being optionally intended for preparing standard solutions of said soluble protein(s).
As above-indicated, said at least one surfactant-containing solution more preferably comprises less than 0.6% (w/v), even more preferably no more than 0.5% (w/v), most preferably no more than 0.45% (w/v).
As above-indicated, said at least one surfactant-containing solution is intended for diluting said fluid or sample thereof at a dilution of preferably at least 1/50.
As above-indicated, it is highly recommended to mix said at least one surfactant-containing solution and said fluid or sample thereof together for at least 5 seconds, e.g. by high speed shaking or vortexing.
A kit may thus comprise means appropriate to apply a high speed shaking, e.g., a vortex sharker.
As above-indicated, it is highly recommended not to heat the diluted fluid or sample thereof: said mixing is preferably intended to be performed at a temperature of about 3-5°C, e.g., on ice.
A kit may thus comprise means appropriate to apply a temperature of about 3-5°C, e.g., ice.
Preferably, said at least one surfactant-containing solution is Ca- and Mg-free, and contains at least one anti-protease agent, e.g., leupeptin, for example 25 %g/mL leupeptin.
Such a kit may further comprise:
- at least one assay buffer solution, intended for being mixed with said detection ligand to formulate it in liquid form and/or to dilute it, wherein said assay buffer solution may comprise a surfactant (preferably not SDS); such an assay buffer solution is useful when said detection ligand is provided in the form of a concentrate, notably in the form of a powder concentrate, and/or
- at least one wash buffer solution, intended for removing unbound soluble protein(s) and/or for washing microplate wells, wherein said wash buffer solution comprises a surfactant (preferably not SDS), and/or

- an instruction leaflet, which comprises advising of pre-diluting at least one sample of said fluid in said at least one surfactant-containing solution, preferably at a dilution of at least 1/50 (v/v), more preferably by serial dilutions, and optionally of vortexing the diluted sample(s) for at least 15 seconds, before contacting said diluted and optionally vortexed fluid sample(s) with said capture ligand, and/or
- at least one sample buffer diluent, intended for diluting samples of said fluid and/or for preparing standard solutions for the quantification of said soluble protein(s), wherein said sample buffer diluent does not comprise any surfactant, and/or
- at least one said soluble protein(s) in isolated form, which is intended as a positive control and/or a standard for the quantification of the amount of soluble protein(s) bound to said capture ligand, such as e.g., in the case of a soluble protein being VE-cadherin, a VEcadherin-related molecule selected from VE-cadherin, VE-cadherin extracellular region, and fragments of said VE-cadherin and/or of said VE-cadherin extracellular region, and/or
- at least one substrate for said enzyme (e.g., HRP), whereby upon incubation with said substrate, said enzyme elicites a detectable signal (e.g., a chromogenic or fluorescent signal), or means appropriate to provide such a substrate (e.g., tetramethyl-benzidine and hydrogen peroxide, which can be provided separately, and are intended to be mixed together to form a substrate solution for the HRP enzyme), and/or
- at least one stop solution (e.g., phosphoric acid), which is intended for stopping the enzymatic reaction, and/or
- at least one multiwell plate (e.g., a 96-well plate), and/or
- at least one plate cover, and/or
- at least one colour-giving reagent (e.g., at least one dye), and/or
- at least one spectrophotometer or other optical device adapted to the detection, optionally the quantification, of said enzyme signal.
Said at least one said VEcadherin-related molecule, which is intended as a positive control and/or a standard for the quantification of the amount of soluble VE-cadherin bound to said capture ligand, advantageously is the VE-cadherin fragment of SEQ ID NO: 4, or any sub-fragment thereof that retained the capacity of being detectable on western blot (preferably in denaturing and reducing conditions) at a quantity as low as 1 nanogramme.
The surfactant that is contained is said at least one surfactant-containing solution may be different from the surfactant(s) that is(are) contained in said at least assay buffer solution and/or said at least one wash buffer solution.

The application also relates to a kit, which is intended for detecting and/or quantifying soluble protein(s) by ELISA, wherein said kit comprises:
- at least two ligands, wherein each of said at least two ligands binds to said soluble protein(s), and wherein:
   one of said at least two ligands is intended as a capture ligand to capture said soluble protein(s), said capture ligand being optionally immobilized in the wells of a multiwell plate, and
   the other of said at least two ligands is intended as a detection ligand that is intended to detect and/or quantify the soluble protein(s) that is(are) bound to said capture ligand, said detection ligand being either directly coupled to an enzyme, or coupled to a molecule (e.g., biotin) enabling an indirect coupling of said second ligand to an enzyme, said enzyme (e.g., Horse Radish Peroxidase) being capable of eliciting a detectable signal (preferably, a chromogenic or fluorescent signal), e.g., upon incubation with an appropriate substrate, and
- at least one surfactant-containing solution, intended for diluting said fluid or at least one sample thereof, before contacting said fluid or sample thereof with said capture ligand, wherein said at least one surfactant-containing solution comprises at least one surfactant (preferably not SDS, more preferably said solution does not comprise any SDS), said at least one surfactant-containing solution being optionally intended for preparing standard solutions of VE-cadherin, and
- at least one assay buffer solution, intended for being mixed with said detection ligand to formulate it in liquid form and/or to dilute it, wherein said assay buffer solution comprises at least one surfactant (preferably not SDS, more preferably said solution does not comprise any SDS), such an assay buffer solution being useful when said detection ligand is provided in the form of a concentrate, notably in the form of a powder concentrate, and
- at least one wash buffer solution, intended for removing unbound VE-cadherin and/or for washing microplate wells, wherein said wash buffer solution comprises at least one (preferably not SDS, more preferably said solution does not comprise any SDS).
As above-indicated, said at least one surfactant-containing solution preferably comprises less than 1% of surfactant more preferably less than 0.6% (w/v), even more preferably no more than 0.5% (w/v), most preferably no more than 0.45% (w/v).
Said at least one wash buffer solution preferably comprises at least 1% (w/v) of surfactant. Said at least one assay buffer solution preferably comprises at least 1% (w/v) of surfactant.
As above-indicated, it is highly recommended to mix said at least one surfactant-containing solution and said fluid or sample thereof together for at least 5 seconds, e.g. by high speed shaking or vortexing.
A kit may thus comprise means appropriate to apply a high speed shaking, e.g., a vortex sharker.
As above-indicated, it is highly recommended not to heat the diluted fluid or sample thereof: said mixing is preferably intended to be performed at a temperature of about 3-5°C, e.g., on ice.
A kit may thus comprise means appropriate to apply a temperature of about 3-5°C, e.g., ice.
Preferably, said at least one surfactant-containing solution is Ca- and Mg-free, and contains at least one anti-protease agent, e.g., leupeptin, for example 25 %g/mL leupeptin.
Said kit may further comprise:
- an instruction leaflet, which comprises advising of pre-diluting at least one sample of said fluid in said at least one surfactant-containing solution, preferably at a dilution of at least 1/50 (v/v), more preferably by serial dilutions, and optionally of vortexing the diluted sample(s) for at least 15 seconds, before contacting said diluted and optionally vortexed fluid sample(s) with said capture ligand, and/or
- at least one sample buffer diluent, intended for diluting samples of said fluid and/or for preparing VE-cadherin standard solutions, wherein said sample buffer diluent does not comprise any surfactant, and/or
- at least one said soluble protein(s) in isolated form, which is intended as a positive control and/or a standard for the quantification of the amount of soluble protein(s) bound to said capture ligand, such as e.g., in the case of a soluble protein being VE-cadherin, a VEcadherin-related molecule selected from VE-cadherin, VE-cadherin extracellular region, and fragments of said VE-cadherin and/or of said VE-cadherin extracellular region, wherein at least one VEcadherin-related molecule is intended for preparing at least one standard solution for quantification of the amount of VE-cadherin bound to said capture ligand, and/or
- at least one substrate for said enzyme (e.g., HRP), whereby upon incubation with said substrate, said enzyme elicites a detectable signal (e.g., a chromogenic or fluorescent signal), or means appropriate to provide such a substrate (e.g., tetramethyl-benzidine and hydrogen peroxide, which can be provided separately, and are intended to be mixed together to form a substrate solution for the HRP enzyme), and/or
- at least one stop solution (e.g., phosphoric acid), which is intended for stopping the enzymatic reaction, and/or
- at least one multiwell plate (e.g., a 96-well plate), and/or
- at least one plate cover, and/or
- at least one colour-giving reagent (e.g., at least one dye), and/or
- at least one spectrophotometer or other optical device adapted to the detection, optionally the quantification, of said enzyme signal.
Said at least one said VEcadherin-related molecule; which is intended as a positive control and/or a standard for the quantification of the amount of soluble VE-cadherin, bound to said capture ligand advantageously is the VE-cadherin fragment of SEQ ID NO:4, or any sub-fragment thereof that retained the capacity of being detectable on western blot (preferably in denaturing and reducing conditions) at a quantity as low as 1 nanogramme. The surfactant that is contained is said at least one surfactant-containing solution may be different from the surfactant(s) that is(are) contained in said at least assay buffer solution and/or said at least one wash buffer solution.

In the above-mentioned ELISA kits, when said soluble protein is soluble VE-cadherin, said capture ligand may e.g., be an anti-human VE-cadherin mAb recognizing the extracellular juxtamembrane domains EC3 and EC4, such as the BV9 mAb (mouse anti-human VE-cadherin mAb, available from, e.g., Abcam). Said capture ligand is advantageously provided in a quantity of at least 1 µg per sample well.

In the above-mentioned ELISA kits, when said soluble protein is soluble VE-cadherin, said VE-cadherin related molecule, which is intended for preparing at least one standard solution for quantification of the amount of VE-cadherin bound to said capture ligand, can advantageously be the polypeptide of SEQ ID NO: 4 (CAD1-4), provided at e.g., a quantity of at least 0.016 ng per standard solution well, or a conservative sub-fragment thereof, which has retained the capacity of being detectable on western blot at a quantity as low as 1 nanogramme.

The application also relates to a kit, which is intended for detecting and/or quantifying soluble protein(s) by SDS-PAGE and/or western blot, wherein said kit comprises:
- at least one ligand, which binds to said soluble protein(s), and
   and at least two distinct and separate solutions, namely:
- at least one SDS-containing solution, which is intended for denaturing the proteins and/or polypeptides that are contained in said fluid, said at least one SDS-containing solution optionally further comprising a reducing agent such as beta-mercaptoethanol, and
- at least one surfactant-containing solution, which is intended for diluting said fluid or at least one sample thereof, before contacting said fluid or sample thereof with said at least one ligand.
As above-indicated, said at least one surfactant-containing solution preferably comprises less than 1% (w/v) of surfactant, preferably less than 0.6% (w/v), more preferably no more than 0.5% (w/v), most preferably no more than 0.45% (w/v).
As above-indicated, said at least one surfactant-containing solution does preferably not comprise SDS.
As above-indicated, it is highly recommended to mix said at least one surfactant-containing solution and said fluid or sample thereof together for at least 5 seconds, e.g. by high speed shaking or vortexing.
A kit may thus comprise means appropriate to apply a high speed shaking, e.g., a vortex sharker.
As above-indicated, it is highly recommended not to heat the diluted fluid or sample thereof: said mixing is preferably intended to be performed at a temperature of about 3-5°C, e.g., on ice.
A kit may thus comprise means appropriate to apply a temperature of about 3-5°C, e.g., ice.
Preferably, said at least one surfactant-containing solution is Ca- and Mg-free, and contains at least one anti-protease agent, e.g., leupeptin, for example 25 %g/mL leupeptin.

All the kits can be intended for detecting and/or quantifying the soluble protein(s), which is(are) contained in a fluid selected from the extracellular fluids of a multicellular organism, the blood of a multicellular organism, the fluids that are derivable from such extracellular fluids, such as serum, plasma, and the cell-free (or substantially cell-free) fluid of a cell- or tissue-culture medium.
As above-indicated, the kits are more particularly intended for soluble angiogenesis-related proteins, preferably soluble VGF, endostatin, soluble cadherins, more preferably soluble cadherins, most preferably soluble VE-cadherin.
As will be described below, they can also be intended for the prediction of sensitivity or resistance to anti-tumor treatment.

The means for detecting, optionally quantifying, soluble proteins are more particularly adapted to the detection, optionally the identification, of angiogenesis- and/or inflammation-related markers, such as a tumor marker, a marker of resistance of resistance or non-resistance to anti-tumor treatment, a marker of a vascular pathology, a marker of sepsis (such as sepsis preeclampsia), a marker of rheumatoid arthriotis.
The means further enabled to identify a new predictive maker of responsiveness or non-responsive to anti-tumor treatment, namely sVE-cadherin. To the best of the inventors' knowledge, prior art sVe-cadherin kits did not enable to identify that sVE-cadherin is such a predictive marker.

### Means for the prediction of resistance or sensitivity to anti-tumor treatment:

The improved means for the detection of circulating VE-cadherin enabled the inventors to demonstrate that circulating VE-cadherin is a reliable prognostic marker of sensitivity or resistance to anti-tumor treatment.
More particularly, the inventors show that a VE-cadherin content that is significantly lower than the mean VE-cadherin content that is observed in comparable fluids of healthy volunteers, is indicative of the patient being at high risk of being resistant to anti-tumor treatment (see e.g., Figure 6).

These means enabled to determined that circulating VE-cadherin is a reliable marker for the prognosis of sensitivity or resistance to anti-tumour therapy: see Figure 6.
More particularly, a VE-cadherin content that is significantly lower than the mean VE-cadherin content that is observed in comparable fluids of healthy volunteers, is indicative of the patient being at high risk of being resistant to anti-tumor treatment (see e.g., Figure 6).

As illustrated in example 6 below, prior art sVE-cadherin kits were not accurate enough to enable such a determination.
In fact, it is believed that most prior art results relating to circulating VE-cadherin might be biased.

The invention accordingly relates to VE-cadherin as a predictive marker of sensitivity or resistance to anti-tumor treatment.

In the present application, the term tumor is intended for neoplastic tumor, and includes cancer (benign as well malignant tumor).

The application thus relates to a method for predicting the resistance or sensitivity of an animal or patient to anti-tumor treatment,
which comprises analyzing a fluid sample for its content in VE-cadherin (including a content in VE-cadherin that is of zero, i.e., absence of VE-cadherin),
wherein said fluid sample is a sample of fluid, which has been previously collected from said animal or patient (e.g., blood or an extracellular fluid), or is a sample of a product, preferably of a fluid derivable from such a previously-collected fluid sample (e.g. serum, plasma).
A content in VE-cadherin that is significantly lower than the mean VE-cadherin content that is observed in comparable fluids of healthy volunteers, is indicative of the patient being at high risk of being resistant to anti-tumor treatment.
More particularly, in accordance with the invention, a content in VE-cadherin that is at least five times (preferably at least six times, more preferably at least seven times, most preferably at least eight times) inferior to the mean VE-cadherin content that is observed in comparable fluids of healthy volunteers (said VE-cadherin contents being of course measured in accordance with the invention). For example, a VE-cadherin content that is equal to or inferior to 100 ng/mL of human serum or plasma, is clearly indicative of the patient being resistant, or at least non-responsive, to (future) anti-tumor treatment.

A content in VE-cadherin not significantly lower than the mean VE-cadherin content that is observed in comparable fluids of healthy volunteers, is indicative of the patient being at high risk of being sensitive to anti-tumor treatment.
For example, as illustrated in example 5 below, the mean VE-cadherin content that is observed in comparable fluids of healthy human volunteers is of about 700-800 ng/mL (as measured in accordance with the invention); and the inventors demonstrate that a VE-cadherin content that is inferior to about 100 ng/mL can reliably be correlated to (future) resistance to anti-tumor therapy, whereas VE-cadherin contents of about 500-1,000 ng/mL are indicative of the patient being at high risk of being sensitive to anti-tumor treatment.

The expression "analyzing a fluid sample for its content in VE-cadherin" encompasses a quantitative analysis, wherein the content in VE-cadherin is numerically determined, as well as a qualitative analysis, wherein the content in VE-cadherin is qualitatively determined, e.g., by determining that said content is "null", "low", "normal" or "high".
The content in circulating VE-cadherin is not equal to zero in healthy subjects. In some tumor-containing subjects, this content may have fallen down to zero, or at least down to an amount that is so low that is undetectable; or this content may be low, compared to healthy subjects. In accordance with the invention, such subjects are at high risk of being resistant to anti-tumor treatment.
Quite similarly, without numerically determining the content in VE-cadherin present in the sample, the skilled person may, e.g., by visual comparison, determine whether this content is comparable to the one that would have been observed in a healthy subject. In accordance with the invention, such subjects are likely to be sensitive to anti-tumor treatment.
Hence, a qualitative evaluation of the content in VE-cadherin may be sufficient to conclude on the resistant or sensitive status of the animal or patient.

Of course, if desired or required, a quantitative evaluation may be done. Such a quantification may be achieved by reference to a standard solution, which contains a known amount of VE-cadherin, said standard solution being then submitted to the same process as the subject's sample. Such a standard solution can advantageously comprise the VE-cadherin fragment of SEQ ID NO: 4, or a sub-fragment thereof, which has retained the capacity of being detectable on western blot (SDS-PAGE in denaturing and reducing conditions, followed by western blotting) at a quantity as low as 1 nanogramme.
Preferably, said tumor is selected from as lung tumor, liver tumor, breast tumor, kidney tumor, prostate tumor, CNS neoplasm, brain tumor, intracranial tumor, glioma, carcinoma, sarcoma (e.g., Kaposi's sarcoma), mesothelioma, choriocarcinoma. More preferably, said tumor is a glioma selected from ependymomas, astrocytomas, oligodendrogliomas, or mixed gliomas, such as oligoastrocytomas. Even more preferably, said tumor is an astrocytoma. Most preferably, said tumor is a gliobastoma multiforme (GBM). Advantageously, said tumor can be a tumor that does not derive from blood cells.

Said anti-tumor treatment is global or targeted chemotherapy and/or radiotherapy and/or immunotherapy and/or hormone suppression.
Said chemotherapy may involve the administration of at least one element selected from alkylating agents, anti-metabolites, plant alkaloids and terpenoids, vinca alkaloids, podophyllotoxins, taxanes, toposiomerase inhibitors. Said chemotherapy may involve the administration of temozolomide.
Said chemotherapy may involve the administration of a more targeted chemical agent, such as an inhibitor of tyrosine kinases (e.g., herceptin).
Said immunotherapy may involve the administration of a monoclonal antibody, e.g., a monoclonal antibody directed to VEGF, e.g., Bevacizumab®.

Preferably, said anti-tumor treatment is global or targeted chemotherapy, most preferably global chemotherapy (i.e., a non-specific or non-targeted chemotherapy).

Response to therapy can be investigated by any means known to the skilled person or to the physician. For example, response to anti-glioma treatment can be evaluated using RMI following the cairncross criteria. Such an evaluation enables to determine whether the patient is sensitive or resistant to the treatment.

Prediction of response to therapy is a major objective in the treatment of glial tumors. Temozolomide (Temodal®; available from Schering-Plough) is now a standard regimen for glioblastomas associated to radiotherapy, or alone in relapsing glioblastoma and oligodendrogliomas. The goal of the inventors was to investigate potential peripheral serum biomarkers potentially relevant for the prediction of response to therapy.
The neuropathological diagnosis in glioma remains imprecise and poorly reproducible whatever the classification used. The diagnosis of oligodendroglioma versus astrocytoma remains questionable in more than 40% of cases.
lp/19q heterozygothy loss has been associated to response to chemotherapy in oligodendrogliomas. Similarly, the methylation status of 06-methylguanine-DNA-methyltransferase (MGMT) has been associated to response to the association of Temodal® to radiotherapy (New England Journal of Medicine 2005, March 10, 352(10): 987-996).
The biomarker of the inventors can be investigated at the periphery of the disease in absence of tissue biopsy.

Said VE-cadherin content analysis is advantageously performed by submitting said fluid sample to the method of qualitative or quantitative detection of circulating VE-cadherin of the invention (see above, as well in the examples below). As above-described, said method thus comprises diluting said fluid or sample thereof in a surfactant-containing solution prior to assaying said fluid sample for its content in circulating VE-cadherin (qualitative and/or quantitative content), as well as prior to any SDS-denaturation step that said assay may comprise.

The application also relates to at least one anti-VEcadherin ligand for use to predict sensitivity or resistance to anti-tumor treatment, e.g., in the manufacture of a composition intended for predicting sensitivity or resistance to anti-tumor treatment.

The expression "binds to VE-cadherin" does of course not exclude the fact that the ligand may bind to a VE-cadherin fragment.
In fact, a preferred ligand of the invention binds to the extracellular region of VE-cadherin, which implies that it binds to a polypeptide, the sequence of which is identical to the sequence of a VE-cadherin extracellular region or to the sequence of a fragment of VE-cadherin extracellular region.
Preferably, said at least one ligand binds to VE-cadherin in a region, which comprises at least one, preferably both of the EC3 and/or EC4 domains.
Said ligand advantageously is an anti-VEcadherin antibody, preferably a monoclonal antibody (e.g., the BV9 clone, which is available from Abcam).

Said ligand may be a functional equivalent of such antibodies.
Functional equivalents have retained the capacity of binding to VE-cadherin, preferably human VE-cadherin, most preferably to the extracellular region of said VE-cadherins.
Such functional equivalents notably comprise conservative variants of anti-VEcadherin antibodies,
wherein said conservative variant:
is a fragment of anti-VEcadherin antibody, or comprises at least one fragment of anti-VEcadherin antibody, and
has retained the capacity of binding to VE-cadherin, preferably to the extracellular region of VE-cadherin, more preferably to VE-cadherin in a region, which comprises at least one, preferably both of the EC3 and/or EC4 domains.

Preferred ligands are those ligands, which do not bind to any molecule that is naturally occurring in the serum of a mammal, preferably of a human, other than VE-cadherin. Illustrative functional equivalents comprise artificially altered gene recombinant antibodies, such as chimeric antibodies or humanized antibodies, which can be used e.g., for lowering heteroantigenicity against a human. These altered antibodies can be produced using a known method.
Chimeric antibodies can e.g., be obtained by ligating the DNA encoding the antibody V-region to a DNA encoding a human antibody C-region, incorporating the product into an expression vector, and then introducing the vector into a host to cause the host to produce the antibodies. Using this known method, chimeric antibodies useful in the present invention can be obtained.
Humanized antibodies are also referred to as reshaped human antibodies, which are prepared by grafting an antibody CDR (complementarity determining region) of a mammal other than a human, such as a mouse, to the CDR of a human antibody. The general gene recombination technique thereof is also known (see European Patent application EP 125 023 and WO 96/02576).

An antibody used in the present invention is not limited to the whole molecule, and may be a fragment of the antibody or the modified product thereof, as long as it still binds to VE-cadherin, preferably on the extracellular region of VE-cadherin.
Illustrative functional equivalents therefore also comprise bivalent antibodies, as well as monovalent antibodies, as well as antibody fragments. Examples of antibody fragments include Fab, F(ab')2, Fv, Fab/c having one Fab and a complete Fc, and a single chain Fv (scFv) wherein the Fv of the H-chain or the L-chain is ligated with an appropriate linker. Specifically, an antibody fragment is synthesized by treating the antibody with an enzyme such as papain or pepsin, or genes encoding these antibody fragments are constructed, the genes are introduced into expression vectors, and the genes are then expressed by appropriate host cells (see e.g., Rousseaux, J. et al., Methods in Enzymology (1989) 121, 663-669, and Bird, R. E. et al., TIBTECH (1991) 9, 132-137).

scFv is obtained by linking the H-chain V-region and the L-chain V-region of antibodies. In the scFv, the H-chain V-region and the L-chain V-region are linked via a linker, or preferably a peptide linker (Huston, J. S. et al., Proc. Natl. Acad. Sci. U.S.A. (1988) 85, 5879-5883). The H-chain V-region and the L-chain V-region in scFv may be derived from any of those described as antibodies in this specification. As a peptide linker to link the V-regions, for example, any single-stranded peptide comprising 12 to 19 amino acid residues is used.

A DNA encoding scFv can be obtained as follows. Amplification is performed by the PCR method using as templates the entire or DNA portions encoding desired amino acid sequences (of a DNA encoding the H-chain or the H-chain V-region of the above antibody, and a DNA encoding the L-chain or the L-chain V-region), and using a primer pair that specifies both ends. Amplification is then further performed by a combined use of a DNA encoding a peptide linker portion and a primer pair that specify to cause both ends to ligate respectively to the H-chain and L-chain.

Furthermore, once a DNA encoding scFv is prepared, expression vectors containing the DNAs, and hosts transformed with the expression vectors, can be obtained according to the standard method. In addition, by the use of the host, scFv can be obtained according to the standard method.

These antibody fragments can be produced using hosts by obtaining the genes thereof in a manner similar to the above method, and then causing the expression of the genes. The "antibody" in the present invention also encompasses these antibody fragments.
While transgenic mammalian cells (e.g., Chinese hamster ovary cells) grown in culture are the industry standard for producing full length mAb, mammalian cells may be less suited for the production of antibody fragments such as Fab or scFv, and prokaryotic expression systems (e.g., *E. coli*) or other eukaryotic expression systems, such as yeast or plant cells, may preferably be used

Furthermore, the antibody used in the present invention may be a bispecific antibody, which can also be prepared by genetic engineering techniques.

The antibodies expressed and produced as described above can be isolated from the cells or host animals, and purified to a uniform level. Isolation and purification of the antibodies to be used in the present invention can be performed using affinity columns. An example of a column using a protein A column is a Hyper D, POROS, Sepharose F. F. (Pharmacia). Other standard isolation and purification methods that are employed for proteins may be used, and there is no limitation regarding their use. For example, a chromatography column other than the above affinity column, a filter, ultrafiltration, a method of salting out, dialyses and the like may be appropriately selected and combined for use, so that antibodies can be isolated and purified (Antibodies A Laboratory Manual. Ed Harlow, David Lane, Cold Spring Harbor Laboratory, 1988).

Said at least one antibody or conservative variant thereof preferably binds to VE-cadherin in a region, which comprises at least one, preferably both of the EC3 and/or EC4 domains. Examples of such antibodies comprise the BV9 antibody, which is a mouse monoclonal antibody that binds to human VE-cadherin in a region spanning the EC3 and the EC4 domains, and which is commercially available from Abcam (e.g., Abcam Inc., U.S.A.) under product reference ab7047.

Said at least one antibody or conservative variant thereof more preferably binds to the VE-cadherin fragment of SEQ ID NO: 2 and/or to the VE-cadherin fragment of SEQ ID NO: 4, or to a sub-fragment thereof.
The sequence of SEQ ID NO: 2 is a sequence of human VE-cadherin extracellular region; it has the following amino acid sequence (fragment 1-593 of the sequence available from EMBL/Genbank under accession number CAA56306):

1 mqrlmmllat sgaclgllav aavaaaganp aqrdthsllp thrrqkrdwi wnqmhideek
61 ntslphhvgk ikssvsrkna kyllkgeyvg kvfrvdaetg dvfaierldr eniseyhlta
121 vivdkdtgen letpssftik vhdvndnwpv fthrlfnasv pessavgtsv isvtavdadd
181 ptvgdhasvm yqilkgkeyf aidnsgriit itksldrekq aryeivvear daqglrgdsg
241 tatvlvtlqd indnfpfftq tkytfvvped trvgtsvgsl fvedpdepqn rmtkysilrg
301 dyqdaftiet npahnegiik pmkpldyeyi qqysfiveat dptidlryms ppagnraqvi
361 initdvdepp ifqqpfyhfq lkenqkkpli gtvlamdpda arhsigysir rtsdkgqffr
421 vtkkgdiyne keldrevypw ynltveakel dstgtptgke sivqvhievl dendnapefa
481 kpyqpkvcen avhgqlvlqi saidkditpr nvkfkftlnt ennftltdnh dntanitvky
541 gqfdrehtkv hflpvvisdn gmpsrtgtst ltvavckcne qgeftfcedm aaq

SEQ ID NO: 2 (593 amino acids)

A DNA sequence encoding such a fragment has the following sequence (SEQ ID NO: 1; fragment 25-1803 the sequence available from EMBL/Genbank under accession number X79981):

25 atgcag aggctcatga tgctcctcgc cacatcgggc
61 gcctgcctgg gcctgctggc agtggcagca gtggcagcag caggtgctaa ccctgcccaa

121 cgggacaccc acagcctgct gcccacccac cggcgccaaa agagagattg gatttggaac
181 cagatgcaca ttgatgaaga gaaaaacacc tcacttcccc atcatgtagg caagatcaag
241 tcaagcgtga gtcgcaagaa tgccaagtac ctgctcaaag gagaatatgt gggcaaggtc
301 ttccgggtcg atgcagagac aggagacgtg ttcgccattg agaggctgga ccgggagaat
361 atctcagagt accacctcac tgctgtcatt gtggacaagg acactggtga aaacctggag
421 actccttcca gcttcaccat caaagttcat gacgtgaacg acaactggcc tgtgttcacg
481 catcggttgt tcaatgcgtc cgtgcctgag tcgtcggctg tggggacctc agtcatctct
541 gtgacagcag tggatgcaga cgaccccact gtgggagacc acgcctctgt catgtaccaa
601 atcctgaagg ggaaagagta ttttgccatc gataattctg gacgtattat cacaataacg
661 aaaagcttgg accgagagaa gcaggccagg tatgagatcg tggtggaagc gcgagatgcc
721 cagggcctcc gggggactc gggcacggcc accgtgctgg tcactctgca agacatcaat
781 gacaacttcc ccttcttcac ccagaccaag tacacatttg tcgtgcctga agacacccgt
841 gtgggcacct ctgtgggctc tctgtttgtt gaggacccag atgagcccca gaaccggatg
901 accaagtaca gcatcttgcg gggcgactac caggacgctt tcaccattga gacaaacccc
961 gcccacaacg agggcatcat caagcccatg aagcctctgg attatgaata catccagcaa
1021 tacagcttca tcgtcgaggc cacagacccc accatcgacc tccgatacat gagccctccc
1081 gcgggaaaca gagcccaggt cattatcaac atcacagatg tggacgagcc ccccatttc
1141 cagcagcctt tctaccactt ccagctgaag gaaaaccaga agaagcctct gattggcaca
1201 gtgctggcca tggaccctga tgcggctagg catagcattg gatactccat ccgcaggacc
1261 agtgacaagg gccagttctt ccgagtcaca aaaaaggggg acatttacaa tgagaaagaa
1321 ctggacagag aagtctaccc ctggtataac ctgactgtgg aggccaaaga actggattcc
1381 actggaaccc ccacaggaaa agaatccatt gtgcaagtcc acattgaagt tttggatgag
1441 aatgacaatg ccccggagtt tgccaagccc taccagccca aagtgtgtga gaacgctgtc
1501 catggccagc tggtcctgca gatctccgca atagacaagg acataacacc acgaaacgtg
1561 aagttcaaat tcaccttgaa tactgagaac aactttaccc tcacggataa tcacgataac
1621 acggccaaca tcacagtcaa gtatgggcag tttgaccggg agcataccaa ggtccacttc
1681 ctacccgtgg tcatctcaga caatgggatg ccaagtcgca cgggcaccag cacgctgacc
1741 gtggccgtgt gcaagtgcaa cgagcagggc gagttcacct tctgcgagga tatggccgcc
1801 cag

SEQ ID NO: 1 (1779 nucleotides)

The sequence of SEQ ID NO: 4 is a sequence of a fragment of human VE-cadherin extracellular region (Asp48-Glu478 fragment of the sequence of SEQ ID NO:2, preceded by Met). The sequence of SEQ ID NO: 4 has retained the EC3 and EC4 domains of human VE-cadherin.

atggattggatttggaaccagatgcacattgatgaagagaaaacacctcacttccccat
 M  D  W  I  W  N  Q  M  H  I  D  E  E  K  N  T  S  L  P  H
catgtaggcaagatcaagtcaagcgtgagtcgcaagaatgccaagtacctgctcaaagga
 H  V  G  K  I  K  S  S  V  S  R  K  N  A  K  Y  L  L  K  G
gaatatgtgggcaaggtcttccgggtcgatgcagagacaggagacgtgttcgccattgag

E Y V G K V F R V D A E T G D V F A I E
aggctggaccgggagaatatctcagagtaccacctcactgctgtcattgtggacaaggac
R L D R E N I S E Y H L T A V I V D K D
actggtgaaaacctggagactccttccagcttcaccatcaaagttcatgacgtgaacgac
T G E N L E T P S S F T I K V H D V N D
aactggcctgtgttcacgcatcggttgttcaatgcgtccgtgcctgagtcgtcggctgtg
N W P V F T H R L F N A S V P E S S A V
gggacctcagtcatctctgtgacagcagtggatgcagacgaccccactgtgggagaccac
G T S V I S V T A V D A D D P T V G D H
gcctctgtcatgtaccaaatcctgaaggggaaagagtatttgccatcgataattctgga
A S V M Y Q I L K G K E Y F A I D N S G
cgtattatcacaataacgaaaagcttggaccgagagaagcaggccaggtatgagatcgtg
R I I T I T K S L D R E K Q A R Y E I V
gtggaagcgcgagatgcccagggcctccgggggactcgggcacggccaccgtgctggtc
V E A R D A Q G L R G D S G T A T V L V
actctgcaagacatcaatgacaacttccccttcttcacccagaccaagtacacatttgtc
T L Q D I N D N F P F F T Q T K Y T F V
gtgcctgaagacacccgtgtgggcacctctgtgggctctctgtttgttgaggacccagat
V P E D T R V G T S V G S L F V E D P D
gagccccagaaccggatgaccaagtacagcatcttgcggggcgactaccaggacgctttc
E P Q N R M T K Y S I L R G D Y Q D A F
accattgagacaaaccccgcccacaacgagggcatcatcaagcccatgaagcctctggat
T I E T N P A H N E G I I K P M K P L D
tatgaatacatccagcaatacagcttcatcgtcgaggccacagaccccaccatcgacctc
Y E Y I Q Q Y S F I V E A T D P T I D L
cgatacatgagccctcccgcgggaaacagagcccaggtcattatcaacatcacagatgtg
R Y M S P P A G N R A Q V I I N I T D V
gacgagcccccatttccagcagcctttctaccacttccagctgaaggaaaaccagaag
D E P P I F Q Q P F Y H F Q L K E N Q K
aagcctctgattggcacagtgctggccatggaccctgatgcggctaggcatagcattgga
K P L I G T V L A M D P D A A R H S I G
tactccatccgcaggaccagtgacaagggccagttcttccgagtcacaaaaaaggggac
Y S I R R T S D K G Q F F R V T K K G D
atttacaatgagaaagaactggacagagaagtctacccctggtataacctgactgtggag
I Y N E K E L D R E V Y P W Y N L T V E
gccaaagaactggattccactggaacccccacaggaaaagaatccattgtgcaagtccac
A K E L D S T G T P T G K E S I V Q V H
attgaagttttggatgagaatgacaatgccccggag
I E V L D E N D N A P E
= fragment Asp48-Glu478 of human VE-cadherin extracellular region of SEQ ID NO: 2
= CAD 1-4 fragment (preceded by Met)
= SEQ ID NO: 4

A sequence coding for said CAD 1-4 fragment of SEQ ID NO: 4 is the sequence extending from nucleotide 166 to nucleotide 1458 of SEQ ID NO:1 (preceded by the atg codon), i.e.:

atggattggatttggaaccagatgcacattgatgaagagaaaaacacctcacttccccatcatgtaggcaagatcaagtcaagcgt
gagtcgcaagaatgccaagtacctgctcaaaggagaatatgtgggcaaggtcttccgggtcgatgcagagacaggagacgtgtt
cgccattgagaggctggaccgggagaatatctcagagtaccacctcactgctgtcattgtggacaaggacactggtgaaaacctg
gagactccttccagcttcaccatcaaagttcatgacgtgaacgacaactggcctgtgttcacgcatcggttgttcaatgcgtccgtgc
ctgagtcgtcggctgtggggacctcagtcatctctgtgacagcagtggatgcagacgaccccactgtgggagaccacgcctctgt
catgtaccaaatcctgaaggggaaagagtattttgccatcgataattctggacgtattatcacaataacgaaaagcttggaccgaga

gaagcaggccaggtatgagatcgtggtggaagcgcgagatgcccagggcctccgggggactcggcacggccaccgtgct
ggtcactctgcaagacatcaatgacaacttccccttcttcacccagaccaagtacacatttgtcgtgcctgaagacacccgtgtggg
cacctctgtgggctctctgtttgttgaggacccagatgagccccagaaccggatgaccaagtacagcatcttgcggggcgactac
caggacgctttcaccattgagacaaaccccgcccacaacgagggcatcatcaagcccatgaagcctctggattatgaatacatcc
agcaatacagcttcatcgtcgaggccacagaccccaccatcgacctccgatacatgagccctcccgcgggaaacagagcccag
gtcattatcaacatcacagatgtggacgagcccccatttccagcagcctttctaccacttccagctgaaggaaaaccagaagaa
gcctctgattggcacagtgctggccatggaccctgatgcggctaggcatagcattggatactccatccgcaggaccagtgacaag
ggccagttcttccgagtcacaaaaaaggggacatttacaatgagaaagaactggacagagaagtctacccctggtataacctga
ctgtggaggccaaagaactggattccactggaacccccacaggaaaagaatccattgtgcaagtccacattgaagttttggatga
gaatgacaatgccccggag
= nucleic acid sequence coding for SEQ ID NO: 4
= SEQ ID NO: 3

Said at least one anti-VEcadherin antibody or conservative variant thereof most preferably is a specific antibody entity. Preferred specific antibody entities are those which do not bind to any other molecule that may be contained in the fluid into which it is intended to be added; more preferably, they do not bind to any other molecule that may be contained in the serum or plasma of a human individual.

Advantageously, said at least one anti-VEcadherin antibody or conservative variant thereof is a monoclonal antibody.

Anti-VEcadherin antibodies are available to the skilled person. For example, anti-VEcadherin antibodies have been described in WO 98/25946 (IMCLONE Systems; Inventors DEJANA et al.). Anti-VEcadherin antibodies are also commercially available from various companies, such as Gene Tex, Novus Biologicals, Cell Science or Abcam (clone BV9), or Bender MedSystems (anti-human VE-cadherin BIOTIN, product reference BMS 158BT).

Said ligand can be directly or indirectly linked to a detectable label.

The present application also relates to VE-cadherin, to VE-cadherin extracellular region, to antigenic fragments of VE-cadherin and/or of VE-cadherin extracellular region, and to antigenic variants of VE-cadherin and/or of VE-cadherin extracellular region. The application more particularly relates to these VEcadherin-related products, for use to produce an anti-VEcadherin antibody, wherein said antibody is intended for predicting sensitivity or resistance to anti-tumoral treatment.
Preferably, said VE-cadherin is human a VE-cadherin.

The application thus relates to at least one VEcadherin-related element, for use in the production of at least one anti-VEcadherin antibody, said at least one anti-VEcadherin antibody being intended for predicting sensitivity or resistance to anti-tumor treatment, wherein said at least one VEcadherin-related element is selected from:
- VE-cadherin,
- VE-cadherin extracellular region,
- antigenic fragments of VE-cadherin or of VE-cadherin extracellular region,
- antigenic variants of VE-cadherin or of VE-cadherin extracellular region, which derives from said VE-cadherin or VE-cadherin extracellular region by substitution and/or deletion and/or addition of at least one amino acid,
said antigenic fragments or variants having retained the capacity of inducing the formation of antibodies upon administration into a non-human animal, said antigenic fragments or variants being optionally conjugated to a protein that is immunogenic in the species to be immunized and/or being optionally associated to an immunogenicity enhancer adjuvant.

Said non-human animal is used to produce said antibodies. The goal is not to restore or maintain its physical integrity or well-being. Hence, although all due care should be taken to avoid unnecessary sufferings that such a production may induce, said animal may be killed after production of said antibodies.

The invention relates to a method for predicting the resistance or sensitivity of an animal, preferably a patient, to anti-tumor treatment, which comprises analyzing the content (including present or absence) of circulating VE-cadherin that is present in said animal or patient as defined in the appended claims.
Analyzing the content in circulating VE-cadherin is preferably achieved by analyzing the content in VE-cadherin of a fluid sample, which has been previously collected from said animal or patient (e.g., blood or an extracellular fluid), or a fluid sample that is derivable from such a previously-collected fluid sample (e.g., plasma, serum).

In the present application, the term "fragment" or "sub-fragment" does not imply that the polypeptide has necessarily been obtained by cleavage. Indeed, the term also encompasses fragments or sub-fragments that are obtained by recombinant techniques and/or by chemical synthesis. The term reflects that the polypeptide is an isolated polypeptide, which has the same sequence as would have a cleaved fragment or sub-fragment.

Antigenic fragments of VE-cadherin are polypeptides, the sequence of which derives from VE-cadherin by deletion of one amino acid or by deletion of at least two contiguous amino acids. Antigenic VE-cadherin fragments have retained the capacity of inducing the formation of antibodies upon administration into an animal, preferably a non-human animal, said antigenic fragment being optionally (i.e., if desired or required) conjugated to a protein that is immunogenic in the species to be immunized and/or being optionally associated to an immunogenicity enhancer adjuvant. Preferably, an antigenic fragment has retained the capacity of inducing the formation of antibodies that can bind to a VE-cadherin that occurs in an animal, preferably a human being.

Advantageously, said VE-cadherin antigenic fragment is a polypeptide, the sequence of which is identical to the VE-cadherin extracellular region, or to a sub-fragment thereof. Examples of such VE-cadherin fragments comprise the sequence of SEQ ID NO: 2, as well as sequences comprising SEQ ID NO: 2.
Preferred sub-fragments of VE-cadherin extracellular region are those sub-fragments that are antigenic, i.e., those sub-fragments, which have retained a capacity of inducing the formation of antibodies, preferably the formation of antibodies that can bind to a VE-cadherin that occurs in an animal, preferably a human being.
Said sub-fragment of VE-cadherin extracellular region preferably comprises at least ten amino acids, more preferably at least 15 amino acids, even more preferably at least 25 aminocids, most preferably at least 50 aminoacids. Examples of such sub-fragments comprise the fragment of SEQ ID NO: 4 (431 amino acids).

Advantageously, said VE-cadherin conservative variant is a polypeptide, which derives from said VE-cadherin or VE-cadherin extracellular region by substitution and/or deletion and/or addition of at least one amino acid, but has retained the capacity of inducing the formation of antibodies upon administration into an animal, preferably a non-human animal, said antigenic variant being optionally (i.e., if desired or required) conjugated to a protein that is immunogenic in the species to be immunized and/or being optionally associated to an immunogenicity enhancer adjuvant. Preferably, an antigenic variant has retained the capacity of inducing the formation of antibodies that can bind to a VE-cadherin that occurs in an animal, preferably a human being.
The fragment of SEQ ID NO: 4 is an example of antigenic variant, which derives from VE-cadherin, or from VE-cadherin extracellular region, by deletions of (contiguous) amino acids.

Preferred antigenic VE-cadherin variants are those VE-cadherin variants, the sequence of which is at least 80% identical, preferably at least 90% identical, more preferably at least 95% identical, most preferably 100% identical to the parent sequence from which they derive, over the entire length of this parent sequence (full length identity score).

Said VE-cadherin, or antigenic fragment or variant thereof, can optionally be conjugated and/or otherwise associated to an immunogenicity enhancer. For example, said VE-cadherin or at least one fragment thereof can optionally be:
- conjugated to another protein, such as a protein that is immunogenic in the species to be immunized, e.g., keyhole limpet hemocyanin (KLH), serum albumin, bovine thyroglobulin, or soybean trypsin inhibitor, by using a bifunctional or derivatizing agent, for example, maleimidobenzoyl sulfosuccinimide ester (conjugation through cysteine residues), N-hydroxysuccinimide (through lysine residues), glutaraldehyde, succinic anhydrid or SOCl₂,
   and/or
- associated to an adjuvant, such as Freund's (complete or incomplete) adjuvant, mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and Corynebacterium parvum.

If desired or required, more than one of said VEcadherin-related elements can be used, e.g., at least two or three.

Preferably, said at least one VE-cadherin element is used to produce an antibody, which is an anti-VEcadherin specific antibody, e.g., an antibody, which does not bind to any molecule other than VE-cadherin that may be contained in the fluid into which it is intended to be added, more preferably, which does not bind to any molecule other than VE-cadherin that may be contained in the serum or plasma of a human individual. More preferably, said antibody is a monoclonal antibody.

Animals can be immunized with VE-cadherin, or a fragment thereof, or an antigenic functional derivative of VE-cadherin or VE-cadherin fragments, according to known methods. Preferred VE-cadherin fragments are those which have a sequence that is at least 80% identical, preferably at least 90% identical, e.g., 100% identical to the sequence of the extracellular region of a VE-cadherin, or to the sequence of a fragment of VE-cadherin extracellular region, over the entire length of said VE-cadherin extracellular region or of said fragment VE-cadherin extracellular region, respectively. Illustrative VE-cadherin fragments are those of SEQ ID NO: 4 or of SEQ ID NO: 2.

Appropriate animals notably comprise mammals, more particularly non-human mammals, such as rabbit.
For example, a mammal is injected intraperitoneally or subcutaneously with said VE-cadherin (or fragment or variant thereof),
Said VE-cadherin (or fragment or variant thereof) may be diluted with, or suspended in an appropriate volume of PBS (Phosphate-Buffered Saline), physiological saline or the like. An appropriate volume of a standard adjuvant can be mixed with the product, if necessary or desired. Illustrative standard adjuvants notably comprise Freund's (complete or incomplete) adjuvant, mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and Corynebacterium parvum.
It may be useful or advantageous to conjugate said VE-cadherin (or fragment or variant thereof) to a protein that is immunogenic in the species to be immunized, e.g., keyhole limpet hemocyanin (KLH), serum albumin, bovine thyroglobulin, or soybean trypsin inhibitor, by using a bifunctional or derivatizing agent, for example, maleimidobenzoyl sulfosuccinimide ester (conjugation through cysteine residues), N-hydroxysuccinimide (through lysine residues), glutaraldehyde, succinic anhydrid or SOCl2.
The solution is administered to the animals several times, e.g., every 4 to 21 days. In addition, an appropriate carrier can also be used upon immunization with an immunogen.

Polyclonal antibodies are heterogeneous populations of antibody molecules, which can be derived from the sera of animals immunized with said VE-cadherin (or fragment or variant thereof).

Monoclonal antibodies (mAb), which are homogeneous populations of antibodies to a particular antigen, may be obtained by any technique which provides for the production of antibody molecules by continuous cell lines in culture.
These include, but are not limited to:
- the hybridoma technique of Kohler and Milstein (1975) Nature 256:495-497; and U.S. Pat. No. 4,376,110,
- the human B-cell hybridoma technique (Kosbor et al. (1983) Immunology Today 4:72; Cole et al. (1983) Proc. Natl. Acad. Sci. USA 80:2026-2030, and
- the EBV-hybridoma technique (Cole et al. (1985) Monoclonal Antibodies And Cancer Therapy, Alan R. Liss, Inc., pp. 77-96).
Such antibodies may be of any immunoglobulin class including IgG, IgM, IgE, IgA, IgD and any subclass thereof. The hybridoma producing a mAb may be cultivated in vitro or in vivo.
Production of mAb notably comprises the collection of immunocytes, such as splenocytes, from an immunized animal, and the fusion of these immunocytes to a fusion partner.
As a partner cell to be fused with the above immunocyte, a mammalian myeloma cell can be used.
Cell fusion of the above immunocytes with myeloma cells can be basically performed according to a known method, for example, the method of Kohler and Milstein et al (Kohler. G. and Milstein, C., Methods Enzymol. (1981) 73, 3-46).

The thus obtained hybridomas are selected by culturing the hybridomas in a standard selective culture solution such as a HAT culture solution (a culture solution containing hypoxanthine, aminopterin and thymidine). Culture in the above HAT culture solution is continued for a time period sufficient for the cells (unfused cells) other than the target hybridomas to die (normally, several days to several weeks). Subsequently, a standard limiting dilution method is conducted, so that screening for and monocloning of hybridomas that produce a target antibody are performed.

In addition to a method with which the above hybridomas are obtained by immunizing non-human animals with antigens, desired human antibodies having binding activity to said VE-cadherin (or fragment or variant thereof) can also be obtained (see Japanese Patent publication (Kokoku) No. 1-59878 B (1989)), by sensitizing in vitro human lymphocytes with said VE-cadherin (or fragment or variant thereof), and causing the sensitized lymphocytes to fuse with the human-derived myeloma cells having a permanent division potential.

The thus prepared hybridomas producing monoclonal antibodies can be passage-cultured in a standard culture solution, or can be stored for a long period in liquid nitrogen.

One example of a method employed to obtain monoclonal antibodies from the hybridomas involves culturing the hybridomas and obtaining monoclonal antibodies in the culture supernatant according to a standard method. Another method involves administering the hybridomas to mammals that are compatible with the hybridomas to cause them to proliferate, and obtaining monoclonal antibodies in the ascites. The former method is suitable to obtain antibodies of high purity. On the other hand, the latter method is suitable for the mass production of antibodies.

A monoclonal antibody that can be used in the present invention can be a recombinant monoclonal antibody that is prepared by cloning the antibody gene from the hybridoma, incorporating the gene into an appropriate vector, introducing the vector into a host, and then causing the host to produce the recombinant monoclonal antibodies by genetic engineering techniques (e.g., see Vandamme, A. M. et al., Eur. J. Biochem. (1990) 192:767-7755 1990).
In addition to the above host cell, a transgenic animal or plant can also be used to produce a recombinant antibody.

The application also relates to both:
- at least one ligand, which binds to VE-cadherin, preferably to human VE-cadherin, preferably to the extracellular region of said VE-cadherins, and
- a solution, which comprises at least one surfactant,
for use in a kit intended for predicting sensitivity or resistance to anti-tumor treatment.

The application also relates to said kit (as above-described), for use as kit that is intended for the prognosis of resistance or sensitivity to anti-tumor therapy.

The application also relates to a method of identifying at least one pronostic marker of sensitivity or resistance to anti-tumour treatment, which comprises:
- referring to the results of an anti-tumour treatment, which has been previously applied to a population of tumor-containing animals, to identify the sub-population of animals that are resistant to said anti-tumor treatment and the sub-population of animals that are sensitive to said anti-tumor treatment,
- analyzing fluid samples, which have been previously collected from said population of animals, before said animals had received said anti-tumour-treatment, or fluid samples which derives from such pre-treatment collected samples,
   wherein said fluid samples are selected from:
   ∘ samples of blood of said animals,
   ∘ samples of extracellular fluids of said animals,
   ∘ fluids derivable from said blood and/or from said extracellular fluids, such as plasma, serum,
   wherein said fluid sample analysis comprises determining whether at least one analyte is present or absent in every fluid sample of each of said two sub-populations, or determining the concentration at which said at least one analyte is contained in every fluid sample of each of said two sub-populations,
   wherein, for every fluid sample of each of said two sub-populations, said fluid sample analysis is performed by a method comprising:
   - submitting said fluid sample to a contacting step, which comprises:
      - contacting said fluid sample, with at least one ligand that binds to said at least one analyte, and
      - detecting whether said ligand binds to a target in said fluid sample, whereby such a binding is indicative of the presence and/or the the amount of said at least one analyte in said fluid sample, the non-occurrence of such a binding being indicative of the absence of said at least one analyte in said fluid sample,
   - optionally, submitting said fluid sample to a protein separation step, prior to said contacting step, so as to separate the proteins and/or polypeptides that are contained in said fluid sample according to size and/or charge,
   - optionally, submitting said fluid sample to a SDS-denaturation step prior to said protein separation step, so as to denature the proteins and polypeptides that are contained in said fluid sample by incubating it with SDS,
      and
      wherein said contacting step, as well as said optional SDS-denaturation step (if any) and said optional separation step (if any), is/are preceded by the dilution of said fluid sample with a surfactant-containing solution,
   wherein the determination that:
   said at least one analyte is present in one of said two sub-populations and is absent in the other of said two sub-populations, or that
   one of said two sub-populations comprises said at least one analyte at a mean concentration that is (statistically) significantly different from the other of said two sub-populations,
   is indicative of the fact that said at least one analyte is likely to be, preferably is a prognostic marker of sensitivity or resistance to anti-tumor treatment.

The application also relates to a method for monitoring the efficiency of an anti-tumor treatment that is given to an animal,
which comprises determining in a fluid sample selected from:
∘ samples of blood of said animal,
∘ samples of extracellular fluids of said animal,
∘ fluids derivable from said blood and/or from said extracellular fluids, such as plasma, serum,
whether the content of (circulating) VE-cadherin increases during the course of said anti-tumor treatment,
wherein said determination comprises:
- contacting said fluid sample, with at least one ligand that binds to VE-cadherin, and detecting whether said ligand binds to a target in said fluid sample, whereby such a binding is indicative of the presence and/or the amount of VE-cadherin in said fluid sample, the non-occurrence of such a binding being indicative of the absence of VE-cadherin in said fluid sample,
- optionally, submitting said fluid sample to a protein separation step, before said contacting step, so as to separate the proteins and/or polypeptides that are contained in said fluid sample,
- optionally, submitting said fluid sample to a SDS-denaturation step, prior to said protein separation step, so as to denature the proteins and polypeptides that are contained in said fluid sample by incubating it with SDS,
   and
   wherein said contacting step, as well as said optional SDS-denaturation step (if any) and said optional separation step (if any), is/are preceded by the dilution of said fluid sample with a surfactant-containing solution,
   whereby a positive determination is indicative of the fact that said anti-tumor treatment is efficient to treat said tumor in said animal, and/or to decrease or otherwise limit the development of said tumor, and/or to alleviate the side-effects said tumor induces.

Such a method is notably useful for those patients, who are affected by a neoplasm disease or condition, and who have become resistant or non-responsive to a particular anti-tumor treatment, and for which an alternative and more efficient anti-tumor treatment is desired. The method of the invention would then help in selecting the alternative treatment, which is likely to be the most efficient anti-tumor treatment for this particular patient.

### Means for dissociating circulating VE-cadherin from lipid- and/or saccharide-containing entities:

The present invention is believed to enable the dissociation of soluble proteins, more particularly circulating proteins, which are contained in the patients' fluid samples.

Indeed, patients undergoing analysis of an analyte contained in one of their body extracellular fluid, such as plasma, serum or lymph, do not always have an empty stomach. The plasma, serum or lymph collected from them may thus be lipemic. In lipemic samples, proteins are likely to be associated with lipid entities (such as lipoproteins), e.g., in the form of chylomicrons, or of other lipid-protein vesicles. Such lipid-bound proteins may escape immuno-detection due to the masking of the antibody epitope, thereby leading to under-estimated results or to false negative results.
The fluid sample may also comprise saccharide entities, such as glycoproteins.
Hence, when a series of plasma, serum or lymph samples are collected from different patients, some of the collected samples will be very lipemic and/or saccharidic, other will be slightly lipemic and/or saccharidic, while still other samples will be non-lipemic and/or non-saccharidic. In spite of these variations, the same immuno-assay protocol is usually applied to all samples, and, due to lack of appropriate procedure applicable in routine at the hospital level, such a protocol does most generally not comprise any lipid-dissociating step. The improved method of the invention, which teaches to prior-dilute the serum sample in a surfactant-containing diluent, provides a solution to this prior art problem.

The application thus relates to a method for dissociating soluble protein(s) from lipid and/or saccharide entities that may be associated thereto, more particularly, as above-indicated, soluble VE-cadherin.
wherein the fluid wherein said soluble protein(s) is(are) contained contains lipid and/or saccharide entities,
wherein said method comprises in vitro diluting said fluid, or a sample thereof, with a surfactant-containing solution.
The application also relates to at least one surfactant for use to dissociate a fluid-contained soluble protein from lipid and/or saccharide entities that may be associated thereto.

As above-indicated, said surfactant-containing solution does preferably not comprise SDS, more preferably it does not comprise any denaturing surfactant.
Said lipid entities can be any lipid entity that can be naturally-occurring in a circulating fluid, such as vesicles, chylomicrons, micelles.

In accordance with the present invention, and as indicated above, said fluid or sample thereof is diluted in said surfactant-containing solution at a dilution of at least 1/50, which means that, contrary to prior art practice, notably in the field of serum or plasma analysis, the fluid or sample thereof is highly diluted.

Furthermore, the means of the invention do not necessitate the use of any sequestrant.
Indeed, the quantity of surfactant(s) contained in said surfactant-solution can be optimized so as to be a quantity necessary and sufficient to achieve the desired dissociation from lipid. There is no need to have surfactant(s) in excess; it is even not recommended to have it/them, as it might be detrimental to the accuracy of the detection.
As above described, and as below demonstrated, in accordance with the invention, such optimized quantities of surfactant(s) are very small: the concentration in surfactant contained in said surfactant-containing solution does not need to be more than 1%; it advantageously is of no more than 0.5%, e.g., of 0.001%-0.4% (such concentrations being the concentrations before dilution).
For several surfactants, such as Triton® X-100, the concentration in surfactant can even be of 0.004%-0.02%, e.g., of about 0.005%-0.01%.

The term "comprising", which is synonymous with "including" or "containing", is openended, and does not exclude additional, unrecited element(s), ingredient(s) or method step(s), whereas the term "consisting of" is a closed term, which excludes any additional element, step, or ingredient which is not explicitly recited.
The term "essentially consisting of" is a partially open term, which does not exclude additional, unrecited element(s), step(s), or ingredient(s), as long as these additional element(s), step(s) or ingredient(s) do not materially affect the basic and novel properties of the invention.
The term "comprising" (or "comprise(s)") hence includes the term "consisting of" ("consist(s) of"), as well as the term "essentially consisting of" ("essentially consist(s) of'). Accordingly, the term "comprising" (or "comprise(s)") is, in the present application, meant as more particularly encompassing the term "consisting of" ("consist(s) of"), and the term "essentially consisting of" ("essentially consist(s) of").

The following examples are offered by way of illustration, and not by way of limitation.

### EXAMPLES

### EXAMPLE 1:

- **dilution of plasma or serum in a surfactant-containing buffer drastically improves analysis of circulating VE-cadherin;**
- **detection of circulating VE-cadherin in human serum in the form of an about 90 kDa fragment.**

### Material and methods

### Chemical Products

Acrylamide/Bisacrylamide (37.5:1) (Sigma); Surfactants: Triton® X-100, CHAPS, DOC, CEDA (Sigma); Tween® 20 (Sigma); TEMED (Sigma); SDS (Bioprobe); Ethanol, methanol (Sigma); Hoechst Reagent (Sigma); Laemmli 2X buffer (Sigma); PBS (calcium and magnesium free) (BioWhittaker); Gelatin (Sigma); PNNP (Sigma).
The PBS buffer that is used in the present and following examples is obtainable by diluting to 1X in H₂O a 10X stock solution of Ca-free and Mg-free PBS buffer, such as the 10X PBS stock solution available from Biowittaker (10X Ca- and Mg-free PBS: KH₂PO₄ 1440 mg/L; NaCl 90,000 mg/L; Na₂HPO₄•2H₂O 7,950 mg/L), and by adding to this 1X solution at least one anti-protease, such as 25 mg/mL leupeptin, and 1mM EGTA

### Plasticware

75 cm² vented caps culture flask (Falcon); 4 wells multidish (NUNC); Polypropylene conical tube: 50 and 15 ml (Falcon).

### Kits

Chemiluminescence detection kit (Perkin Elmer Life Sciences).

### Antibodies

- peroxidase-coupled antibody: goat anti-mouse IgG coupled to HRP (Horse Radish Peroxidase) (Bio-Rad 170-6516);
- anti-VEcadherin: clone BV9, mouse mAb, which recognizes a recombinant fragment spanning the extracellular juxtamembrane domains EC3 through EC4 (available from Abcam -product datasheet ab7047-);
- sheep anti rabbit IgG Biotin (US Biological MA, USA);
- streptavidin-alkaline phosphatase mAb (ELISA), available from Sigma (ref. S2890);
- rabbit polyclonal anti-VEcadherin antibody (ELISA), available from Bender (CAD3 epitope; product reference BMS253MSTCA).

### Other

Autoradiographic film (Kodak), Nitrocellulose (Scleicher & Schuell).

### Serum samples:

The serum samples of healthy human volunteers were provided by the *Établissement Français du Sang* (*Établissement Français du Sang;* 29, avenue du Maquis du Grésivaudan; 38701 La Tronche; France).

### Western Blot Analysis

### Dilutions of the serum samples prior to VE-cadherin analysis:

Serum samples were serially diluted at 4°C down to 1/500 (serial dilution from 1 to 10, and then from 1 to 50) either in a surfactant-free PBS buffer (Figure 1A), or in a surfactant-containing PBS buffer (Figures 1B, 2 and 3):
   - Figure 1B: Triton® X-100 at 0.5% in PBS (Figure 1B), or
   - Figure 2: Triton® X-100, or CHAPS, or DOC, or CDEA, at 0.5% in PBS (Figure 2), or
   - Figure 3: Triton® X-100 at 0.001%, 0.005%, 0.01%, 0.05% or 0.5% in PBS (lanes 1-5); or Tween® 20 at 0.05% or 0.5% in PBS (lanes 6-7); or SDS at 0.5% in PBS (lane 8).
All surfactant percentages are w/v percentages.

### SDS-PAGE and western blot of the diluted serum samples:

5µL of loading buffer adapted to SDS-PAGE under denaturing and reducing conditions (Laemmli β-mercaptoethanol 2X buffer) were added to 5 µL of each diluted sample; and the resulting 10 µL solution was subjected to SDS-PAGE under denaturing and reducing conditions (12% polyacrylamide gel).
After SDS-PAGE under denaturing and reducing conditions, the separated proteins were electro-transferred onto nitrocellulose. The nitrocellulose was blocked with 5% low fat milk in PBS-Tween 0.05%, and incubated at 4°C overnight with the anti-VE cadherin mouse mAb BV9 at a dilution of 1/5,000 from an initial concentration of 1 mg/mL in PBS-Tween-Milk (i.e., BV9 at 0.2µg/mL).
After being washed three times in PBS-Tween-Milk, the membrane was incubated for four hours at 4°C with a goat anti-mouse HRP-coupled secondary antibody at a dilution of 1/3,000 (in accordance with the supplier's instructions) in PBS-Tween-Milk. After 5 washes in PBS-Tween, the nitrocellulose membrane was incubated in chemiluminescence reagent for a few minutes before the immunoreactive bands were visualized by autoradiography (Figures 1A, 1B, 2, 3A). The immunoreactive bands were scanned, to plot the obtained values in a graph (Figure 3B).

### Results

### Dilution of sample in [PBS + Triton® X-100] better than in PBS (Figures 1A, 1B):

Serum samples of healthy human volunteers were diluted from 1 to 10, and then from 1 to 50 either in PBS (**Figure 1A**), or in PBS containing 0.5% Triton® X-100 (**Figure 1B**), and analyzed by western blot for the presence of the VE-cadherin extracellular fragment (SDS-PAGE under denaturing and reducing conditions, detection by BV9 mAb).

A 90 kDa form of VE-cadherin is detected, suggesting that circulating VE-cadherin is a truncated fragment of VE-cadherin (fragment of extracellular region). Neither cell nor membrane was detected in the serum sample, as analyzed by immunofluorescent staining with BV9. Hence, the 90kDa form of VE-cadherin that is detected in the serum is in a soluble form of VE-cadherin (truncated form).

The presence of Triton® X-100 in the sample diluent highly improves the detection of the VE-cadherin that is contained in the serum samples (90 kDa form).
In western blot, 1/100 and 1/500 dilutions gave better results than 1/10 or 1/50 dilutions. Furthermore, serial dilution (e.g., from 1 to 10, and then from 1 to 10, for a 1/100 dilution; or from 1 to 10, and then from 1 to 50, for a 1/500 dilution) gives better results than direct dilution (from 1 to 100, or from 1 to 500, respectively).

Hence, diluting the serum or plasma sample in a surfactant-containing buffer, such as a Triton® X-100 containing buffer, before subjecting it to standard protein analysis, such as standard western blot, highly improves the detection of the circulating VE-cadherin that is contained in a serum or plasma sample.
Preferably, said dilution comprises the stirring, advantageously the vortexing, of the diluted sample.
Preferably, said dilution is a serial dilution.
For serum or plasma samples, the dilution advantageously is of at least 1/50.

### Detergents other than Triton® X-100 (Figure 2; Figures 3A, 3B):

Serum samples of healthy human volunteers were diluted (from 1 to 10, and from 1 to 50), either in Triton® X-100 (non ionic detergent), or in CHAPS (zwitterionic detergent), or in CDEA (cationic detergent), or in DOC (anionic detergent), each detergent being used at 0.5% in PBS (w/v). The serum samples were then analyzed by western blot for presence of VE-cadherin using the BV9 mAb. Illustrative results are shown in **Figure 2****. No** significant difference is observed between these different types of detergents (Triton® X-100, or CHAPS, or CDEA, or DOC, at 0.5%).

Figures 3A and 3B show that:
- Triton® X-100 is active at a concentration as low as 0.001% in the dilution buffer,
- among those Triton® X-100 concentrations that have been tested, Triton® X-100 at 0.01% in the dilution buffer is the concentration that gives the maximal signal for the detection of circulating VE-cadherin in serum,
- when human serum is diluted at 1/500 (serial dilution) in a Triton® X-100 containing buffer, the signal for the detection of circulating proteins in western blot stabilizes at a Triton® X-100 concentration that is comprised between 0.005% and 0.05%, more precisely between 0.005% and about 0.01%, of Triton® X-100 in the dilution buffer (lower and higher limits of the % included),
- Tween® 20 enables to detect circulating VE-cadherin, even when Tween® 20 is used at a concentration as low as 0.05% in the dilution buffer, and
- the detection signal is increased when Tween® 20 is used at 0.5%.

Figures 3A and 3B also show that the surfactant SDS is drastically less efficient than the other surfactants, as SDS at 0.5% in the dilution buffer is less efficient than Triton® X-100 at 0.005% or Tween® 20 at 0.5%. SDS is less efficient because it precipitates at 4°C (temperature at which the serum dilutions were made) and/or because it is a denaturing surfactant.

### Conclusions:

Prior art analysis of serum- or plasma-circulating VE-vadherin is performed on a crude sample of serum or plasma, i.e., by direct analysis of a 1 µL fraction of the collected serum or plasma.
The inventors demonstrate that said analysis is improved by dilution of the serum or plasma in a surfactant-containing buffer. The presence of a surfactant in said dilution buffer improves the quality of the VE-cadherin detection, more particularly its sensitivity (for example, compare the lanes corresponding to dilutions 1/100 or 1/500 of Figure 1A - PBS only-, to the ones of Figures 1B -PBS with Triton® X-100-).
Hence, the inventors provide an improved technique for the detection of circulating VE-cadherin, more particularly of the VE-cadherin that is contained in serum or plasma, wherein said improved technique comprises the dilution of said fluid (e.g., serum or plasma), or a sample thereof, in a buffer, and wherein said buffer preferably comprises a surfactant.

The concentration of surfactant contained in said buffer does not need to be very high. As above-illustrated, concentrations of about 0.001 to 0.5% (w/v) of surfactant in buffer were sufficient to achieve the improved detection of circulating VE-cadherin. It is recommended to use such optimized quantities of surfactant, and not to overload the buffer, as the presence of very high concentrations of surfactant may be detrimental to the quality of the SDS-PAGE migration.
The inventors further demonstrate that, for a western blot analysis, the optimal concentrations of Triton® X-100 are of 0.005-0.05%, preferably of about 0.005-0.01%
(initial concentration of Triton® X-100 in buffer, before dilution of the serum at 1/500), and that the denaturing surfactant SDS is drastically less efficient that the other non-denaturing surfactants that have been tested (Triton® X-100, Tween® 20, CHAPS, DOC, CDEA).
The improved technique of the inventors further enabled to detect a circulating form of VE-cadherin, which is of about 90 kDa (apparent MW on western blot), which corresponds to a fragment of VE-cadherin extracellular region. To the best of the inventors' knowledge, such a fragment of VE-cadherin extracellular region has never been described in the prior art. These results suggest that the VE-cadherin that circulates in the human serum is a 90 kDa acellular fragment of VE-cadherin extracellular region.

### EXAMPLE 2: the presence of circulating VE-cadherin in human serum, plasma or blood (as detected by using the method described in example 1) is predictive of sensitivity to cancer treatment (chemotherapy and/or radiotherapy)

### Materials and Methods

### Chemical Products

Acrylamide/Bisacrylamide (37.5:1) (Sigma); Triton® X-100, Tween-20 (Sigma); TEMED (Sigma); SDS (Bioprobe); Ethanol, methanol (Sigma); Hoechst Reagent (Sigma); Laemmli 2X loading buffer (Sigma); PBS (calcium and magnesium free; BioWhittaker, see example 1); Gelatin (Sigma).

### Plasticware

75 cm² vented caps culture flask (Falcon); 4 wells multidish (NUNC); Polypropylene conical tube: 50 and 15 mL (Falcon).

### Commercial kits

Chemiluminescence detection kit (Perkin Elmer Life Sciences)

### Medium and cell culture products

DMEM (GibcoBRL)
Trypsin-EDTA (GibcoBRL)
Fetal Bovine Serum (Biochrom)
Antibiotics: penicillin, streptomycin (GibcoBRL)

### Antibodies

anti-VEcadherin mAb:
- BV9: mouse mAb, which binds to human VE-cadherin, and recognizes a recombinant fragment spanning the extracellular juxtamembrane domains EC3 through EC4 (available from Abcam (Cambridge, MA, USA), product datasheet ab7047);
- C19: mAb, which recognizes the C-terminal part of VE-cadherin (available from Santa Cruz Biotechnology).
Cyanin-coupled antibody: rabbit anti-goat IgG (Jackson Immunoresearch). Peroxidase-coupled antibody: goat anti-mouse IgG coupled to HRP (Horse Radish Peroxidase), available from Bio-Rad (product reference 170-6516).

### Other

Autoradiographic film (Kodak)
Nitrocellulose (Scleicher & Schuell)

### Serum and plasma samples from tumor patients and from healthy volunteers

All patients were diagnosed with a primary brain tumor: malignant oligodendrogliomas (grade III), glioblastomas or malignant oligo-astrocytomas (in accordance with the WHO classification of tumors of the central nervous system; see e.g., Gonzales 2001, J. Clin. Neurosci 8(1):1-3). Patients were treated with temozolomide alone (Temodal®), or temozolomide associated to radiotherapy following the stupp protocol (New England Journal of Medicine, 2005, March 10, 352(10): 987-996). Response to therapy was investigated using RMI following the cairncross criteria.
Response to therapy was observed in 12 patients (11 oligodendroglioma/oligoastrocytomas, 1 glioblastoma), and non-response in 14 patients (7 oligo-oligo-astrocytomas and 7 glioblastomas).

For the Western blot analysis, the cohort has been extended to a total of 56 glioma patients: response to therapy was observed in 28 of them, and non-response to the 28 other patients.
Patients' blood was collected, upon arrival to the hospital (*Centre Hospitalier Universitaire de Grenoble,* Neuro-oncology Department, Grenoble, France), prior to any treatment. Blood was collected in an anticoagulant-containing tube. Plasma was recovered by centrifugation; serum was recovered by removal of the clotting factors.
The serum and plasma samples were frozen (at -80°C) in aliquot fractions of 20 µL. All further steps were performed at 4°C on ice.
All samples were collected in accordance with the protocols approved by the *Etablissement Français du Sang* (29, avenue du Maquis du Grésivaudan; 30701 La Tronche; France).
Sera from ten healthy human volunteers were provided by the *Établissement Français du Sang,* and treated following the same procedures as the patient's samples.

### Western Blot Analysis

Serum or plasma samples were:
- serially diluted at 1/500 (from 1 to 10, and then from 1 to 50) in a PBS buffer comprising 0.5% (w/v) Triton® X-100, and then
- mixed the SDS-PAGE sample buffer (5 µL of diluted serum or plasma, with 5µL of SDS-PAGE sample buffer).
The SDS-PAGE sample buffer was Laemmli 2X concentrate (4% SDS, 10% β-mercaptoethanol, 20% glycerol, 0.004% bromphenol blue and 0.125M Tris-HCl, pH of about 6.8).
The resulting 10 µL samples were subjected to SDS-PAGE under denaturing and reducing conditions, as described in example 1.

### Results and Discussion

### 1. Presence of circulating VE-cadherin in healthy human serum and plasma

We analyzed different dilutions of healthy human sera by western blot for the presence of circulating VE-cadherin. Results are illustrated in **Figures 4A and 4B****.**
The Coomassie staining (Figure 4A) shows that there are many different proteins present in the serum. The most abundant of these proteins is a band at about 68 kDa, which represents albumin. We tested different dilutions of serum by western blot, and found that below a concentration of 1/500, the VE-cadherin was no longer detectable. We observed that only the 90 kDa fragment of VE-cadherin was present in the serum. This could reflect a partial proteolysis at the angiogenic site, or perhaps cleavage in the circulation.
Because serum samples are collected after coagulation, we could not be sure that the samples were free of proteases that are activated during the coagulation process. Thus, we analyzed plasma samples, which were collected in heparin-containing tubes (heparin inhibits proteolytic activity). Coomassie staining and western blot are illustrated in **Figures 5A and 5B****.** The immunoreactive band corresponding to VE-cadherin was observed in plasma at the same molecular weight as in serum. Thus, we can conclude that a 90 kDa circulating VE-cadherin is present in human serum and plasma samples, and that it is not due to way in which the samples were collected.

### 2. Circulating VE-cadherin as an angiogenic marker of glioblastomas: differential level between chemotherapy responsive and non responsive patients

24 human serum samples were collected from glioblastoma patients before chemotherapy and/or radiotherapy. The patients were then classified as being responsive (= sensitive) or non-responsive (= resistant) to the therapy, and the samples were analyzed by western blot.
Ten of the 12 non-responsive samples show an obviously lower signal of immuno-reactive VE-cadherin, than the 12 responsive samples run on the same gel.
We observed that the 90 kDa form of human VE-cadherin was highly detectable in serum from responsive patients, while it was almost undetectable in serum from non-responsive patients. Results are illustrated in **Figure 6****.**
Western blot results obtained with an extended cohort of 56 glioma patients (28 responsive and 28 non-responsive patients) were the same as those already observed with the initial cohort of 24 patients.

Hence, circulating VE-cadherin is much more abundant in the serum of those patients, who will be responsive to chemotherapy and/or radiotherapy, than in the serum of those patients, who won't be responsive to anti-tumor therapy, more particularly to chemotherapy.

### Conclusion

The application of the improved technique described in example 1 above (surfactant-containing buffer) enabled the inventors to determine that circulating VE-cadherin has a prognostic value to predict the responsiveness of cancer patients to treatment, such as glioma patients, or other cancer patients, such as lung, liver, breast, kidney, prostate cancer patients.

It is believed that this prognostic value could not have been determined with the prior art techniques of circulating protein detection.
Using the technique of the invention, the inventors demonstrate that presence or absence of circulating VE-cadherin, more particularly of a 90 kDa form of VE-cadherin (extracellular part of VE-cadherin), in the sera of glioblastoma patients has a prognostic value to determine whether these patients will be responsive or non-responsive to chemotherapy and/or radiotherapy.

Absence of detectable VE-cadherin in serum or plasma, or presence of at a sub-normal level (i.e., significantly lower than the average level observed in healthy patients, as assessed by the technique of the invention) is indicative of the fact that the patient is at high risk of being non-responsive to anti-tumor treatment, more particularly to anti-glioma treatment by chemotherapy, or by chemotherapy and radiotherapy. The higher the difference between said sub-normal level and said normal level, the higher the probability of said patient being at risk of being non-responsive to anti-tumor treatment.
Presence of VE-cadherin at a normal level (not significantly lower than the average level observed in healthy patients, as assessed by the technique of the invention) is indicative of the fact that the patient is at high risk of being responsive to anti-cancer treatment, more particularly to anti-glioma treatment by chemotherapy, or chemotherapy and radiotherapy.

The invention enabled to determine that when accurately determined, e.g., by using the method of the invention, the presence and/or content in circulating proteins involved in angiogenesis and/or inflammation, such as circulating VE-cadherin, can be predictive of the response of a tumor to treatment.
The inventors believe that these means of the invention are applicable to any circulating protein that is involved in an angiogenic and/or inflammatory mechanism(s), and that the invention enables an accurate detection, and optionally quantification, of such proteins. Accordingly, the inventors believe that the means of the invention may be applicable to pathologies other than cancer or tumour, namely any condition involving proteins which are involved such as vascular pathologies, sepsis pre-eclampsia, rheumatoid arthritis.

### EXAMPLE 3: optimization of anti-VEcadherin mAb concentration for western blot analysis

Serum samples of healthy human volunteers (cf. example 1 above) were:
- serially diluted at 1/500 (from 1 to 10, and then from 1 to 50) in a buffer consisting of 0.5% (w/v) Triton® X-100 in PBS, and then
- mixed the SDS-PAGE sample buffer (5 µL of diluted serum or plasma, with 5µL of SDS-PAGE sample buffer).
The SDS-PAGE sample buffer was Laemmli 2X concentrate (4% SDS, 10% β-mercaptoethanol, 20% glycerol, 0.004% bromphenol blue and 0.125M Tris-HCl, pH of about 6.8).
The resulting 10 µL samples were subjected to SDS-PAGE under denaturing and reducing conditions, as described in example 1, except that a range of different anti-VEcadherin mAb (BV9) concentrations have been assayed.

**Figures 8A, 8B** illustrate the results obtained with a BV9 concentration ranging from 0.02 to 1 µg/mL.
Circulating human VE-cadherin is detected by western blot using a concentration in anti-VEcadherin mAb (BV9 mAb; available from Abcam -product datasheet ab7047-) as low as 0.02 µg/mL. A plateau is reached at a concentration of 0.1-1 µg/mL. Hence, a concentration of about 0.2 µg/mL of anti-VEcadherin mAb is an appropriate optimal concentration for implementation of the invention in western blot.

The optimal concentrations in anti-VEcadherin mAb to implement the invention in western blot are different from the concentrations that are recommended by the mAb manufacturer, which are of about 10 µg/mL (BV9 concentration, which is recommended by Abcam for western blot).

### EXAMPLE 4: quantification of circulating proteins contained in a biological fluid, such as serum or plasma

### Material and methods

### Recombinant fragment of VE-cadherin

A recombinant VE-EC1-4 expression vector was produced as described in Legrand P. et al. ("Self-assembly of the vascular endothelial cadherin ectodomain in a Ca2+-dependent hexameric structure", J. Biol. Chem. 2001 Feb 2; 276(5): 3581-8), to produce a fragment of human VE-cadherin extracellular region (CAD 1-4 fragment, corresponding to Asp48-Glu478; nucleic acid sequence **SEQ ID NO: 3;** amino acid sequence **SEQ ID NO: 4**). This CAD 1-4 fragment is used as standard.
A range of CAD1-4 standard solutions are made from an initial standard solution containing CAD1-4 at 1 µg/mL in a Tris buffer, which contains or does not contain any surfactant. The first dilution range is of 1/100, and then six times at ½ (resulting in a range of standard solutions containing CAD1-4 at 10 ng/mL; 5 ng/mL; 2.5 ng/mL; 1.25 ng/mL; 0.63 ng/mL; 0.31 ng/mL; 0.16 ng/mL).

### Serum samples

The serum samples of glioma patients and of healthy human volunteers are the same as those of example 2 above.

### Chemical Products

Acrylamide/Bisacrylamide (37.5:1) (Sigma), Triton® X-100 (Sigma), Tween® 20 (Sigma), SDS (Bioprobe), Ethanol, methanol (Sigma), Hoechst Reagent (Sigma), Laemmli 2X buffer (Sigma), PBS (calcium and magnesium free, as in example 1) (BioWhittaker), Gelatin (Sigma), PNNP (Sigma).
The Tris buffer is a Tris 50 mM HCl pH 8 buffer, containing 0.1% BSA (Hermant et al. 2003, JBC 18 April 2003, 278(16): 14002-14012).

### Plasticware

75 cm² vented caps culture flask (Falcon), 4 wells multidish (NUNC), Polypropylene conical tube: 50 and 15 ml (Falcon)

### Kits

Chemiluminescence detection kit (Perkin Elmer Life Sciences)

### Antibodies

- peroxidase-coupled antibody: goat anti-mouse IgG coupled to HRP (Horse Radish Peroxidase) (Bio-Rad 170-6516);
- anti-VEcadherin: clone BV9, mouse mAb, which recognizes a recombinant fragment spanning the extracellular juxtamembrane domains EC3 through EC4 (available from Abcam -product datasheet ab7047-);
- anti Flk1 (A-3) sc6251 (1158-1345 C Terminus) (Tebu-Bio);
- sheep anti rabbit IgG Biotin (US Biological MA, USA);
- streptavidin-alkaline phosphatase mAb (ELISA), available from Sigma (product reference S2890);
- rabbit polyclonal anti-VEcadherin antibody (ELISA), available from Bender (CAD3 epitope; product reference BMS253MSTCA).

### Other

Autoradiographic film (Kodak), Nitrocellulose (Scleicher & Schuell).

### Western Blot Analysis

The CAD 1-4 standard solutions were submitted to western blot analysis as described in example 1.

### ELISA

The CAD 1-4 standard solutions were diluted in a Tris buffer containing either no detergent, or Tween® 20 (0.05 or 0.5%), or Triton® X-100 (0.001; 0.005 or 0.5%) or SDS (0.5%). The diluted standard solutions were submitted to ELISA.
Serum samples were diluted at the indicated dilutions (1/10; 1/50; 1/100; 1/200; 1/400: 1/500 or 1/1000) in a Tris buffer containing 0.005% Triton® X-100. The diluted serum samples were submitted to ELISA.

The ELISA microplate wells were coated with VE-cadherin antibody BV9 (6h 37°C). Detection is performed with rabbit polyclonal anti-VEcadherin (CAD3 epitope) (5.4 µg/mL), anti-rabbit IgG-biotin conjugate, and streptavidin-alcaline phosphatase + PNNP (substrate).

### Results

### Quantitative positive control:

The Asp48-Glu478 fragment **(SEQ ID NO: 4)** of human VE-cadherin extracellular domain is detected on western blot at a quantity as low as 1 ng.
When this fragment is analyzed by SDS-PAGE and western blot, with the anti-VEcadherin antibody BV9 at the concentrations used for the serum samples (see example 2), this fragment migrates with an apparent molecular weight of about 50 kDa. The lowest quantity that we could detect was 1 ng of the purified protein. See **Figure 9****.**
Such a fragment of VE-cadherin extracellular region can reliably be used as an internal positive control, more particularly as a quantitative internal control, to quantify VE-cadherin in human serum.

### ELISA (standard solutions):

A range of standard solutions of a fragment of the VE-cadherin extracellular region (fragment CAD 1-4; SEQ ID NO: 4) are produced to provide a range of CAD 1-4 standard solutions in a detergent-free buffer (Tris), or in a detergent-containing buffer (Triton® X-100 at 0.5% w/v in Tris).
The ranges of standard solutions are submitted to ELISA with BV9 mAb as capture mAb. Illustrative results are shown in **Figures 10A-10D****.**
The presence of Tween® 20 or Triton® X-100 in the buffer does not alter the reliability of a quantification by ELISA (compare Figures 10B and 10C to Figure 10A).
Using Triton® X-100 at 0.005% in the buffer to dilute the CAD1-4 solutions (Figure 10C) even enables to obtain a quantification regression line that is similar to the one obtained in the absence of detergent (Figure 10A).
Triton® X-100 at 0.005% therefore is the best surfactant concentration to quantify circulating proteins by ELISA.

The surfactant SDS does not enable to achieve a linear regression line (Figure 10D), and is therefore not suitable for quantification by ELISA. It is likely due to the fact that it is a surfactant which precipitates at 4°C and/or to the fact that it is a denaturing surfactant.

### ELISA (serum samples):

Serum samples of human healthy volunteers were diluted at 1/10, 1/50, 1/100, 1/500 or 1/1000 in a Tris buffer containing Triton® X-100 at 0.005%. The diluted serum samples were subjected to ELISA for detection of VE-cadherin.

The CAD 1-4 standard regression line is shown in **Figure 11****.**

Illustrative results obtained with the serum samples are shown in table 1 below.

**Table 1:**

| OD 1 | OD 2 | mean OD | corr. OD | dilution | ng/mL |
|---|---|---|---|---|---|
| 2.293 | 2.366 | 2.330 | 1.264 | 50 | 782 |
| 1.670 | 1.654 | 1.662 | 0.596 | 100 | 738 |
| 0.859 | 0.850 | 0.855 | -0.212 | 500 | ND |
| 0.783 | 0.815 | 0.799 | -0.267 | 1000 | ND |

| | | | | | |
|---|---|---|---|---|---|
| corr. OD = corrected mean = (mean OD - OD at 0 ng/mL) | | | | | |

Table 1 shows that VE-cadherin in serum is detected at a dilution of 1/50 and 1/100, indicating a concentration of abour 800 ng/mL (serum of healthy human volunteer).
Table 1 also shows that VE-cadherin is not detected by ELISA at dilutions 1/500 and 1/1000.

Serum samples of human healthy volunteers were then diluted at 1/10, 1/50, 1/100, 1/200, 1/400 or 1/500 in a Tris buffer containing Triton® X-100 at 0.005%. The diluted serum samples were subjected to ELISA for detection of VE-cadherin.

The CAD 1-4 standard regression line is shown in **Figure 12****.**
Values obtained with the CAD 1-4 positive control are shown in Table 2 below.

**Table 2:**

| **ng/mL** | **OD1** | **OD2** | **mean OD** | **Corr. OD** |
|---|---|---|---|---|
| 0 | 0.710 | 0.700 | 0.705 | 0.000 |
| 0.63 | 0.794 | 0.789 | 0.792 | 0.087 |
| 1.25 | 0.867 | 0.829 | 0.848 | 0.143 |
| 2.5 | 0.988 | 0.985 | 0.987 | 0.282 |
| 5 | 1.313 | 1.208 | 1.261 | 0.556 |
| 10 | 1.763 | 1.758 | 1.761 | 1.056 |

| | | | | |
|---|---|---|---|---|
| corr. OD = corrected mean = (mean OD - OD at 0 ng/mL) | | | | |

Illustrative results obtained with the serum samples are shown in table 3 below.

**Table 3:**

| | **OD 1** | **OD 2** | **Mean OD** | **Corr. OD** | **dilution** | **ng/mL** |
|---|---|---|---|---|---|---|
| | | | | | | |
| **serum #16** | 2.268 | 2.105 | 2.187 | 1.482 | 50 | 692 |
| | 1.562 | 1.444 | 1.503 | 0.798 | 100 | 745 |
| | 1.103 | 1.002 | 1.053 | 0.348 | 200 | 649 |
| | 0.808 | 0.718 | 0.763 | 0.058 | 400 | ND |
| | 0.712 | 0.784 | 0.748 | 0.043 | 500 | ND |
| | | | | | | |
| **serum # 17** | 1.892 | 1.994 | 1.943 | 1.238 | 50 | 578 |
| | 1.551 | 1.348 | 1.450 | 0.745 | 100 | 695 |
| | 1.093 | 1.033 | 1.063 | 0.358 | 200 | 669 |
| | 0.792 | 0.811 | 0.802 | 0.097 | 400 | ND |
| | 0.717 | 0.687 | 0.702 | -0.003 | 500 | ND |

| | | | | | | |
|---|---|---|---|---|---|---|
| corr. OD = corrected mean = (mean OD - OD at 0 ng/mL) | | | | | | |

Table 3 shows that the serum of healthy human volunteer contains about 700 ng/mL of circulating VE-cadherin, which is agreement with the results of Table 1 above, and which confirms the reproducibility of the ELISA method of the invention applied to the detection of VE-cadherin.

Table 3 also shows that, in accordance with the present invention, the dilution factor that is recommended to dilute the serum or plasma in the surfactant containing buffer is of about 1/100-1/200, when the protein detection is performed by ELISA.

### EXAMPLE 5: application of the ELISA method of the invention to the quantification of circulating VE-cadherin, for the prediction of therapy-responsive and therapy-resistant cancer patients

Serum samples of glioma patients are those described in example 2 above.
These serum samples were collected before any treatment has been applied to the patients, and were classified as "therapy-sensitive" or "therapy-resistant" after the patient had been submitted to chemotherapy and/or radiotherapy.

These serum samples were serially diluted at 1/100 and 1/200 in a Tris buffer (Tris 50 mM, HCl pH 8, as in example 4), which contains 0.005% Triton® X-100. The diluted serum samples were submitted to ELISA as described in example 4.

The CAD 1-4 standard regression line is shown in **Figure 13****.**
Values obtained with the CAD 1-4 positive control are shown in Table 4 below.

**Table 4:**

| **ng/mL** | **OD** | **OD** | **mean OD** | **Corr. OD** |
|---|---|---|---|---|
| 0 | 0.918 | 0;853 | 0.886 | 0 |
| 0.63 | 0.975 | 0.899 | 0.937 | 0.051 |
| 1.25 | 1.072 | 0.932 | 1.002 | 0.116 |
| 2.5 | 1.262 | 1.086 | 1.174 | 0.288 |
| 5 | 1.345 | 1.451 | 1.398 | 0.512 |
| 10 | 1.902 | 1.995 | 1.949 | 1,063 |

| | | | | |
|---|---|---|---|---|
| corr. OD = corrected mean = (mean OD - OD at 0 ng/mL) | | | | |

Illustrative results obtained with the patients' serum samples are shown in Table 5 below.

Patients GP, BJ and Mat were non-responsive to chemotherapy and/or radiotherapy (NR patients), whereas patients MT, Can and DD were responsive to chemotherapy and/or radiotherapy (R patients).

**Table 5:**

| sera | | **OD 1** | **OD 2** | **Mean OD** | **Corr. OD** | **dilution** | **ng/mL** |
|---|---|---|---|---|---|---|---|
| | | | | | | | |
| **NR** | **GP** | 1.114 | 1.246 | 1.180 | 0.294 | 100 | 278 |
| | | 0.947 | 0.895 | 0.921 | 0.035 | 200 | 66 |
| | **BJ** | 0.914 | 0.885 | 0,900 | 0.014 | 100 | 13 |
| | | 0.759 | 0.828 | 0.794 | -0.093 | 200 | ND |
| | **Mat** | 0.924 | 0,905 | 0,915 | 0,029 | 100 | 27 |
| | | 0.900 | 0.941 | 0.921 | 0.035 | 200 | 65 |
| | | | | | | | |
| **R** | **MT** | 2.136 | 2.022 | 2.079 | 1.193 | 100 | 1129 |
| | | 1.678 | 1.693 | 1.686 | 0.800 | 200 | 1513 |
| | **Can** | 1.311 | 1.358 | 1.335 | 0.449 | 100 | 424 |
| | | 1.074 | 1.047 | 1.061 | 0.175 | 200 | 330 |
| | **DD** | 1.418 | 1.425 | 1.422 | 0.536 | 100 | 507 |
| | | 1.112 | 1.139 | 1.126 | 0.240 | 200 | 453 |

| | | | | | | | |
|---|---|---|---|---|---|---|---|
| corr. OD = corrected mean = (mean OD - OD at 0 ng/mL) | | | | | | | |

The results obtained by ELISA in accordance with the present invention (Table 5 above) correlates with those that had been observed in western blot (see Figure 6): the VE-cadherin immunoreactivity in the serum samples of those patients, who are resistant to anti-tumor therapy, is significantly inferior to the VE-cadherin immuno-reactivity detected in the serum samples of those patients, who are sensitive to anti-tumor therapy.
This correlation between the concentration in circulating VE-cadherin and the future response of the patient to anti-tumor therapy is further illustrated by Figure 14, which shows a wetern blot analysis of the VE-cadherin contained in the serum of a patient, who will be resistant to anti-tumor therapy (patient "MT"), and of a patient, who will be sensitive to anti-tumor therapy (patient "GP").

Diluting the serum samples at 1/200 (in the surfactant-containing buffer) appears to be an optimal dilution rate for ELISA detection, as a reliable linearity is observed between dilution 1/100 and 1/200.

On the basis of a 1/200 dilution of the serum sample, resistance to anti-tumor therapy can be reliably predicted (at a very high probability) when the concentration of VE-cadherin is inferior to 100 ng/mL in serum.
When the VE-cadherin concentration in serum is of about 500 ng/mL or above, e.g., of about 500-1000 ng/mL, sensitivity to tumor treatment (chemotherapy and/or radiotherapy) can be reliably predicted. Such values are in fact closed to those observed in normal healthy human volunteers (700-800 ng/mL).
Hence, the invention provides a quantitative assay (ELISA), which enables to reliably predict whether a tumor patient is likely to be resistant or sensitive to anti-tumor treatment.

### EXAMPLE 6 (comparative example): the commercially-available prior art kit does not enable the detection of VE-cadherin in the serum or plasma of cancer patients.

The commercially-available kit for the detection of soluble VE-cadherin is an ELISA kit, available from Bender MedSystems GmbH (Campus Vienna Biocenter 2; A-1030 Wien; Austria) -product reference BMS253-. This prior art kit was used in accordance with the manufacturer's instructions, in an attempt to detect VE-cadherin in human serum.
In accordance with the manufacturer's instructions, 20 µL of serum sample were mixed with 80 µL of the sample diluent that is provided in the kit (Proclin300 diluent), and were subjected to ELISA with the mAb provided in the kit, without any prior dilution in a surfactant-containing buffer.

### Standard curve:

Standard VE-cadherin solutions were made to determine the standard curve for the OD measures made with the commercial prior art kit.

OD values are shown in table 6 below.

**Table 6:**

| Standards | | | | | | | | |
|---|---|---|---|---|---|---|---|---|
| | 2.408 | 1.292 | 0.610 | 0.371 | 0.265 | 0.258 | 0.145 | 0.103 |
| | 2.846 | 1.054 | 0.621 | 0.454 | 0.222 | 0.181 | 0.162 | 0.113 |
| | | | | | | | | |
| mean | 2.627 | 1.173 | 0.616 | 0.413 | 0.244 | 0.220 | 0.154 | 0.108 |
| corr. mean | 2.519 | 1.065 | 0.508 | 0.305 | 0.136 | 0.112 | 0.046 | 0.000 |
| ng/mL | 10 | 5 | 2.5 | 1.25 | 0.63 | 0.32 | 0.16 | 0 |

| | | | | | | | | |
|---|---|---|---|---|---|---|---|---|
| corr. mean = corrected mean = (measured sample OD - OD at 0 ng/mL) | | | | | | | | |

The regression line obtained with the commercial prior art kit is shown in **Figure 7****.**

### Analysis of patients' serum samples:

The serum samples of the glioma patients of example 2 were analyzed for VE-cadherin content, using the commercial prior art kit, in accordance with the manufacturer's instructions (no prior dilution in a surfactant-containing buffer).
Values are reported in table 7 below (R= responsive = serum of glioma patients that will be responsive to anti-tumour therapy; NR= non-responsive = serum of glioma patients that won't be responsive to anti-tumour therapy).

**Table 7 (commercially-available kit):**

| RESPONSIVE | | OD1 | OD2 | mean | corr. mean | ng/mL |
|---|---|---|---|---|---|---|
| | A | 0.141 | 0.136 | 0.139 | 0.031 | ND |
| | B | 0.121 | 0.120 | 0.121 | 0.013 | ND |
| | C | 0.150 | 0.138 | 0.144 | 0.036 | ND |
| | D | 0.178 | 0.147 | 0.163 | 0.055 | 56.255 |
| | E | 0.116 | 0.123 | 0.120 | 0.012 | ND |
| | F | 0.121 | 0.132 | 0.127 | 0.019 | ND |
| | G | 0.203 | 0.153 | 0.178 | 0.070 | 72.254 |
| | H | 0.126 | 0.126 | 0.126 | 0.018 | ND |
| | I | 0.122 | 0.123 | 0.123 | 0.015 | ND |
| | J | 0.191 | 0.125 | 0.158 | 0.050 | 51.610 |
| | K | 0.160 | 0.209 | 0.185 | 0.077 | 78.964 |
| | L | 0.124 | 0.124 | 0.124 | 0.016 | ND |
| | | | | | | |

| NON RESPONSIVE | | OD1 | OD2 | mean | corr. mean | ng/mL |
|---|---|---|---|---|---|---|
| | M | 0.240 | 0.121 | 0.181 | 0.073 | 74.835 |
| | N | 0.119 | 0.123 | 0.121 | 0.013 | ND |
| | O | 0.114 | 0.117 | 0.116 | 0.008 | ND |
| | P | 0.114 | 0.121 | 0.118 | 0.009 | ND |
| | Q | 0.159 | 0.119 | 0.139 | 0.031 | ND |
| | R | 0.139 | 0.191 | 0.165 | 0.057 | 58.836 |
| | S | 0.127 | 0.134 | 0.131 | 0.023 | ND |
| | T | 0.131 | 0.137 | 0.134 | 0.026 | ND |
| | U | 0.204 | 0.140 | 0.172 | 0.064 | 66.061 |
| | V | 0.117 | 0.119 | 0.118 | 0.010 | ND |
| | W | 0.122 | 0.125 | 0.124 | 0.016 | ND |
| | X | 0.111 | 0.117 | 0.114 | 0.006 | ND |

| | | | | | | |
|---|---|---|---|---|---|---|
| ND = not detected corr. mean = corrected mean = (measured sample OD - OD at 0 ng/mL) | | | | | | |

When the prior art kit is being used to measure sVE-cadherin, no correlation can be observed between the responsive/no-responsive status of the patient, and the content in circulating VE-cadherin, whereas the analysis performed in accordance with the invention (VE-cadherin detection performed with prior dilution in a surfactant-containing buffer; see e.g., example 2) proves that such a correlation can be reliably made (the serum of those patients, who will be responsive to anti-tumor treatment contains circulating VE-cadherin (90 kDa on western blot) at a level not significantly lower than the normal level observed in a healthy volunteer)).
Hence, the prior art sVE-cadherin kit is much less accurate and/or reliable than the means of the invention, and did not enable to attain the results achieved by the invention.

### EXAMPLE 7: circulating VE-cadherin is associated with lipids in serum

Examples 1-6 above demonstrate that using a surfactant, preferably a non denaturing surfactant, such as Triton® X-100, highly improves the detection of circulating VE-cadherin in biological fluids, such as serum or plasma, and that it even enables a reliable quantification thereof.
The inventors hypothesized that circulating proteins, such as circulating VE-cadherin, are somehow associated to an entity that impedes their accurate detection, when performed in the absence of surfactant.
In order to better understand the effects of detergent on the extractibility of VE-cadherin, the inventors attempted to see if circulating VE-cadherin was associated with lipid entities, such as lipoprotein, in serum. To that purpose, the inventors performed an ultracentrigation to separate particules and lipids. 400 µL of serum (from a responsive patient with a high level of VE-cadherin detected by westernblotting) were ultracentrifuged (45,000 rpm; 100,000g) for 60 min at 4°C.After centrifugation, the lipids at the top of the sample (visible) were delicately transferred in an Eppendorf tube and resolubilized in 40 µL of PBS/Triton® X-100 0.5%.The pellet (not visible) was solubilized in 40 µL of PBS/Triton® X-100 0.5%. An aliquot (5 µL) of each fraction was diluted serially 1/5 and 1/10 (i.e., 1/50) in PBS / Triton® X-100 0.5%. The dilution 1/200 of the serum prior to centrifugation was analyzed (S). As shown in Figure 15A, the presence of VE-cadherin detected in serum diluted 1/200 (left lane) is also found in the lipid fraction (L) diluted 1/5 or 1/50, while no signal was detectable in the pellet (P).
The lipid fraction has been analyzed by Coomassie staining (Figure 15B) at the dilution 1/25 and 1/50 and by western-blotting. The immuoreactive band corresponding to soluble VE-cadherin is found in the lipidic fraction (Figure 15C).

Another protein was also detected in this fraction as shown on Figure 15D: E-cadherin This experiment demonstrates that at least a main part of soluble VE-cadherin is associated with lipids in the serum (E-cadherin is also detected in these fractions).

### EXEMPLE 8: Separation of lipoproteins by precipitation

To obtain the different lipoproteins from human serum, we used a precipitation technique where sodium phosphotungstate and MgCl2 were added to crude serum (see table 8).
Bulk separation of lipoproteins from normolipemic sera was performed in accordance with the original method of Garcia-Parra M, Mejiade, Gardia M, and Weigandt H, "A new method for lipoprotein isolation by precipitation". In Protides of Biological Fluids., Proceedings of 25th Colloquium Brugge, Editor: H Peeters, Pergamon press, New York, 1977, p. 411-415.

Reagents:
- sodium phosphotungstate 4% (w/v),
- magnesium chloride (MgCl₂•6H₂0) 2M in distilled water.
From the crude Lp (a), VLDL and LDL fractions, the purified lipoproteins were obtained by precipitation using 0.2 mL of sodium phosphotungstate and 0.05mL of magnesium chloride solution. The crude HDL fraction was treated with 2mL of sodium phosphotungstate and 0.4 mL of magnesium chloride solution to obtain the precipitate of pure HDL. All the precipitates were dissolved in PBS/Triton® X-100 0.5%

**Table 8:**

| | **Chy+VLDL 1** | **LDL 2** | **HDL 3** |
|---|---|---|---|
| **Sodium phosphotungstate** | **0.05%** | **0.2%** | **2%** |
| **MgCl₂ 2M** | **0.1M** | **0.1M** | **0.2M** |

The samples are centrifuged and the pellets are solubilized in PBS/Triton® X-100 0.5%. 5µL of each sample diluted 1/50 were analyzed by SDS-PAGE (Coomassie; Figure 16A) and SDS-PAGE and western blot using the BV9 anti VE-cadherin antibody (Figure 16B).

### EXEMPLE 9: detection of soluble VE-cadherin after separation of lipoproteins

### 1/ Analysis of serum with or without lipoproteins:

50 µL of serum (glioma patient of example 2, who will be responsive to (chemotherapy) were treated with phosphotungstic acid in the presence of MgCl2 to allow precipitation of all classes of lipoproteins. The sample was then centrifuged 3 min (7,000 rpm) at 4°C. The supernatant was analyzed for the presence of VE-cadherin after dilution 1/200 in PBS in the presence or absence of Triton® X-100 0.5%.
Figure 17A shows the protein pattern of 5µL of non-healthy human serum diluted 1/200 (S) in PBS with or without Triton® X-100, 5µL of the same serum without lipoproteins (S-Lipo) diluted 1/200 in PBS with or without Triton® X-100.
Figure 17B shows the immunoblot analysis of the same fractions with BV9 (0.2 µg/mL). VE-cadherin level is very low in S-Lipo and the signal is not increased by Triton®-X-100, while the VE-cadherin signal is higher is whole serum and increased by Triton® X-100, This experiment demonstrates that at least a main part of the sVE-cadherin is associated with lipoproteins fractions.

### 2/ Analysis of the lipoproteins fraction obtained by precipitation with Phosphotungstic acid.

After centrifugation of the serum treated with phosphotungstic acid, the pellet was solubilized in PBS/0.5% Triton® X-100, 0.5% DOC; the 1/25 and 1/50 dilutions were analyzed by Coomassie staining and western blotting with BV9. Figure 17C shows the proteins patterns of 5µL of each dilution. Figure 17D shows the analysis of the same fractions by westernblotting with BV9. The presence of VE-cadherin is found associated with the lipoproteins fraction. The experiment has been repeated on 2 independent preparations of lipoproteins fraction from the same serum (Lipo1 and Lipo2)

### Abbreviations

- CAM: Cellular Adhesion Molecule
- DMEM: Dulbecco's Modified Eagle Medium
- HDL: High Density Lipoprotein
- HUVEC: Human Umbilical Vein Endothelial Cell
- IL: Interleukin
- KDa: kiloDalton
- LDL: Low Density Lipoprotein
- PBS: Phosphate Buffered Saline
- SDS: Sodium Dodecyl Sulphate
- TEMED: N, N, NT ,NT - Tetramethylethylenediamine
- TWEEN 20: Polyoxyethylenesorbitan monolaurate
- VE-cadherin (or VEcadherin): Vascular Endothelial cadherin
- VLDL: Very Low Density Lipoprotein
- VPF: Vascular Permeability Factor

### REFERENCES

(1) The molecular organization of endothelial cell to cell junctions: differential association of plakoglobin, B catenin, and a catenin with Vascular Endothelial Cadherin (VE-cadherin). Lampugnani, M. G et al. The Journal of Cell Biology, Volume 129, Number 1, 203- 217 April 1995
(2) Tyrosine phosphorylation of vascular endothelial cadherin and the regulation of microvascular permeability. Fiemu E. Nwariaku et al (Surgery 2002; 132:180-5)
(3) The cadherin superfamily: diversity in form and function. Angst BD, Marcozzi C, and Magee AI. J Cell Sci 114: 629-641, 2001
(4) VE-cadherin: adhesion at arm's length. Vincent P. A, Xiao K, Buckley K. M and Kowalczyk A. P Am J Physiol Cell Physiol 286: C987-C997, 2004
(5) Monoclonal antibodies directed to different regions of vascular endothelial cadherin extracellular domain affect adhesion and clustering of the protein and modulate endothelial permeability. Corada, M et al Blood, 15 March 2001. Volume 97, Number 6.
(6) Angiogenesis in health and disease. Carmeliet, P. Nature Medicine, Volume 9, Number 6, 653- 660 June 2003
(7) Endothelial proliferation in tumours and normal tissues: continuous labelling studies. Hobson, B, Denekamp, J. Br J Cancer 1984; 49. 405-413
(8) Vascular endothelial cadherin tyrosine phosphorylation in angiongenic and quiescent adult tissues. Lambeng, N et al. (Circ Res 2005; 96: 384-391)
(9) Molecular regulation of vessel maturation. Jain, R. K. Nature Medicine, Volume 9, Number 6, 685- 693 June 2003
(10) Vascular endothelial growth factor is a secreted angiogenic mitogen. Leung D. W., Cachianes G., Kuang W. J., Goeddel D. V., Ferrara N.Science (Wash. DC), 246: 1306-1309, 1989.
(11) Vascular Endothelial Growth Factor induces VE-cadherin tyrosine phosphorylation in endothelial cells. Esser, S et al. Journal of cell science, 111, 1853-1865 (1998)
(12) Vascular Endothelial Growth Factor-Trap Decreases Tumor Burden, Inhibits Ascites, and Causes Dramatic Vascular Remodeling in an Ovarian Cancer Model. Byrne, A. T. et al Clinical Cancer Research Vol. 9, 5721-5728, November 15, 2003
(13) Human vascular permeability factor. Isolation from U937 cells. Connolly D. T et al. J.Biol. Chem., 264: 20017-20024, 1989
(14) Serum markers of angiogenesis and myocardial ultrasonic tissue characterization in patients with dilated cardiomyopathy. Ohtsuka, T et al. European Journal of Heart Failure Volume 7, Issue 4, June 2005, Pages 689-695
(15) Preoperative assessment of tumor angiogenesis by vascular endothelial growth factor mRNA expression in homogenate samples of breast carcinoma: fine-needle aspirates vs. resection samples. Anan K, et al J Surg Oncol. 1997 Dec; 66(4):257-63.
(16) The role of interleukin-8 and its receptors in gliomagenesis and tumoral angiogenesis. Brat DJ, Bellail AC, Van Meir EG. Neuro-oncol. 2005 Apr; 7(2): 122-33.
(17) Antiangiogenic strategies in neuroblastoma. Ribatti, D and Ponzoni, M Cancer Treatment Reviews Volume 31, Issue 1 , February 2005, Pages 27-34
(18) Circulating plasma vascular endothelial growth factor in mice bearing human ovarian carcinoma xenograft correlates with tumor progression and response to therapy. Manenti L, et al Mol Cancer Ther. 2005 May; 4(5):715-25.
(19) Current management of glioblastoma multiforme. Grossman SA, Batara JF.Semin Oncol. 2004 Oct; 31(5):635-44.
(20) Bull Cancer. 2005 Apr; 92(4):360-72. Angiogenesis and anti-angiogenic strategies for glioblastoma. de Bouard S, Guillamo JS.

## Claims

1. A method for predicting the sensitivity or resistance of an animal or patient to anti-tumor treatment, comprising the steps of:
a. providing a fluid sample previously collected from said animal or patient;
b. diluting said sample in one surfactant-containing solution comprising at least one surfactant;
c. analysing, in said diluted fluid sample, its content in circulating VE-cadherin;
d. comparing said content in circulating VE-cadherin obtained at step c) with a mean content in circulating VE-cadherin observed in comparable fluids of healthy volunteers; a significantly lower content is indicative of the said patient of being at high risk of being resistant to anti-tumor treatment,
wherein said anti-tumor treatment (i) is chemotherapy with an alkylating agent or chemotherapy with an alkylating agent and radiotherapy and (ii) is administered to a patient affected by a central nervous system tumor.

2. The method of claim 1, wherein said circulating VE-cadherin consists of its extracellular region.

3. The method of claim 1 or 2, wherein the central nervous system tumor is brain tumor.

4. The method of any one of claims 1 to 3, wherein the central nervous system tumor is glioma.

5. The method of claim 4, wherein the glioma is glioblastoma, oligodendroglioma or oligo-astrocytoma.

6. The method of any one of claims 1 to 5, wherein said sample is serum or plasma.

7. The method of of any one of claims 1 to 6, wherein said alkylating agent is Temolozomide.

## Patentansprüche

1. Verfahren zum Prognostizieren der Empfindlichkeit oder Resistenz eines Tiers oder Patienten gegenüber bzw. gegen eine Antitumorbehandlung, das die Schritte umfasst:
a. Bereitstellen einer Fluidprobe, die zuvor einem Tier oder Patienten entnommen wurde;
b. Verdünnen der Probe in einer tensidhaltigen Lösung, die zumindest ein Tensid umfasst;
c. Analysieren des Gehalts an zirkulierendem VE-Cadherin in der verdünnten Fluidprobe;
d. Vergleichen des Gehalts an zirkulierendem VE-Cadherin, der in Schritt c) erhalten wurde, mit einem mittleren Gehalt an zirkulierendem VE-Cadherin, der bei vergleichbaren Fluiden gesunder Freiwilliger beobachtet wurde; wobei ein signifikant geringerer Gehalt dafür indikativ ist, dass bei dem Patienten ein hohes Risiko einer Resistenz gegen eine Antitumorbehandlung besteht,
wobei die Antitumorbehandlung (i) eine Chemotherapie mit einem Alkylierungsmittel oder eine Chemotherapie mit einem Alkylierungsmittel und Strahlentherapie ist und (ii) an einen Patienten verabreicht wird, der an einem Tumor des zentralen Nervensystems leidet.

2. Verfahren nach Anspruch 1, wobei das zirkulierende VE-Cadherin aus seiner extrazellulären Region besteht.

3. Verfahren nach Anspruch 1 oder 2, wobei der Tumor des zentralen Nervensystems ein Gehirntumor ist.

4. Verfahren nach einem der Ansprüche 1 bis 3, wobei der Tumor des zentralen Nervensystems ein Gliom ist.

5. Verfahren nach Anspruch 4, wobei das Gliom ein Glioblastom, Oligodendrogliom oder Oligoastrozytom ist.

6. Verfahren nach einem der Ansprüche 1 bis 5, wobei die Probe Serum oder Plasma ist.

7. Verfahren nach einem der Ansprüche 1 bis 6, wobei das Alkylierungsmittel Temozolomid ist.

## Revendications

1. Procédé pour prédire la sensibilité ou la résistance d'un animal ou d'un patient à un traitement antitumoral, comprenant les étapes qui consistent à :
a. mettre à disposition un échantillon de liquide préalablement collecté chez ledit animal ou patient ;
b. diluer ledit échantillon dans une solution contenant un tensioactif comprenant au moins un tensioactif ;
c. analyser, dans ledit échantillon de liquide dilué, sa teneur en VE-cadhérine circulante ;
d. comparer ladite teneur en VE-cadhérine circulante obtenue à l'étape c) avec une teneur moyenne en VE-cadhérine circulante observée dans des liquides comparables de volontaires sains ; une teneur significativement plus faible indiquant que ledit patient est exposé à un risque élevé de résistance à un traitement antitumoral,
où ledit traitement antitumoral (i) est une chimiothérapie avec un agent alkylant ou une chimiothérapie avec un agent alkylant et une radiothérapie et (ii) est administré à un patient affecté par une tumeur du système nerveux central.

2. Procédé selon la revendication 1, dans lequel ladite VE-cadhérine circulante consiste en sa région extracellulaire.

3. Procédé selon la revendication 1 ou 2, dans lequel la tumeur du système nerveux central est une tumeur cérébrale.

4. Procédé selon l'une quelconque des revendications 1 à 3, dans lequel la tumeur du système nerveux central est un gliome.

5. Procédé selon la revendication 4, dans lequel le gliome est un glioblastome, un oligodendrogliome ou une oligoastrocytome.

6. Procédé selon l'une quelconque des revendications 1 à 5, dans lequel ledit échantillon est du sérum ou du plasma.

7. Procédé selon l'une quelconque des revendications 1 à 6, dans lequel ledit agent alkylant est le témozolomide.
